# EUROPEAN PATENT SPECIFICATION

(11) **EP 2 509 941 B1**
(45) Date of publication and mention of the grant of the patent: **23.01.2019**
(21) Application number: 10793381.4
(22) Date of filing: 07.12.2010
(51) Int. Cl.: C07C 311/48, A61K 31/18, A61P 9/04, C07D 265/00, C07C 317/14

(54) **N-ACYLATED HYDROXYLAMINE DERIVATIVES AND O-ACYLATED HYDROXYLAMINE DERIVATIVES**
N-ACELYERTE HYDROXYLAMINDERIVATE UND O-ACYLIERTE HYDROXYLAMINDERIVATE
DÉRIVÉS D'HYDROXYLAMINE N-ACYLÉS OU O-ACYLÉS

(30) Priority: 07.12.2009 US 267401 P; 30.12.2009 US 291224 P
(43) Date of publication of application: 17.10.2012
(73) Proprietor: The Johns Hopkins University, Baltimore, MD 21218 (US); Cardioxyl Pharmaceuticals Inc., Chapel Hill, NC 27517 (US)
(72) Inventor: TOSCANO, John P., Glen Arm Maryland 21057 (US); SUTTON, Art, Baltimore Maryland 21218 (US); KALISH, Vincent J., Annapolis Maryland 21401 (US); BROOKFIELD, Frederick Arthur, Abingdon Oxfordshire OX14 4SA (GB); COURTNEY, Stephen Martin, OX14 4SA (GB); FROST, Lisa Marie, OX14 4SA (GB)
(74) Representative: J A Kemp
(86) International application number: PCT/US2010/059335
(87) International publication number: WO 2011/071951

(56) References cited:
- WO-A1-2007/109175
- WO-A1-2009/137717
- WO-A2-03/020221
- US-B1- 6 420 409
- KLAUS REHSE AND CO: "Hydroxylamine Derivatives", ARCHIV DER PHARMAZIE, vol. 331, 24 April 1998 (1998-04-24), pages 365-367, XP002660162,
- TERRY T. CONWAY ET AL: "Diethylcarbamoylating/Nitroxylating Agents as Dual Action Inhibitors of Aldehyde Dehydrogenase: A Disulfiram-Cyanamide Merger", JOURNAL OF MEDICINAL CHEMISTRY, vol. 42, no. 20, 1 October 1999 (1999-10-01), pages 4016-4020, XP055007586, ISSN: 0022-2623, DOI: 10.1021/jm990235p
- LEE M J C ET AL: "PRODRUGS OF NITROXYL AS INHIBITORS OF ALDEHYDE DEHYDROGENASE", JOURNAL OF MEDICINAL CHEMISTRY, AMERICAN CHEMICAL SOCIETY, US, vol. 35, 1 January 1992 (1992-01-01), pages 3648-3652, XP002056135, ISSN: 0022-2623, DOI: 10.1021/JM00098A008
- MELINDA J C LEE AND CO: "N-hydroxybenzenecarboximic acid derivatives", NITRIC OXIDE, vol. 5, no. 3, 9 May 2001 (2001-05-09), pages 278-287, XP002661262,
- ROBERT B HARRIS AND CO: "Synthesis of t-butyl aminocarbonate", TETRAHEDRON LETTERS, vol. 24, no. 3, 1 January 1983 (1983-01-01), pages 231-232, XP002661263,
- N E ALEXANDROU AND CO: "1,3-addition reactiond of the benzonitrile oxide with acid anions", TETRAHEDRON LETTERS, vol. 22, 29 March 1966 (1966-03-29), pages 2497-2499, XP002661264,
- ZINNER G ET AL: "DARSTELLUNG UND ACYLIERUNGEN VON 1.1-DIALKYL1-3-HYDROXYHARNSTOFFEN//S YNTHESIS AND ACYLATIONS OF 1.1-DIALKYL-3-HYDROXYUREAS", ARCHIV DER PHARMAZIE, WILEY - VCH VERLAG GMBH & CO. KGAA, DE, vol. 307, 1 January 1974 (1974-01-01), pages 7-12, XP009041543, ISSN: 0365-6233
- P F ALEWOOD AND CO: "an improved preparation of N-(t-butyl)-N-(3,5-dinitrobenzoyl)-nitroxy l", SYNTHESIS, 1 February 1981 (1981-02-01), pages 121-122, XP002661266,
- HAROLD W. HEINE ET AL: "Synthesis and reactions of N-(2,4-dichloro-6-oxo-2,4-cyclohexadien-1- ylidene)-4-nitrobenzamide with alkenes", THE JOURNAL OF ORGANIC CHEMISTRY, vol. 49, no. 14, 1 July 1984 (1984-07-01), pages 2560-2565, XP055009403, ISSN: 0022-3263, DOI: 10.1021/jo00188a009
- PETER A. S. SMITH ET AL: JOURNAL OF THE AMERICAN CHEMICAL SOCIETY, vol. 82, no. 21, 5 November 1960 (1960-11-05), pages 5731-5740, XP055009081, ISSN: 0002-7863, DOI: 10.1021/ja01506a043

## Description

This application claims the benefit of U.S. Provisional Application No. 61/267,401, filed on December 7, 2009, and U.S. Provisional Application No. 61/291,224, filed on December 30, 2009.

### Congestive Heart Failure (CHF)

Congestive heart failure (CHF) is a generally progressive, life threatening condition in which myocardial contractility is depressed such that the heart is unable to adequately pump the blood returning to it, also referred to as decompensation. Symptoms include breathlessness, fatigue, weakness, leg swelling, and exercise intolerance. On physical examination, patients with heart failure often have elevated heart and respiratory rates (an indication of fluid in the lungs), edema, jugular venous distension, and/or enlarged hearts. The most common cause of CHF is atherosclerosis, which causes blockages in the coronary arteries that provide blood flow to the heart muscle. Ultimately, such blockages may cause myocardial infarction with subsequent decline in heart function and resultant heart failure. Other causes of CHF include valvular heart disease, hypertension, viral infections of the heart, alcohol consumption, and diabetes. Some cases of CHF occur without clear etiology and are called idiopathic. The effects of CHF on an individual experiencing the condition can be fatal.

There are several types of CHF. Two types of CHF are identified according to which phase of the cardiac pumping cycle is more affected. Systolic heart failure occurs when the heart's ability to contract decreases. The heart cannot pump with enough force to push a sufficient amount of blood into the circulation leading to a reduced left ventricular ejection fraction. Lung congestion is a typical symptom of systolic heart failure. Diastolic heart failure refers to the heart's inability to relax between contractions and allow enough blood to enter the ventricles. Higher filling pressures are required to maintain cardiac output, but contractility as measured by left ventricular ejection fraction is typically normal. Swelling (edema) in the abdomen and legs is a typical symptom of diastolic heart failure. Often, an individual experiencing heart failure will have some degree of both systolic heart failure and diastolic heart failure.

CHF is also classified according to its severity. The New York Heart Association classifies CHF into four classes: Class I involves no obvious symptoms, with no limitations on physical activity; Class II involves some symptoms during or after normal activity, with mild physical activity limitations; Class III involves symptoms with less than ordinary activity, with moderate to significant physical activity limitations; and Class IV involves significant symptoms at rest, with severe to total physical activity limitations. Typically, an individual progresses through the classes as they live with the condition.

Although CHF is generally thought of as a chronic, progressive condition, it can also develop suddenly. This type of CHF is called acute CHF, and it is a medical emergency. Acute CHF can be caused by acute myocardial injury that affects either myocardial performance, such as myocardial infarction, or valvular/chamber integrity, such as mitral regurgitation or ventricular septal rupture, which leads to an acute rise in left ventricular and diastolic pressure resulting in pulmonary edema and dyspnea.

Common treatment agents for CHF include vasodilators (drugs that dilate blood vessels), positive inotropes (drugs that increase the heart's ability to contract), and diuretics (drugs to reduce fluid). Additionally, *beta*-antagonists (drugs that antagonize *beta-*adrenergic receptors) have become standard agents for treating mild to moderate heart failure. Lowes et al., Clin. Cardiol. 2000, 23, III, 1-6 .

Positive inotropic agents include beta-adrenergic agonists, such as dopamine, dobutamine, dopexamine, and isoproterenol. However, use of a beta-agonist has potential complications, such as arrhythmogenesis and increased oxygen demand by the heart. Additionally, the initial short-lived improvement of myocardial contractility afforded by these drugs is followed by an accelerated mortality rate resulting largely from a greater frequency of sudden death. Katz, Heart Failure: Pathophysiology, Molecular Biology And Clinical Management 1999, Lippincott, Williams & Wilkins.

*Beta*-antagonists antagonize *beta*-adrenergic receptor function. While initially contraindicated in heart failure, they have been found to provide a marked reduction in mortality and morbidity in clinical trials. Bouzamondo et al., Fundam. Clin. Pharmacol. 2001, 15, 95- 109. Accordingly, they have become an established therapy for heart failure. However, even individuals that improve under *beta*-antagonist therapy may subsequently decompensate and require acute treatment with a positive inotropic agent. Unfortunately, as their name suggests, *beta*-antagonists block the mechanism of action of the positive inotropic *beta*-agonists that are used in emergency care centers. Bristow et al., J. Card. Fail., 2001, 7, 8-12.

Vasodilators, such as nitroglycerin, have been used for a long period of time to treat heart failure. However, the cause of nitroglycerin's therapeutic effect was not known until late in the last century when it was discovered that the nitric oxide molecule (NO) was responsible for nitroglycerin's beneficial effects. In some individuals experiencing heart failure, a nitric oxide donor is administered in combination with a positive inotropic agent to both cause vasodilation and to increase myocardial contractility. However, this combined administration can impair the effectiveness of positive inotropic treatment agents. For example, Hart et al, Am. J. Physiol. Heart Circ. Physiol. 2001, 281, 146-54, reported that administration of the nitric oxide donor sodium nitroprusside, in combination with the positive inotropic, *beta-* adrenergic agonist dobutamine, impaired the positive inotropic effect of dobutamine. Hare et al., Circulation 1995, 92, 2198-203, also disclosed the inhibitory effect of nitric oxide on the effectiveness of dobutamine.

As described in U.S. Patent No. 6,936,639, compounds that donate nitroxyl (HNO) under physiological conditions have both positive inotropic and lusotropic effects and offer significant advantages over existing treatments for failing hearts. Due to their concomitant positive inotropic/lusotropic action and unloading effects, nitroxyl donors were reported as helpful in treating cardiovascular diseases characterized by high resistive load and poor contractile performance. In particular, nitroxyl-donating compounds were reported as useful in the treatment of heart failure, including heart failure in individuals receiving *beta*-antagonist therapy.

### Ischemia

Ischemia is a condition characterized by an interruption or inadequate supply of blood to tissue, which causes oxygen deprivation in the affected tissue. Myocardial ischemia is a condition caused by a blockage or constriction of one or more of the coronary arteries, such as can occur with atherosclerotic plaque occlusion or rupture. The blockade or constriction causes oxygen deprivation of the non-perfused tissue, which can cause tissue damage. Further, upon reperfusion with subsequent reoxygenation of the tissue, when the blood is able to flow again or the oxygen demand of the tissue subsides, additional injury can be caused by oxidative stress.

Ischemia/reperfusion injury refers to tissue damage caused by oxygen deprivation followed by reoxygenation. The effects of ischemia/reperfusion injury in an individual experiencing the condition can be fatal, particularly when the injury occurs in a critical organ such as the heart or brain.

Accordingly, compounds and compositions effective in preventing or protecting against ischemia/reperfusion injury would be useful pharmaceuticals. Compounds such as nitroglycerin have been used for a long period of time to help control vascular tone and protect against myocardial ischemia/reperfusion injury. It was discovered that the nitric oxide molecule was responsible for nitroglycerin's beneficial effects. This discovery prompted interest in medical uses for nitric oxide and investigations into related species such as nitroxyl. As reported in U.S. Patent Application Serial No. 10/463,084 (U.S. Publication No. 2004/0038947), administration of a compound that donates nitroxyl under physiological conditions, prior to ischemia, can attenuate ischemia/reperfusion injury to tissues, for example, myocardial tissues. This beneficial effect was reported as a surprising result given that nitroxyl was previously reported to increase ischemia/reperfusion injury (*see,* Ma et al., Proc. Nat'l Acad. Sci. 1999, 96(25), 14617- 14622, reporting that administration of Angeli's salt (a nitroxyl donor under physiological conditions) to anesthetized rabbits during ischemia and 5 minutes prior to reperfusion increased myocardial ischemia/ reperfusion injury, and Takahira et al., Free Radical Biology & Medicine 2001, 31(6), 809-815, reporting that administration of Angeli's salt during ischemia and 5 minutes before reperfusion of rat renal tissue contributed to neutrophil infiltration into the tissue, which is believed to mediate ischemia/reperfusion injury). In particular, pre-ischemic administration of Angeli's salt and isopropylamine/NO has been reported to prevent or reduce ischemia/reperfusion injury.

### Cancer

One of the challenges in developing anti-cancer drugs is to discover compounds that are selectively toxic to tumor cells over normal cells. It has been found that tumor tissues have an acidic microenvironment with a pH from 6.0 to 7.0, while the extra- and intracellular milieu of normal cells has a pH of 7.4. Angeli's salt has been reported to exhibit strong cytotoxicity to cancer cells in weakly acidic solutions, whereas no toxicity was observed at pH 7.4 (Stoyanovsky, D.A. et al. J. Med. Chem. 2004, 47, 210-217; and PCT Publication No. WO/2003/020221). In a subcutaneous xenograft model of pheochromocytoma, Angeli's salt was found to inhibit tumor growth at a dose that was nontoxic to nude mice. Nitroxyl derivatives that are not known to release HNO, such as ruboxyl, a nitroxyl analogue of daunorubicin, have been shown to be active against hepatic metastases from colorectal carcinoma (Sirovich, I. et al Tumor Biol. 1999, 20, 270-276).

Norris A. J. et al., Intl. J. Cancer 2008, 122, 1905-1910, reported that Angeli's salt inhibits the proliferation of cultured breast cancer cells and decreases tumor mass in a mouse xenograft model. Norris A. J. *et al* proposed that HNO released from Angeli's salt blocks glycolysis in cancer cells by inhibiting the enzyme glyceraldehyde 3-phosphate dehydrogenase (GAPDH), resulting in decreased levels of HIF- 1 α (hypoxia-inducible factor) protein and activity, lower VEGF (vascular endothelial growth factor) production, decreased tumor angiogenesis and an increase in apoptotic cells.

### Pulmonary Hypertension

Pulmonary hypertension (PH) is a generic term for a group of conditions characterized by elevated blood pressure in the arteries of the lungs (pulmonary arteries). In patients with PH, characteristic changes occur within the pulmonary circulation. These changes include thickening of the linings and obstruction of the small pulmonary blood vessels. As a result of these changes, pressure in the pulmonary circulation rises, and resistance in the blood flowing through the vessels increases. This increased resistance puts a strain on the right side of the heart as it must work harder to pump blood to the lungs. This strain can cause the heart to enlarge. Eventually, heart failure can develop.

The World Health Organization (WHO) classification of PH¹, as updated in the 2008 4^{th} World Conference in Dana Point, California, includes five groups: pulmonary arterial hypertension (PAH)(Group 1), PH owing to left heart disease (Group 2), PH owing to lung diseases and/or hypoxia (Group 3), chronic thromboembolic PH (Group 4), and PH with unclear multifactorial mechanisms (Group 5).
¹ The initial attempt to develop a classification for PH was undertaken during the WHO Conference on PH in 1973. Since then, the PH classification has been revised three times, first at the 1998 2^{nd} World Symposium in Evian, France, then at the 2003 3^{rd} World Symposium in Venice, Italy, and most recently at the 2008 4th World Symposium in Dana Point, California.

Notwithstanding the current WHO classification, some literature still refer to the older classification system of "primary" and "secondary" PH. Primary PH refers to idiopathic PH, while secondary PH refers to PH that develops from another medical condition. For example, under the older classification system, PH owing to left heart disease was classified as PH secondary to left heart disease.

Current therapies for PH include supplemental oxygen, diuretics, oral vasodilators such as calcium channel blockers, anticoagulants, inotropic agents, prostanoids, endothelin receptor antagonists, and phosphodiesterase type-5 inhibitors. While such therapies have met with some success, many PH patients fail to respond to these therapies.

### Nitroxyl Donors

Due to its inherent reactivity, HNO must be generated *in situ* from donor compounds. To date, the vast majority of studies of the biological effect of HNO have used the donor sodium α-oxyhyponitrite ("Angeli's salt" or "AS"). However, the chemical stability of AS has made it unsuitable to develop as a therapeutic agent. Angeli's salt also releases nitrite, which possesses its own biological profile. *N*-hydroxybenzenesulfonamide ("Piloty's acid" or "PA") has previously been shown to be a nitroxyl donor only at high pH (>9) (Bonner, F.T. et al., Inorg. Chem. 1992, 31, 2514-2519). Under physiological conditions, PA has been shown to be a nitric oxide donor via an oxidative pathway (Zamora, R. et al., Biochem. J. 1995, 312, 333-339). PCT Patent Application Publication No. WO/2007/109175 describes *N*-hydroxylsulfonamide derivatives that donate nitroxyl under physiological conditions.

Acyloxy nitroso compounds have been reported to yield nitroxyl *in situ* when reacted with nucleophiles (Sha, X. et al., J. Am. Chem. Soc. 2006, 128, 9687-9692). Although Rehse et al., Arch. Pharm. Med. Chem. 1998, 331, 104-110, showed acyloxy nitroso compounds inhibit platelet aggregation and thrombus formation (indicative of NO release), they generate only small amounts (<1%) of NO and HNO under neutral conditions. International Patent Application Publication WO 2007/120839 describes conjugates of acyloxy nitroso compounds with non-steroidal anti-inflammatory drugs (NSAID) as nitroxyl donors for treating congestive heart failure.

### Significant Medical Need

Despite efforts towards the development of new therapies for the treatment of the diseases and conditions described above, there remains a significant medical need for additional or alternative compounds that treat, prevent or delay the onset and/or development of these and related diseases or conditions. In particular, there remains a significant medical need for alternative or additional therapies for the treatment of diseases or conditions that are responsive to nitroxyl therapy. New compounds that donate nitroxyl under physiological conditions and methods of using compounds that donate nitroxyl under physiological conditions may thus find use as therapies for treating, preventing and/or delaying the onset and/or development of diseases or conditions responsive to nitroxyl therapy, including heart disease, ischemia/reperfusion injury and cancer. Preferably, the therapeutic agents can improve the quality of life and/or prolong the survival time for patients with the disease or condition.
Rehse *et al* describe hydroxylamine derivatives in Achiv der Pharmazie, vol. 331,24 April 1998, pages 365-367.
Conway *et al* describe diethylcarbamoylating/nitroxylating agents as dual action inhibitors of aldehyde dehydrogenase in J. Med. Chem. 1999, 42, 4016-4020. Lee *et al* describe prodrugs of nitroxyl as inhibitors of aldehyde dehydrogenase in J. Med. Chem. 1992, 35, 3648-3652.
Lee *et al* describe N-hydroxybenzenecarboximidic acid derivatives as nitroxyl-generating prodrugs (Nitric Oxide, vol.5, no. 3, 2001, pages 278-287).

### Brief Description of the Drawings

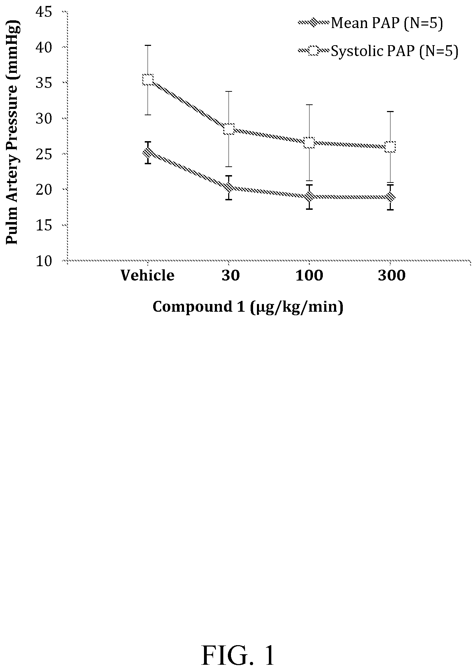

FIG. 1 shows the intravenous effects of a nitroxyl (HNO) donor on mean and systolic (peak) pulmonary artery pressure (PAP) in rats.

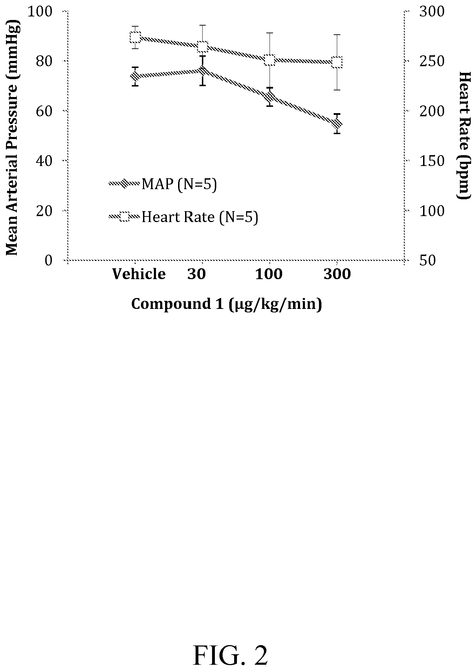

FIG. 2 shows the intravenous effects of a nitroxyl (HNO) donor on mean arterial pressure (MPAP) and heart rate in rats.

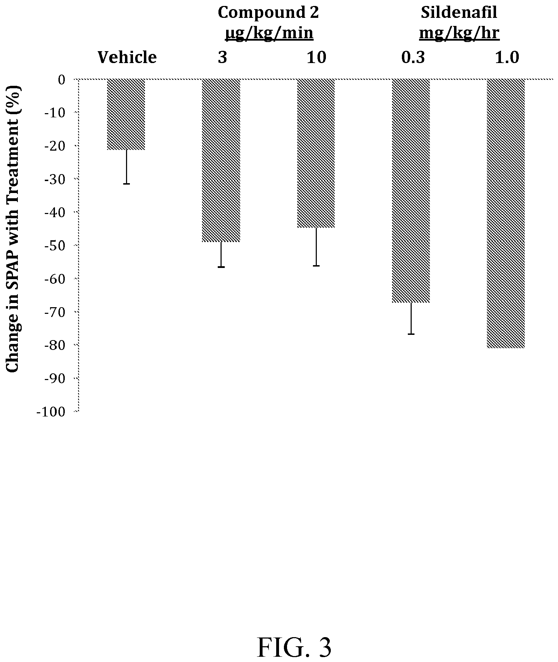

FIG. 3 shows the intravenous effects of a nitroxyl (HNO) donor on mean change in systolic pulmonary arterial pressure (SPAP) during hypoxic period relative to normoxic period compared to sildenafil citrate in dogs.

### Definitions

Unless clearly indicated otherwise, the following terms as used herein have the meanings indicated below.

"A", "an" and the like refers to one or more.

"Eq" or "equiv" or "equivalent" refers to molar equivalent.

"Hr" or "h" refers to hour.

"Min" or "m" refers to minute.

"Alkyl" intends linear hydrocarbon structures having 1 to 20 carbon atoms, 1 to 12 carbon atoms or 1 to 8 carbon atoms. Alkyl groups of fewer carbon atoms are embraced, such as so-called "lower alkyl" groups having 1 to 4 carbon atoms. "Alkyl" also intends branched or cyclic hydrocarbon structures having 3 to 20 carbon atoms, 3 to 12 carbon atoms and 3 to 8 carbon atoms. For any use of the term "alkyl," unless clearly indicated otherwise, it is intended to embrace all variations of alkyl groups disclosed herein, as measured by the number of carbon atoms, the same as if each and every alkyl group was explicitly and individually listed for each usage of the term. For instance, when a group such as R³ may be an "alkyl," intended is a C₁-C₂₀ alkyl or a C₁-C₁₂ alkyl or a C₁-C₈ alkyl or a lower alkyl or a C₂-C₂₀ alkyl or a C₃-C₁₂ alkyl or a C₃-C₈ alkyl. The same is true for other groups listed herein, which may include groups under other definitions, where a certain number of atoms is listed in the definition. When the alkyl group is cyclic, it may also be referred to as a cycloalkyl group and have, for example, 1 to 20 annular carbon atoms, 1 to 12 annular carbon atoms and 1 to 8 annular carbon atoms. When an alkyl residue having a specific number of carbons is named, all geometric isomers having that number of carbons are intended to be encompassed; thus, for example, "butyl" is meant to include n-butyl, sec-butyl, iso-butyl and t-butyl; "propyl" includes n-propyl and isopropyl. Examples of alkyl groups include methyl, ethyl, n-propyl, i-propyl, t-butyl, n-heptyl, octyl, cyclopentyl, cyclopropyl, cyclobutyl, norbornyl, and the like.

"Substituted alkyl" refers to an alkyl group having from 1 to 5 substituents. For instance, an alkyl group substituted with a group such as halo, nitro, cyano, oxo, aryl, alkoxy, acyl, acylamino, amino, hydroxyl, carboxyl, carboxylalkyl, thiol, thioalkyl, heterocyclyl, - OS(O)₂-alkyl, and the like is a substituted alkyl. Likewise, "substituted alkenyl" and "substituted alkynyl" refer to alkenyl or alkynyl groups having 1 to 5 substituents.

"Substituted" means that a hydrogen radical on a compound or group (such as, for example, alkyl, substituted alkyl, alkenyl, substituted alkenyl, alkynyl, substituted alkynyl, aryl, substituted aryl, aralkyl, substituted aralkyl, heteroaryl, substituted heteroaryl, heteroaralkyl, substituted heteroaralkyl, cycloalkyl, substituted cycloalkyl, heterocycloalkyl, substituted heterocycloalkyl, heterocyclyl and substituted heterocyclyl) is replaced with a group (the "substituent") that does not substantially adversely affect the stability of the compound. In some embodiments, the substituents are those which do not adversely affect the activity of a compound. The term "substituted" refers to one or more substituents (which may be the same or different), each replacing a hydrogen atom. Examples of substituents include, but are not limited to, halo (F, Cl, Br, or I), hydroxyl, amino, alkylamino, arylamino, dialkylamino, diarylamino, cyano, nitro, mercapto, oxo, carbonyl, thio, imino, formyl, carbamido, carbamyl, carboxyl, thioureido, thiocyanato, sulfoamido, sulfonylalkyl, sulfonylaryl, alkyl, alkenyl, alkoxy, mercaptoalkoxy, aryl, heteroaryl, cyclyl, heterocyclyl, wherein alkyl, alkenyl, alkyloxy, aryl, heteroaryl, cyclyl, and heterocyclyl are optionally substituted with alkyl, aryl, heteroaryl, halogen, hydroxyl, amino, mercapto, cyano, nitro, oxo (=O), thioxo (=S), or imino (=Nalkyl). In some embodiments, substituents on any group (such as, for example, alkyl, substituted alkyl, alkenyl, substituted alkenyl, alkynyl, substituted alkynyl, aryl, substituted aryl, aralkyl, substituted aralkyl, heteroaryl, substituted heteroaryl, heteroaralkyl, substituted heteroaralkyl, cycloalkyl, substituted cycloalkyl, heterocycloalkyl, substituted heterocycloalkyl, heterocyclyl and substituted heterocyclyl) are at any atom of that group (such as on a carbon atom of the primary carbon chain of a substituted alkyl group or on a substituent already present on a substituted alkyl group), wherein any group that can be substituted (such as, for example, alkyl, alkenyl, alkynyl, aryl, aralkyl, heteroaryl, heteroaralkyl, cycloalkyl, cyclyl, heterocycloalkyl, and heterocyclyl) can be optionally substituted with one or more substituents (which may be the same or different), each replacing a hydrogen atom. Examples of substituents include, but not limited to alkyl, alkenyl, alkynyl, cyclyl, cycloalkyl, heterocyclyl, heterocycloalkyl, aralkyl, heteroaralkyl, aryl, heteroaryl, halo, haloalkyl, cyano, nitro, alkoxy, aryloxy, hydroxyl, hydroxylalkyl, oxo, carbonyl, carboxyl, formyl, alkylcarbonyl, alkylcarbonylalkyl, alkoxycarbonyl, alkylcarbonyloxy, aryloxycarbonyl, heteroaryloxy, heteroaryloxycarbonyl, thio, mercapto, mercaptoalkyl, arylsulfonyl, amino, aminoalkyl, dialkylamino, alkylcarbonylamino, alkylaminocarbonyl, or alkoxycarbonylamino; alkylamino, arylamino, diarylamino, alkylcarbonyl, or arylamino-substituted aryl; arylalkylamino, aralkylaminocarbonyl, amido, alkylaminosulfonyl, arylaminosulfonyl, dialkylaminosulfonyl, alkylsulfonylamino, arylsulfonylamino, imino, carbamido, carbamyl, thioureido, thiocyanato, sulfoamido, sulfonylalkyl, sulfonylaryl, or mercaptoalkoxy. Additional examples of substituents include, without limitation, halo, CN, NO₂, OR¹¹, SR¹¹, S(O)₂OR¹¹, NR¹¹R¹², C₁-C₂perfluoroalkyl, C₁-C₂ perfluoroalkoxy, 1,2- methylenedioxy, (=O), (=S), (=NR¹¹), O(CH₂)ₙOR¹¹, C(O)R¹¹, C(O)OR¹¹, C(OR¹¹)R¹², C(O)NR¹¹R¹², OC(O)R¹³, OC(O)NR¹¹R¹², NR¹¹C(O)NR¹¹R¹², C(NR¹²)NR¹¹R¹², NR¹¹C(NR¹²)NR¹¹R¹², S(O)₂NR¹¹R¹²R¹³, C(O)H, C(O)R¹³, NR¹¹C(O)R¹³, NR¹¹C(O)OR¹³, Si(R¹¹)₃, OSi(R¹¹)₃, Si(OH)₂R¹¹, B(OH)₂, P(0)(OR¹¹)₂, S(O)R¹³, and S(O)₂R¹³. Each R¹¹ is independently hydrogen, C₁-C₆ alkyl optionally substituted with alkoxy, cycloalkyl, aryl, heterocyclyl, or heteroaryl. Each R¹² is independently hydrogen, C₃-C₆ cycloalkyl, aryl, heterocyclyl, heteroaryl, C₁-C₄ alkyl or C₁-C₄ alkyl substituted with C₃-C₆ cycloalkyl, aryl, heterocyclyl or heteroaryl. Each R¹³ is independently C₃-C₆ cycloalkyl, aryl, heterocyclyl, heteroaryl, C₁- C₄ alkyl or C₁-C₄ alkyl substituted with C₃-C₆ cycloalkyl, aryl, heterocyclyl or heteroaryl. Each C₃-C₆ cycloalkyl, aryl, heterocyclyl, heteroaryl and C₁-C₄ alkyl in each R¹¹, R¹² and R¹³ can optionally be substituted with halo, CN, C₁- C₄ alkyl, OH, C₁-C₄ alkoxy, COOH, C(O)OC₁-C₄ alkyl, NH₂, C₁-C₄ alkylamino, or C₁-C₄ dialkylamino. Each n is an integer from 1 to 6. Substituents can also be "electron-withdrawing groups."

"Alkenyl" refers to a group of 2 or more carbon atoms, such as 2 to 10 carbon atoms and 2 to 6 carbon atoms, and having at least one double bond. Examples of an alkenyl group include -C=CH2, -CH2CH=CHCH3 and -CH2CH=CH-CH=CH2.

"Alkynyl" refers to group having 2 or more carbon atoms, such as 2 to 10 carbon atoms and 3 to 6 carbon atoms, and having at least one triple bond, such as the moiety -C=CH.

"Heterocyclyl" or "heterocycloalkyl" refers to a cycloalkyl residue in which one to four of the carbons is replaced by a heteroatom such as oxygen, nitrogen or sulfur. Examples of heterocycles whose radicals are heterocyclyl groups include tetrahydropyran, morpholine, pyrrolidine, piperidine, thiazolidine, oxazole, oxazoline, isoxazole, dioxane, tetrahydrofuran and the like. A specific example of a heterocyclyl residue is tetrahydropyran-2-yl.

"Substituted heterocyclyl" or "substituted heterocylcoalkyl" refers to an heterocyclyl group having from 1 to 5 substituents. For instance, a heterocyclyl group substituted with 1 to 5 groups such as halo, nitro, cyano, oxo, aryl, alkoxy, alkyl, acyl, acylamino, amino, hydroxyl, carboxyl, carboxyalkyl, thiol, thioalkyl, heterocyclyl, -OS(O)₂-alkyl, and the like is a substituted alkyl. A particular example of a substituted heterocylcoalkyl is N-methylpiperazino.

"Aryl" refers to a monocyclic, bicyclic or tricyclic aromatic ring radical. In some embodiments, an aryl group is a 5- or 6-membered aromatic ring; a bicyclic 9- or 10-membered aromatic ring system (meaning the ring system has 9 or 10 annular atoms) or a tricyclic 13- or 14-membered aromatic ring system (meaning the ring system has 13 or 14 annular atoms). Examples of aryl radicals include, for example, phenyl, naphthalenyl, 1, tetralinyl.

"Substituted aryl" refers to a group having from 1 to 3 substituents. For instance, an aryl group substituted with 1 to 3 groups, such as halo, nitro, cyano, oxo, aryl, alkoxy, alkyl, acyl, acylamino, amino, hydroxyl, carboxyl, carboxylalkyl, thiol, thioalkyl, heterocyclyl, - OS(O)₂-alkyl and the like, is a substituted aryl.

"Aralkyl" refers to a residue in which an aryl moiety is attached to the parent structure via an alkyl residue. Examples include benzyl (-CH2-Ph), phenethyl (-CH₂CH₂Ph), phenylvinyl (-CH=CH-Ph), phenylallyl and the like.

"Heteroaryl" refers to an aromatic ring system having at least one annular heteroatom selected from O, N, or S. An heteroaryl group is preferably a 5- or 6-membered aromatic ring containing 1-3 annular heteroatoms selected from O, N, or S; a bicyclic 9- or 10-membered aromatic ring system (meaning the ring system has 9 or 10 annular atoms) containing 1-3 annular heteroatoms selected from O, N, or S; or a tricyclic 13- or 14-membered aromatic ring system (meaning the ring system has 13 or 14 annular atoms) containing 1-3 annular heteroatoms selected from O, N, or S. Examples of groups whose radicals are heteroaryl groups include *e.g.,* imidazole, pyridine, indole, thiophene, benzopyranone, thiazole, furan, benzimidazole, benzoxazole, benzthiazole, quinoline, isoquinoline, quinoxaline, pyrimidine, pyrazine, tetrazole and pyrazole.

"Alkoxy" refers to an alkyl group that is connected to the parent structure through an oxygen atom (-O-alkyl). When a cycloalkyl group is connected to the parent structure through an oxygen atom, the group may also be referred to as a cycloalkoxy group. Examples include methoxy, ethoxy, propoxy, isopropoxy, cyclopropyloxy, cyclohexyloxy and the like. When the cylcoalkyl group contains one or more heteroatoms, the group may also be referred to as "heterocycloalkoxy" group. Examples of heteroatoms include O, S, N, P, Se, Si and the like. A "perhaloalkoxy" intends a perhaloalkyl group attached to the parent structure through an oxygen, such as the residue -O-CF₃.

"Aryloxy" refers to an aryl group that is connected to the parent structure through an oxygen atom (-O-aryl), which by way of example includes the residues phenoxy, naphthoxy, and the like. "Substituted aryloxy" refers to a substituted aryl group connected to the parent structure through an oxygen atom (-O-substituted aryl).

"Electron withdrawing group" refers to a group that reduces electron density of the moiety to which it is attached (relative to the density of the moiety without the substituent). Examples include, without limitation, F, Cl, Br, I, -CN, -CF₃, -NO₂, -SH, -C(O)H, -C(O)alkyl,-C(O)Oalkyl, -C(O)OH, -C(O)Cl, -S(O)₂OH, -S(O)₂NHOH, -NH₃ and the like.

"Halo" refers to fluoro, chloro, bromo or iodo.

"Alkylsulfonyl" refers to groups -S02alkyl and -SO₂substituted alkyl, which includes the residues -S02cycloalkyl, -SO₂substituted cycloalkyl, -SO₂alkenyl, -SO₂substituted alkenyl,-S02alkynyl, -SO₂substituted alkynyl, where alkyl, substituted alkyl, alkenyl, substituted alkenyl, alkynyl, substituted alkynyl, cycloalkyl and substituted cycloalkyl are as defined herein.

"N-hydroxylsulfonamidyl" refers to -S(O)₂NROH, where R is H or alkyl.

"Perhaloalkyl" refers to an alkyl group where each H of the hydrocarbon is replaced with F. Examples of perhalo groups include -CF₃ and -CF₂CF₃.

"Alkylsulfanyl" refers to an alkyl group that is connected to the parent structure through a sulfur atom (-S-alkyl) and refers to groups -S-alkyl and -S-substituted alkyl, which include the residues -S-cycloalkyl, -S-substituted cycloalkyl, -S-alkenyl, -S-substituted alkenyl, -S-alkynyl, and -S-substituted alkynyl, where alkyl, substituted alkyl, alkenyl, substituted alkenyl, alkynyl, substituted alkynyl, cycloalkyl and substituted cycloalkyl are as defined herein. When a cycloalkyl group is connected to the parent structure through an sulfur atom, the group may also be referred to as a cycloalkylsulfanyl group. By way of example, alkylsulfanyl includes-S-CH(CH₃), -S-CH₂CH₃ and the like.

"Alkylsulfinyl" refers to an alkyl group that is connected to the parent structure through a S(O) moiety and refers to groups -S(O)alkyl and -S(O)substituted alkyl, which includes the residues -S(O)cycloalkyl, -S(O)substituted cycloalkyl, -S(O)alkenyl, -S(O)substituted alkenyl,-S(O)alkynyl, -S(O)substituted alkynyl, where alkyl, substituted alkyl, alkenyl, substituted alkenyl, alkynyl, substituted alkynyl, cycloalkyl and substituted cycloalkyl are as defined herein. By way of example, alkylsulfinyl includes the residues -S(O)CH(CH₃), -S(O)CH₃,-S(O)cyclopentane and the like.

"Arylsulfinyl" refers to an aryl group that is connected to the parent structure through a S(O) moiety, which by way of example includes the residue -S(O)Ph.

"Acyl" refers to and includes the groups -C(O)H, -C(O)alkyl, -C(O)substituted alkyl, - C(O)alkenyl, -C(O)substituted alkenyl, -C(O)alkynyl, -C(O)substituted alkynyl, - C(O)cycloalkyl, -C(O)substituted cycloalkyl, -C(O)aryl, -C(O)substituted aryl, - C(O)heteroaryl, -C(O)substituted heteroaryl, -C(O)heterocyclic, and -C(O)substituted heterocyclic wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic and substituted heterocyclic are as defined herein or otherwise known in the art.

"Dialkylamino" refers to the group -NR₂ where each R is an alkyl group. Examples of dialkylamino groups include -N(CH₃)₂, -N(CH₂CH₂CH₂CH₃)₂, and N(CH₃)(CH₂CH₂CH₂CH₃).

"Optional" or "optionally" means that the subsequently described event or circumstance may or may not occur, and that the description includes instances where the event or circumstance occurs and instances in which it does not. For example, an alkyl that is "optionally substituted" encompasses both an alkyl that is unsubstituted and an alkyl that is substituted.

"Pharmaceutically acceptable" refers to those properties and/or substances that are acceptable to the patient from a pharmacological and/or toxicological point of view, and/or to the manufacturing pharmaceutical chemist from a physical and/or chemical point of view regarding composition, formulation, stability, patient acceptance, bioavailability and compatibility with other ingredients.

"Pharmaceutically acceptable salt" refers to pharmaceutically acceptable salts of a compound described herein, such as a compound of formula (I), (II), (Ia) or (IIa) or other nitroxyl donors, which salts may be derived from a variety of organic and inorganic counter ions well known in the art and include, by way of example, sodium, potassium, calcium, magnesium, ammonium, tetraalkylammonium, and the like; when the molecule contains a basic functionality, salts of organic or inorganic acids, such as hydrochloride, hydrobromide, tartrate, mesylate, acetate, maleate, oxalate and the like. Illustrative salts include, but are not limited, to sulfate, citrate, acetate, chloride, bromide, iodide, nitrate, bisulfate, phosphate, acid phosphate, lactate, salicylate, acid citrate, tartrate, oleate, tannate, pantothenate, bitartrate, ascorbate, succinate, maleate, besylate, fumarate, gluconate, glucaronate, saccharate, formate, benzoate, glutamate, methanesulfonate, ethanesulfonate, benzenesulfonate, and p- toluenesulfonate salts. Accordingly, a salt may be prepared from a compound of any one of the formulae disclosed herein having an acidic functional group, such as a carboxylic acid functional group, and a pharmaceutically acceptable inorganic or organic base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxysubstituted mono-, di-, or trialkylamines; dicyclohexylamine; tributyl amine; pyridine; N-methyl,N-ethylamine; diethylamine; triethylamine; mono-, bis-, or tris-(2-hydroxy- lower alkyl amines), such as mono-, bis-, or tris-(2-hydroxyethyl)amine, 2-hydroxy- tert-butylamine, or tris-(hydroxymethyl)methylamine, N,N,-di-lower alkyl-N-(hydroxy lower alkyl)-amines, such as N,N-dimethyl-N-(2-hydroxyethyl) amine, or tri-(2-hydroxyethyl)amine; N-methyl-D-glucamine; and amino acids such as arginine, lysine, and the like. A salt may also be prepared from a compound of any one of the formulae disclosed herein having a basic functional group, such as an amino functional group, and a pharmaceutically acceptable inorganic or organic acid. Suitable acids include hydrogen sulfate, citric acid, acetic acid, hydrochloric acid (HCl), hydrogen bromide (HBr), hydrogen iodide (HI), nitric acid, phosphoric acid, lactic acid, salicylic acid, tartaric acid, ascorbic acid, succinic acid, maleic acid, besylic acid, fumaric acid, gluconic acid, glucaronic acid, formic acid, benzoic acid, glutamic acid, methanesulfonic acid, ethanesulfonic acid, benzenesulfonic acid, and p-toluenesulfonic acid.

"Pharmaceutically acceptable excipient" refers to any substance, not itself a therapeutic agent, used as a carrier, diluent, adjuvant, binder, and/or vehicle for delivery of a therapeutic agent to a patient, or added to a pharmaceutical composition to improve its handling or storage properties or to permit or facilitate formation of a compound or composition into a unit dosage form for administration. Pharmaceutically acceptable excipients are well known in the pharmaceutical arts and are described, for example, in Remington's Pharmaceutical Sciences, Mack Publishing Co., Easton, Pa (e.g., 20th Ed., 2000), and Handbook of Pharmaceutical Excipients, American Pharmaceutical Association, Washington, D.C., (*e.g.,* 1^{st}, 2^{nd} and 3rd Eds., 1986, 1994 and 2000, respectively). As will be known to those skilled in the art, pharmaceutically acceptable excipients may provide a variety of functions and may be described as wetting agents, buffering agents, suspending agents, lubricating agents, emulsifiers, disintegrants, absorbents, preservatives, surfactants, colorants, flavorants, and sweeteners. Examples of pharmaceutically acceptable excipients include without limitation: (1) sugars, such as lactose, glucose and sucrose; (2) starches, such as corn starch and potato starch; (3) cellulose and its derivatives, such as sodium carboxymethyl cellulose, ethyl cellulose, cellulose acetate, hydroxypropylmethylcellulose, and hydroxypropylcellulose; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

"Unit dosage form" refers to a physically discrete unit suitable as a unitary dosage for human or other animal patients. Each unit dosage form may contain a predetermined amount of an active substance (*e.g.*, a compound of formula (I), (II), (Ia) or (IIa) calculated to produce a desired effect.

Unless clearly indicated otherwise, an "individual" or "patient" refers to an animal, such as a mammal, including but not limited, to a human. Hence, the methods described herein can be useful in human therapy and veterinary applications. In some embodiments, the individual or patient is a mammal. In some embodiments, the individual or patient is a human.

"Effective amount" refers to such amount of a compound or a pharmaceutically acceptable salt thereof, which in combination with its parameters of efficacy and toxicity, as well as based on the knowledge of the practicing specialist should be effective in a given therapeutic form. As is understood in the art, an effective amount may be in one or more doses.

"Treatment" or "treating" is an approach for obtaining a beneficial or desired result, including clinical results. For purposes of this invention, beneficial or desired results include but are not limited to inhibiting and/or suppressing the onset and/or development of a disease or condition or reducing the severity of such disease or condition, such as reducing the number and/or severity of symptoms associated with the disease or condition, increasing the quality of life of those suffering from the disease or condition, decreasing the dose of other medications required to treat the disease or condition, enhancing the effect of another medication an individual is taking for the disease or condition, and prolonging survival of individuals having the disease or condition.

"Preventing" refers to reducing the probability of developing a disorder or condition in an individual who does not have, but is at risk of developing a disorder or condition. An individual "at risk" may or may not have a detectable disease or condition, and may or may not have displayed a detectable disease or condition prior to the treatment methods described herein. "At risk" denotes that an individual has one or more so-called risk factors, which are measurable parameters that correlate with development of a disease or condition and are known in the art. An individual having one or more of these risk factors has a higher probability of developing the disease or condition than an individual without these risk factor(s).

"Nitroxyl" refers to the species HNO.
"Nitroxyl donor" or "HNO donor" refers to a compound that donates nitroxyl under physiological conditions. As used herein, nitroxyl donors may alternatively be referred to as "a compound" or "the compound." In some embodiments, the nitroxyl donor is capable of donating an effective amount of nitroxyl in vivo and has a safety profile indicating the compound would be tolerated by an individual in the amount necessary to achieve a therapeutic effect. One of ordinary skill in the art would be able to determine the safety of administering particular compounds and dosages to live subjects. One of skill in the art may also determine whether a compound is a nitroxyl donor by evaluating whether it releases HNO under physiological conditions. Compounds are easily tested for nitroxyl donation with routine experiments. Although it is impractical to directly measure whether nitroxyl is donated, several tests are accepted for determining whether a compound donates nitroxyl. For example, the compound of interest can be placed in solution, for example in phosphate buffered saline (PBS) or phosphate buffered solution at a pH of about 7.4, in a sealed container. After sufficient time for disassociation has elapsed, such as from several minutes to several hours, the headspace gas is withdrawn and analyzed to determine its composition, such as by gas chromatography and/or mass spectroscopy. If the gas N₂O is formed (which occurs by HNO dimerization), the test is positive for nitroxyl donation and the compound is a nitroxyl donor. The level of nitroxyl donating ability may be expressed as a percentage of a compound's theoretical maximum. A compound that donates a "significant level of nitroxyl" intends a compound that donates 40 % or more or 50 % or more of its theoretical maximum amount of nitroxyl. In some embodiments, the compounds for use herein donate 60 % or more of the theoretical maximum amount of nitroxyl. In some embodiments, the compounds for use herein donate 70 % or more of the theoretical maximum amount of nitroxyl. In some embodiments, the compounds for use herein donate 80% or more of the theoretical maximum amount of nitroxyl. In some embodiments, the compounds for use herein donate 90% or more of the theoretical maximum amount of nitroxyl. In some embodiments, the compounds for use herein donate between about 70% and about 90% of the theoretical maximum amount of nitroxyl. In some embodiments, the compounds for use herein donate between about 85 % and about 95 % of the theoretical maximum amount of nitroxyl. In some embodiments, the compounds for use herein donate between about 90 % and about 95 % of the theoretical maximum amount of nitroxyl. Compounds that donate less than 40% or less than 50 % of their theoretical amount of nitroxyl are still nitroxyl donors and may be used in the invention disclosed herein. A compound that donates less than 50 % of the theoretical amount of nitroxyl may be used in the methods described, and may require higher dosing levels as compared to compounds that donate a significant level of nitroxyl. Nitroxyl donation also can be detected by exposing the test compound to metmyoglobin (Mb³⁺). Nitroxyl reacts with Mb³⁺ to form an Mb²⁺-NO complex, which can be detected by changes in the ultraviolet/visible spectrum or by Electron Paramagnetic Resonance (EPR). The Mb²⁺-NO complex has an EPR signal centered around a g-value of about 2. Nitric oxide, on the other hand, reacts with Mb³⁺ to form an Mb³⁺-NO complex that is EPR silent. Accordingly, if the candidate compound reacts with Mb³⁺ to form a complex detectable by common methods, such as ultraviolet/visible or EPR, then the test is positive for nitroxyl donation. Testing for nitroxyl donation may be performed at physiologically relevant pH. Examples of nitroxyl donors include, without limitation, sodium dioxotrinitrate ("Angeli's salt" or "AS"), *N*-hydroxybenzenesulfonamide ("Piloty's acid" or "PA"), and the compounds disclosed in US Patent No. 6,936,639, US Patent Publication Nos. 2004/0038947, 2007/0299107 and 2009/0163487, and PCT Publication Nos. WO/2007/002444, WO/2005/074598 and WO/2009/137717.

"Positive inotrope" refers to an agent that causes an increase in myocardial contractile function. Such an agent includes a beta-adrenergic receptor agonist, an inhibitor of phosphodiesterase activity, and calcium-sensitizers. Beta-adrenergic receptor agonists include, among others, dopamine, dobutamine, terbutaline, and isoproterenol. Analogs and derivatives of such compounds are also intended. For example, U.S. Pat. No. 4,663,351 describes a dobutamine prodrug that can be administered orally. One of ordinary skill in the art would be able to determine if a compound is capable of causing positive inotropic effects and also additional *beta*-agonist compounds. In particular embodiments, the *beta-*receptor agonist is selective for the *beta*-1 receptor. In other embodiments the *beta*-agonist is selective for the *beta*-2 receptor, or is not selective for any particular receptor.

Diseases or conditions that are "responsive to nitroxyl therapy" includes any disease or condition in which administration of a compound that donates an effective amount of nitroxyl under physiological conditions treats and/or prevents the disease or condition, as those terms are defined herein. A disease or condition whose symptoms are suppressed or diminished upon administration of nitroxyl donor is a disease or condition responsive to nitroxyl therapy. Non- limiting examples of diseases or conditions that are responsive to nitroxyl therapy include coronary obstructions, coronary artery disease (CAD), angina, heart attack, myocardial infarction, high blood pressure, ischemic cardiomyopathy and infarction, diastolic heart failure, pulmonary congestion, pulmonary edema, cardiac fibrosis, valvular heart disease, pericardial disease, circulatory congestive states, peripheral edema, ascites, Chagas' disease, ventricular hypertrophy, heart valve disease, heart failure, including but not limited to congestive heart failure such as acute congestive heart failure and acute decompensated heart failure. Other cardiovascular diseases or conditions are also intended, as are diseases or conditions that implicate ischemia/reperfusion injury. Cancer is another example of disease or condition that is responsive to nitroxyl therapy.

"Pulmonary hypertension" or "PH" refers to a condition in which the pulmonary arterial pressure is elevated. The current haemodynamic definition of PH is a mean pulmonary arterial pressure (MPAP) at rest of greater than or equal to 25 mmHg.² Examples of PH include, but are not limited to, the conditions listed in the updated classification of PH (Table 1).³
² Badesch D. et al. Diagnosis and assessment of pulmonary arterial hypertension. J Am Coll Cardiol 2009; 54(Suppl.): S55-S66.
³ Simonneau G. et al. Updated clinical classification of pulmonary hypertension. JAm Coll Cardiol 2009; 54(1 Suppl): S43-54.

**Table 1. Classification of Pulmonary Hypertension (PH):**

| |
|---|
| 1. Pulmonary artery hypertension (PAH) |
| ∘ 1.1. Idiopathic PAH |
| ∘ 1.2. Heritable |
| ▪ 1.2.1. BMPR2 |
| ▪ 1.2.2. ALK1, endoglin (with or without hereditary hemorrhagic telangiectasia |
| ▪ 1.2.3. Unknown |
| ∘ 1.3. Drug- and toxin-induced |
| ∘ 1.4. Associated with: |
| ▪ 1.4.1. Connective tissue diseases |
| ▪ 1.4.2. Human immunodeficiency virus (HIV) infection |
| ▪ 1.4.3. Portal hypertension |
| ▪ 1.4.4. Congenital heart diseases |
| ▪ 1.4.5. Schistosomiasis |
| ∘ 1.5 Persistent pulmonary hypertension of the newborn |
| ∘ 1'. Pulmonary veno-occlusive disease (PVOD) and/or pulmonary capillary hemangiomatosis (PCH) |
| 2. Pulmonary hypertension owing to left heart disease |
| ∘ 2.1. Systolic dysfunction |
| ∘ 2.2. Diastolic dysfunction |
| ∘ 2.3. Valvular disease |
| 3. Pulmonary hypertension owing to lung disease and/or hypoxemia |
| ∘ 3.1. Chronic obstructive pulmonary disease |
| ∘ 3.2. Interstitial lung disease |
| ∘ 3.3. Other pulmonary diseases with mixed restrictive and obstructive pattern |
| ∘ 3.4. Sleep-disordered breathing |
| ∘ 3.5. Alveolar hypoventilation disorders |
| ∘ 3.6. Chronic exposure to high altitude |
| ∘ 3.7. Developmental abnormalities |
| 4. Chronic thromboembolic pulmonary hypertension (CTEPH) |
| 5. Pulmonary hypertension with unclear multifactorial mechanisms |
| ∘ 5.1. Hematologic disorders: myeoloproliferative disorders, splenectomy |
| ∘ 5.2. Systemic disorders: sarcoidosis, pulmonary Langerhans cell histiocytosis: lymphangioleiomyomatosis, neurofibromatosis, vasculitis |
| ∘ 5.3. Metabolic disorders: glycogen storage disease, Gaucher disease, thyroid disorders |
| ∘ 5.4. Others: tumoral obstruction, fibrosing mediastinitis, chronic renal failure on dialysis. |

The invention relates to certain N-acyloxysulfonamide derivative compounds, methods of using such compounds, and pharmaceutical compositions and kits comprising such compounds. In particular, the present invention provides the following embodiments [1] to [10].
[1] A compound of formula (I) 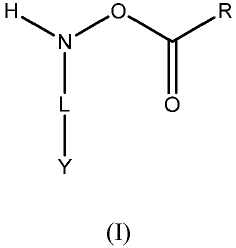 or a pharmaceutically acceptable salt, hydrate, or solvate thereof wherein:
   R is hydrogen, alkyl, heterocycloalkyl, aryl, benzyl, alkoxy, heterocycloalkoxy, aryloxy, benzyloxy, -NR³R⁴ or -N(OR³)R⁴, wherein said alkyl, heterocycloalkyl, aryl, benzyl, alkoxy, heterocycloalkoxy, aryloxy and benzyloxy are unsubstituted or substituted with one or more substituents;
   R³ and R⁴ are independently alkyl, heterocycloalkyl or aryl, wherein said alkyl, heterocycloalkyl and aryl are unsubstituted or substituted with one or more substituents;
   L is SO₂,
      Y is aryl and said aryl is unsubstituted or substituted with one or more substituents independently selected from W, and
      W is halo, -OH, -CN, -NO₂, -COR¹, -COOR¹, -CONR¹R², -CH(C(O)R¹)₂, -SO₂R¹, or -COX, wherein X is halo and R¹ and R² are independently alkyl or aryl, or R¹ and R² are taken together to form a cycloalkyl or heterocycloalkyl, wherein said cycloalkyl and heterocycloalkyl are unsubstituted or substituted with one or more substituents;
   and where the substituents which may be present when the above groups include said one or more substituents are the same or different and are selected from halo, hydroxyl, amino, alkylamino, arylamino, dialkylamino, diarylamino, cyano, nitro, mercapto, oxo, carbonyl, thio, imino, formyl, carbamido, carbamyl, carboxyl, thioureido, thiocyanato, sulfoamido, sulfonylalkyl, sulfonylaryl, alkyl, alkenyl, alkoxy, mercaptoalkoxy, aryl, heteroaryl, cyclyl, heterocyclyl, wherein alkyl, alkenyl, alkyloxy, aryl, heteroaryl, cyclyl, and heterocyclyl are optionally substituted with alkyl, aryl, heteroaryl, halogen, hydroxyl, amino, mercapto, cyano, nitro, oxo, thioxo, or imino;
   said compound, salt, hydrate, or solvate being for use in a method of treating a disease or condition selected from cardiovascular diseases, ischemia, reperfusion injury, cancerous disease and pulmonary hypertension.
[2] The compound, salt, hydrate or solvante of [1] for use as defined in [1] wherein W is chloro, bromo or -SO₂R¹.
[3] The compound, salt, hydrate or solvate of [1] for a use as defined in [1], wherein R is alkyl or phenyl, wherein said alkyl and phenyl are unsubstituted or substituted with one or more halos.
[4] The compound, salt, hydrate or solvate of [1] for a use as defined in [1], which compound is selected from:
   N-acetyloxy-benzenesulfonamide;
   N-benzoyloxy-benzenesulfonamide;
   N-(trifluoroacetyloxy)-benzenesulfonamide;
   *N*-(acetyloxy)-2-bromobenzenesulfonamide;
   *N*-acetyloxy-2,6-dichlorobenzenesulfonamide; and
   pharmaceutically acceptable salts, hydrates and solvates thereof.
[5] A compound, salt, hydrate or solvate of any one of [1] to [4] for a use as defined in [1], wherein the disease or condition is a cardiovascular disease.
[6] A compound, salt, hydrate or solvate of [5] for a use as defined in [5], wherein the cardiovascular disease is heart failure.
[7] A compound, salt, hydrate or solvate of [6] for a use as defined in [6], wherein the heart failure is congestive heart failure, acute congestive heart failure, or acute decompensated heart failure.
[8] A compound, salt, hydrate or solvate of any one of [1] to [4] for a use as defined in [1], wherein the disease or condition is ischemia or reperfusion injury.
[9] A compound, salt, hydrate or solvate of any one of [1] to [4] for a use as defined in [1], wherein the disease or condition is a cancerous disease.
[10] A compound, salt, hydrate or solvate of any one of [1] to [4] for a use as defined in [1], wherein the disease or condition is pulmonary hypertension.

### Compounds

The compound, salt, hydrate or solvate for use in the invention is a compound of formula (I) 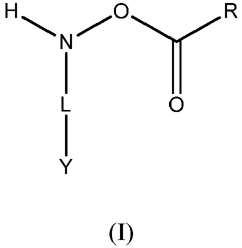 or a pharmaceutically acceptable salt, hydrate, or solvate thereof wherein:
R is hydrogen, alkyl, heterocycloalkyl, aryl, benzyl, alkoxy, heterocycloalkoxy, aryloxy, benzyloxy, -NR³R⁴ or -N(OR³)R⁴, wherein said alkyl, heterocycloalkyl, aryl, benzyl, alkoxy, heterocycloalkoxy, aryloxy and benzyloxy are unsubstituted or substituted with one or more substituents;
R³ and R⁴ are independently alkyl, heterocycloalkyl or aryl, wherein said alkyl, heterocycloalkyl and aryl are unsubstituted or substituted with one or more substituents;
L is SO₂,
   Y is aryl and said aryl is unsubstituted or substituted with one or more substituents independently selected from W, and
   W is halo, -OH, -CN, -NO₂, -COR¹, -COOR¹, -CONR¹R², -CH(C(O)R¹)₂, -SO₂R¹, or -COX, wherein X is halo and R¹ and R² are independently alkyl or aryl, or R¹ and R² are taken together to form a cycloalkyl or heterocycloalkyl, wherein said cycloalkyl and heterocycloalkyl are unsubstituted or substituted with one or more substituents;
and where the substituents which may be present when the above groups include said one or more substituents are the same or different and are selected from halo, hydroxyl, amino, alkylamino, arylamino, dialkylamino, diarylamino, cyano, nitro, mercapto, oxo, carbonyl, thio, imino, formyl, carbamido, carbamyl, carboxyl, thioureido, thiocyanato, sulfoamido, sulfonylalkyl, sulfonylaryl, alkyl, alkenyl, alkoxy, mercaptoalkoxy, aryl, heteroaryl, cyclyl, heterocyclyl, wherein alkyl, alkenyl, alkyloxy, aryl, heteroaryl, cyclyl, and heterocyclyl are optionally substituted with alkyl, aryl, heteroaryl, halogen, hydroxyl, amino, mercapto, cyano, nitro, oxo, thioxo, or imino.

Also disclosed herein, for reference purposes, is a compound of formula (I') 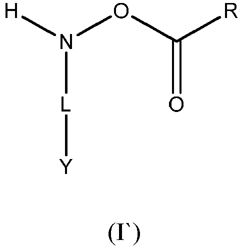 or a pharmaceutically acceptable salt, hydrate, or solvate thereof wherein:
L is a bond, -SO₂- or -O-;
Y is W, alkyl or aryl, wherein said alkyl and aryl are unsubstituted or substituted with one or more substituents independently selected from W;
W is halo, -OH, -CN, -NO₂, -COR¹, -COOR¹, -CONR¹R², -CH(C(O)R¹)₂, -SO₂R¹, or-COX, wherein X is halo, and R¹ and R² are independently alkyl or aryl, or R¹ and R² are taken together to form a cycloalkyl or heterocycloalkyl, wherein said cycloalkyl and heterocycloalkyl are unsubstituted or substituted with one or more substituents;
R is hydrogen, alkyl, heterocycloalkyl, aryl, benzyl, alkoxy, heterocycloalkoxy, aryloxy, benzyloxy, -NR³R⁴ or -N(OR³)R⁴, wherein said alkyl, heterocycloalkyl, aryl, benzyl, alkoxy, heterocycloalkoxy, aryloxy and benzyloxy are unsubstituted or substituted with one or more substituents; and
R³ and R⁴ are independently alkyl, heterocycloalkyl or aryl, wherein said alkyl, heterocycloalkyl and aryl are optionally substituted with one or more substituents.

In some aspects of formula (I'):
L is a bond, -SO₂- or -O-;
Y is W, alkyl or aryl, wherein said alkyl and aryl are unsubstituted or substituted with one or more substituents independently selected from W;
W is halo, -OH, -CN, -NO₂, -COR¹, -COOR¹, -CONR¹R², -CH(C(O)R¹)₂, -SO₂R¹, or-COX, wherein X is halo, and R¹ and R² are independently alkyl or aryl, or R¹ and R² are taken together to form a cycloalkyl or heterocycloalkyl, wherein said cycloalkyl and heterocycloalkyl are unsubstituted or substituted with one or more substituents;
R is hydrogen, alkyl, heterocycloalkyl, aryl, benzyl, alkoxy, heterocycloalkoxy, aryloxy, benzyloxy or -NR³R⁴, wherein said alkyl, heterocycloalkyl, aryl, benzyl, alkoxy, heterocycloalkoxy, aryloxy and benzyloxy are unsubstituted or substituted with one or more substituents; and
R³ and R⁴ are independently alkyl or aryl.

In some aspects, when L is -SO₂- and R is methyl, then Y is not phenyl; and when W is -OH, then L is a bond.

Included in any of the aspectsdisclosed above are the following additional aspectswhich may be combined in any variation.

In some aspects, L is -SO₂-.

In some aspects, Y is aryl and said aryl is unsubstituted or substituted with one or more substituents independently selected from W.

In some aspects, Y is aryl and said aryl is unsubstituted or substituted with one or two substituents independently selected from W.

In some aspects, Y is phenyl and said phenyl is unsubstituted or substituted with one or more substituents independently selected from W.

In some aspects, W is halo or -SO₂.

In some aspects, W is chloro, bromo or -SO₂.

In some aspects, R is hydrogen, alkyl, aryl, benzyl, alkoxy, aryloxy, benzyloxy, -NR³R⁴ or -N(OR³)R⁴, wherein said alkyl, aryl, benzyl, alkoxy, aryloxy and benzyloxy are unsubstituted or substituted with one or more substituents independently selected from halo, nitro, alkylsulfonyl, trihalomethyl, alkyl, hydroxy, heteroaryl, alkyl, -NR¹¹R¹², -NR¹¹C(O)R¹³,-NR¹¹C(O)OR¹³, -C(O)OR¹¹, -C(O)R¹¹, -C(O)NR¹¹R¹², -OC(O)R¹¹, -O(CH₂)ₙOR¹¹, heterocycloalkyl optionally substituted with alkyl, heterocycloalkylalkoxy substituted with C(O)R¹¹, and alkoxy optionally substituted with alkoxy or with -O(CH₂)ₙOR¹¹.

In some aspects, R is hydrogen, alkyl, aryl, benzyl, alkoxy, aryloxy, benzyloxy or -NR³R⁴, wherein said alkyl, aryl, benzyl, alkoxy, aryloxy and benzyloxy are unsubstituted or substituted with one or more substituents independently selected from halo, nitro, alkylsulfonyl and trihalomethyl.

In some aspects, R is hydrogen, alkyl, aryl, benzyl, alkoxy, aryloxy, benzyloxy or -NR³R⁴, wherein said alkyl, aryl, benzyl, alkoxy, aryloxy and benzyloxy are unsubstituted or substituted with one or more substituents independently selected from halo, nitro, alkylsulfonyl and trihalomethyl.

In some aspects, R is alkyl or phenyl, wherein said alkyl and phenyl are unsubstituted or substituted with one or more halos.

In some aspects, Y is alkyl and said alkyl is unsubstituted or substituted with one or more substituents independently selected from W.

In some aspects, Y is alkyl and said alkyl is unsubstituted or substituted with one or more halos.

In some aspects, R is alkyl or phenyl, wherein said alkyl and phenyl are unsubstituted or substituted with one or more substituents independently selected from halo, nitro, alkylsulfonyl, trihalomethyl, -OC(O)R¹¹, and heteroaryl optionally substituted with oxo or phenyl.

In some aspects, R is alkyl or phenyl, wherein said alkyl and phenyl are unsubstituted or substituted with one or more substituents independently selected from halo, nitro, alkylsulfonyl and trihalomethyl.

In some aspects, Y is phenyl substituted with halo and -CONR¹R².

In some aspects, R¹ and R² are taken together to form a cycloalkyl.

In some aspects, R¹ and R² are taken together to form a cycloalkyl wherein said cycloalkyl is substituted with one or more substituents.

In some aspects, R¹ and R² are taken together to form a heterocycloalkyl.

In some aspects, R¹ and R² are taken together to form a heterocycloalkyl wherein said heterocycloalkyl is substituted with one or more substituents.

In some aspects, R³ and R⁴ are alkyl, heterocycloalkyl or aryl, wherein said alkyl heterocycloalkyl and aryl are optionally substituted with carboxy, alkoxy, heteroaryl and oxo.

Also disclosed herein, for reference purposes, is a compound of formula (II) 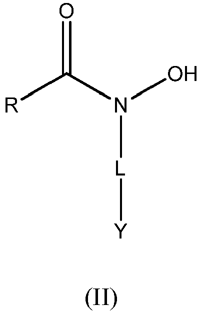 or a pharmaceutically acceptable salt, hydrate, or solvate thereof wherein:
L is a bond, -SO₂- or -O-;
Y is W, alkyl or aryl, wherein said alkyl and aryl are unsubstituted or substituted with one or more substituents independently selected from W;
W is halo, -OH, -CN, -NO₂, -COR¹, -COOR¹, -CONR¹R², -CH(C(O)R¹)₂, -SO₂R¹ or-COX, wherein X is halo, R¹ and R² are independently alkyl or aryl, or R¹ and R² are taken together to form a cycloalkyl or a heterocycloalkyl, wherein said cycloalkyl and heterocycloalkyl are unsubstituted or substituted with one or more substituents;
R is hydrogen, alkyl, heterocycloalkyl, aryl, benzyl, alkoxy, heterocycloalkoxy, aryloxy, benzyloxy or -NR³R⁴, wherein said alkyl, heterocycloalkyl, aryl, benzyl, alkoxy, heterocycloalkoxy, aryloxy and benzyloxy are unsubstituted or substituted with one or more substituents; and
R³ and R⁴ are independently alkyl or aryl.

In some aspects, when W is -OH, then L is a bond.

Included in any of the aspectsdisclosed above are the following additional aspectswhich may be combined in any variation.

In some aspects, L is -SO₂-.

In some aspects, Y is aryl and said aryl is unsubstituted or substituted with one or more substituents independently selected from W.

In some aspects, Y is aryl and said aryl is unsubstituted or substituted with one or two substituents independently selected from W.

In some aspects, Y is phenyl and said phenyl is unsubstituted or substituted with one or more substituents independently selected from W.

In some aspects, W is halo or -SO₂.

In some aspects, W is chloro, bromo or -SO₂.

In some aspects, R is hydrogen, alkyl, heterocycloalkyl, aryl, benzyl, alkoxy, aryloxy, benzyloxy or -NR³R⁴, wherein said alkyl, heterocycloalkyl, aryl, benzyl, alkoxy, aryloxy and benzyloxy are unsubstituted or substituted with one or more substituents independently selected from halo, nitro, alkylsulfonyl and trihalomethyl.

In some aspects, R is alkyl, heterocycloalkyl, or phenyl, wherein said alkyl, heterocycloalkyl and phenyl are unsubstituted or substituted with one or more halos.

In some aspects, Y is alkyl and said alkyl is unsubstituted or substituted with one or more substituents independently selected from W.

In some aspects, Y is alkyl and said alkyl is unsubstituted or substituted with one or more halos.

In some aspects, R is alkyl, heterocycloalkyl or phenyl, wherein said alkyl, heterocycloalkyl and phenyl are unsubstituted or substituted with one or more substituents independently selected from halo, nitro, alkylsulfonyl and trihalomethyl.

In some aspects, Y is phenyl substituted with halo and -CONR¹R².

In some aspects, R¹ and R² are taken together to form a cycloalkyl.

In some aspects, R¹ and R² are taken together to form a cycloalkyl wherein said cycloalkyl is substituted with one or more substituents.

In some aspects, R¹ and R² are taken together to form a heterocycloalkyl.

In some aspects, R¹ and R² are taken together to form a heterocycloalkyl wherein said heterocycloalkyl is substituted with one or more substituents.

Also disclosed herein, for reference purposes, is a compound of formula (Ia) 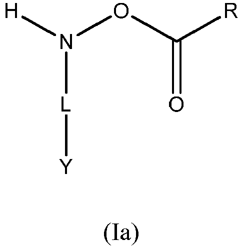 or a pharmaceutically acceptable salt, hydrate, or solvate thereof wherein:
L is a bond, -SO₂- or -O-;
Y is a heteroaryl, wherein said heteroaryl is unsubstituted or substituted with one or more substituents independently selected from W;
W is halo, -OH, -CN, -NO₂, -COR¹, -COOR¹, -CONR¹R², -CH(C(O)R¹)₂, -SO₂R¹, or-COX, wherein X is halo, and R¹ and R² are independently alkyl or aryl, or R¹ and R² are taken together to form a cycloalkyl or heterocycloalkyl, wherein said cycloalkyl and heterocycloalkyl are unsubstituted or substituted with one or more substituents;
R is hydrogen, alkyl, heterocycloalkyl, aryl, benzyl, alkoxy, heterocycloalkoxy, aryloxy, benzyloxy or -NR³R⁴, wherein said alkyl, heterocycloalkyl, aryl, benzyl, alkoxy, heterocycloalkoxy, aryloxy, and benzyloxy are unsubstituted or substituted with one or more substituents; and
R³ and R⁴ are independently alkyl or aryl.

In some aspects, L is -SO₂-.

In some aspects, Y is unsubstituted heteroaryl.

In some aspects, Y is thienyl, furyl, pyrrolyl, pyridyl or benzofuranyl.

In some aspects, Y is heteroaryl substituted with one or more substituents independently selected from W.

In some aspects, Y is thienyl, furyl, pyrrolyl, pyridyl or benzofuranyl substituted with one or more substituents independently selected from W.

In some aspects, Y is thienyl substituted with one or more substituents independently selected from W.

In some aspects, Y is benzofuranyl.

In some aspects, Y is pyridyl.

In some aspects, W is halo.

In some aspects, W is chloro or bromo.

In some aspects, R is alkyl.

Also disclosed herein, for reference purposes, is a compound of formula (IIa) 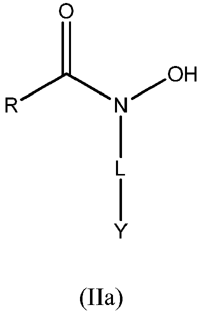 or a pharmaceutically acceptable salt, hydrate, or solvate thereof wherein:
L is a bond, -SO₂- or -O-;
Y is a heteroaryl, wherein said heteroaryl is unsubstituted or substituted with one or more substituents independently selected from W;
W is halo, -OH, -CN, -NO₂, -COR¹, -COOR¹, -CONR¹R², -CH(C(O)R¹)₂, -SO₂R¹ or-COX, wherein X is halo, and R¹ and R² are independently alkyl or aryl, or R¹ and R² are taken together to form a cycloalkyl or heterocycloalkyl, wherein said cycloalkyl and heterocycloalkyl are unsubstituted or substituted with one or more substituents;
R is hydrogen, alkyl, heterocycloalkyl, aryl, benzyl, alkoxy, heterocycloalkoxy, aryloxy, benzyloxy or -NR³R⁴, wherein said alkyl, heterocycloalkyl, aryl, benzyl, alkoxy, heterocycloalkoxy, aryloxy and benzyloxy are unsubstituted or substituted with one or more substituents; and
R³ and R⁴ are independently alkyl or aryl.

In some aspects, L is -SO₂-.

In some aspects, Y is unsubstituted heteroaryl.

In some aspects, Y is heteroaryl substituted with one or more substituents independently selected from W.

In some aspects, Y is thienyl, furyl, pyrrolyl, pyridyl or benzofuranyl.

In some aspects, Y is thienyl, furyl, pyrrolyl, pyridyl or benzofuranyl substituted with one or more substituents independently selected from W.

In some aspects, Y is thienyl substituted with one or more substituents independently selected from W.

In some aspects, Y is benzofuranyl.

In some aspects, Y is pyridyl.

In some aspects, W is halo.

In some aspects, W is chloro or bromo.

In some aspects, R is alkyl.

Representative compounds of formulae (I), (I'), (II), (Ia) and (IIa) include, but are not limited to, the following compounds (Table 2). Compounds marked with a "*" are included for reference purposes.

**Table 2. Representative compounds of formulae (I), (I'), (II), (Ia) and (IIa):**

| Compound No: | Name | Structure |
|---|---|---|
| 1 | *N*-acetyloxy-benzenesulfonamide | 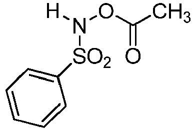 |
| 2 | *N*-(acetyloxy)-2-bromo benzenesulfonamide | 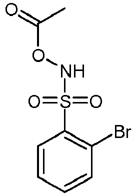 |
| 3 | *N*-acetyloxy-2,6-dichlorobenzenesulfonamide | 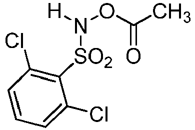 |
| 4 | *N*-acetyloxy-2,6-dibromobenzenesulfonamide | 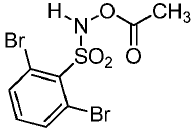 |
| 5 | *N*-benzoyloxy-benzenesulfonamide | 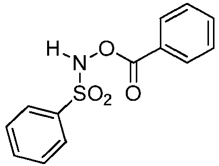 |
| 6 | *N-*(trifluoroacetyloxy)-benzenesulfonamide | 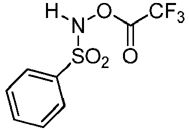 |
| 7 | *N*-(trifluoroacetyloxy)-2,6-dichlorobenzenesulfonamide | 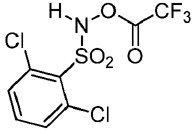 |
| 8 | *N*-(trimethylacetyloxy)-2,6-dichlorobenzesulfonamide | 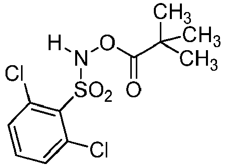 |
| 9 | *N*-(trimethylacetyloxy)-2-bromo benzenesulfonamide | 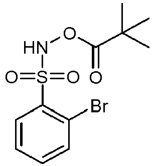 |
| 10 | *N*-(acetyloxy)-2-(methylsulfonyl) benzenesulfonamide | 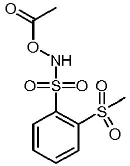 |
| 11 | 2-(methylsulfonyl)-*N-*(propanoyloxy)benzenesulfonamide | 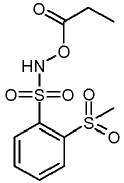 |
| 12 | *N-*[(2-methylpropanoyl)oxy]-2-(methylsulfonyl) benzenesulfonamide | 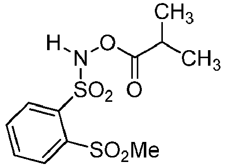 |
| 13 | *N*-[(2,2-dimethylpropanoyl)oxy] -2-(methylsulfonyl) benzenesulfonamide | 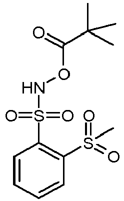 |
| 14 | 2-(methylsulfonyl)-*N-*[(phenylcarbonyl)oxy]benzenesulfonamide | 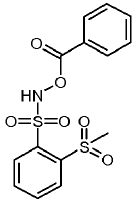 |
| 15* | *N*-hydroxy-*N*-benzoylbenzenesulfonamide | 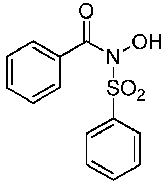 |
| 16* | *N*-hydroxy-*N*-trimethylacetyl-2,6-dichlorobenzenesulfonamide | 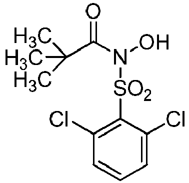 |
| 17* | *N*-[(2-bromophenyl)sulfonyl]-*N-*hydroxymorpholine-4-carboxamide | 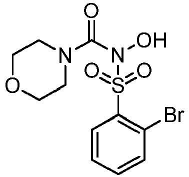 |
| 18 | (2-methanesulfonylbenzene)-sulfonamido-oxan-4-yl carbonate | 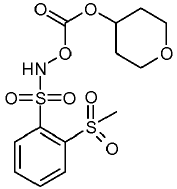 |
| 19 | (2-bromobenzene)sulfonamidooxan-4-yl carbonate | 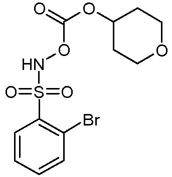 |
| 20 | (1-acetylpiperidin-4-yl)(2-methanesulfonylbenzene)-sulfonamido carbonate | 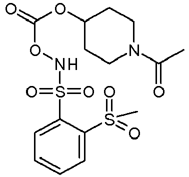 |
| 21 | 2-methanesulfonyl-*N-*[(methoxycarbonyl)oxy]benzene-1-sulfonamide | 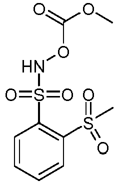 |
| 22 | 2-methanesulfonyl-*N*-{[(2-methoxyethoxy)carbonyl]oxy}-benzene-1-sulfonamide | 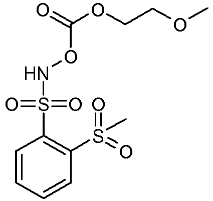 |
| 23 | 2-methanesulfonyl-*N*-({[2-(2-methoxyethoxy)ethoxy]carbonyl}-oxy)benzene-1-sulfonamide | 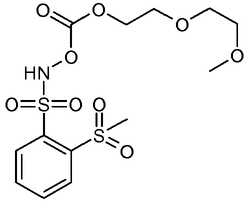 |
| 24 | (4S)-4-[({[(2-methanesulfonylbenzene)sulfonamidooxy] carbonyl}oxy)methyl]-2,2-dimethyl-1,3-dioxolane | 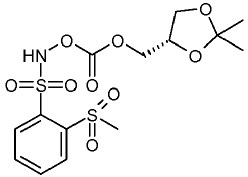 |
| 25 | *N*-({[(1,3-diethoxypropa*N*-2 yl)oxy]carbonyl}oxy)-2-methane sulfonylbenzene-1-sulfonamide | 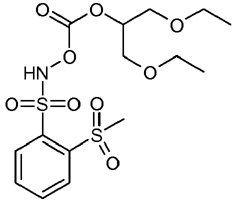 |
| 26 | 3-({[(2-methanesulfonylbenzene)-sulfonamidooxy] carbonyl}oxy)propane-1,2-diol | 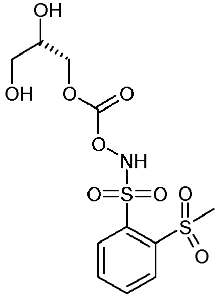 |
| 27 | 4-({[(2-methanesulfonylbenzene)-sulfonamidooxy]carbonyl}oxy)-butan-1-ol | 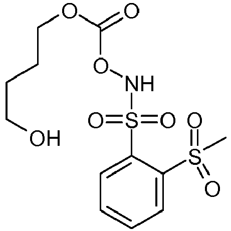 |
| 28 | 2-({[(2-methanesulfonylbenzene)sulfonamid ooxy]carbonyl}oxy)ethan-1-ol | 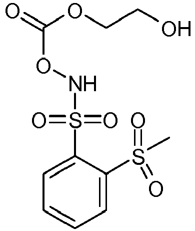 |
| 29 | (2-methanesulfonylbenzene)-sulfonamido *N,N*-dimethylcarbamate | 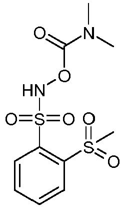 |
| 30 | (2-bromobenzene)sulfonamido *N,N-*dimethylcarbamate | 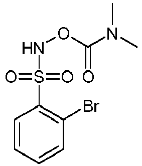 |
| 31 | (2-methanesulfonylbenzene)-sulfonamido morpholine-4-carboxylate | 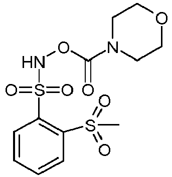 |
| 32 | (2-methanesulfonylbenzene)-sulfonamido4-acetylpiperazine-1-carboxylate | 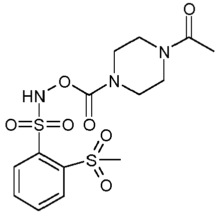 |
| 33 | (2-methanesulfonylbenzene)-sulfonamido *N*-cyclohexyl-*N-*methylcarbamate | 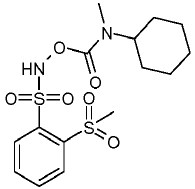 |
| 34 | (2-methanesulfonylbenzene)-sulfonamido piperazine-1-carboxylate | 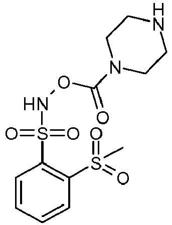 |
| 35 | (2-methanesulfonylbenzene)-sulfonamido *N*-(2-methoxyethyl)-*N-*methylcarbamate | 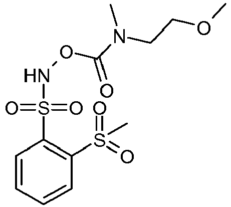 |
| 36 | (2-methanesulfonylbenzene)-sulfonamido 4-(pyridin-4-yl)piperazine-1-carboxylate | 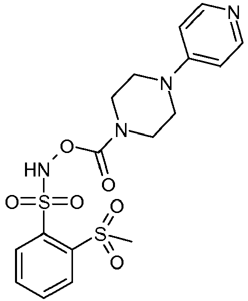 |
| 37 | (2-methanesulfonylbenzene)-sulfonamido 4-(morpholin-4-yl)piperidine-1-carboxylate | 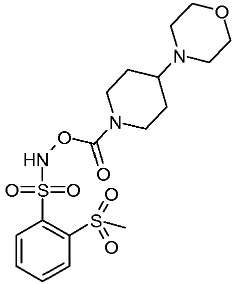 |
| 38 | (2-methanesulfonylbenzene)-sulfonamido *N,N-*diethylcarbamate | 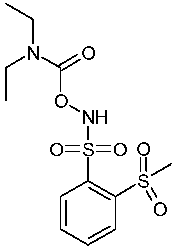 |
| 39 | (2-methanesulfonylbenzene)-sulfonamido 4-(piperidin-1-yl)piperidine-1-carboxylate | 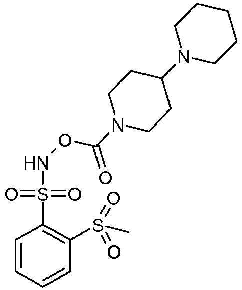 |
| 40 | (2-methanesulfonylbenzene)-sulfonamido *N*-methoxy-*N-*methylcarbamate | 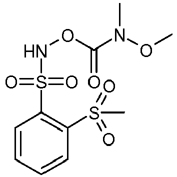 |
| 41 | (2-methanesulfonylbenzene)-sulfonamido pyrrolidine-1-carboxylate | 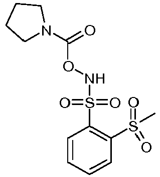 |
| 42 | 2-[(carboxymethyl)( {[(2-methanesulfonyl benzene)sulfonamidooxy]carbonyl}) amino]acetic acid | 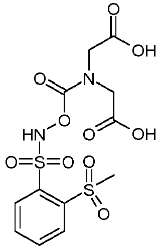 |
| 43 | (2-methanesulfonylbenzene)-sulfonamido 4-carbamoylpiperidine-1-carboxylate | 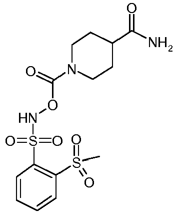 |
| 44 | (2-methanesulfonylbenzene)-sulfonamido *N*-methyl-*N*-(pyridin-3-ylmethyl)carbamate | 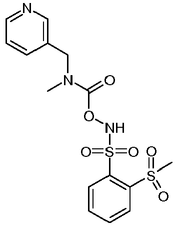 |
| 45 | 2-({ [(2-methanesulfonylbenzene) sulfonamidooxy]carbonyl} (methyl)-amino) acetic acid | 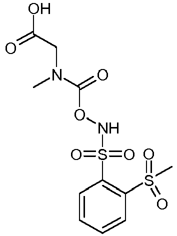 |
| 46 | (2-methanesulfonylbenzene)-sulfonamido *N-*methyl-*N-*(1-methylpiperidin-4-yl)carbamate | 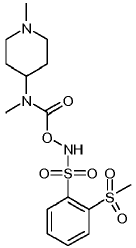 |
| 47 | 2-[(carboxymethyl)({[(2-methanesulfonylbenzene)sulfonamid ooxy]carbonyl})amino]acetic acid | 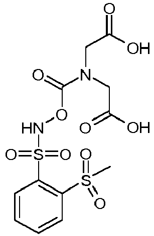 |
| 48 | (2-methanesulfonylbenzene)-sulfonamido 2-oxoimidazolidine-1-carboxylate | 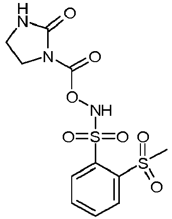 |
| 49 | (2-methanesulfonylbenzene)-sulfonamido 3-oxopiperazine-1-carboxylate | 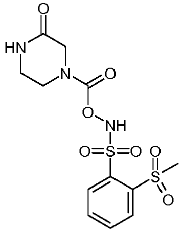 |
| 50 | [2-chloro-5-(dimethylcarbamoyl)benzene]-sulfonamido-2,2-dimethylpropanoate | 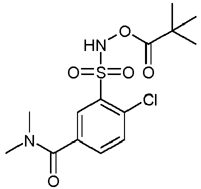 |
| 51 | (2-methanesulfonylbenzene) sulfonamido 2-(acetyloxy)benzoate | 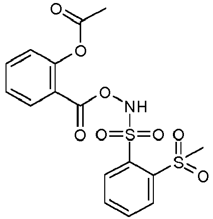 |
| 52 | (2-methanesulfonylbenzene) sulfonamido 2-[4-(2-methylpropyl)phenyl]propanoate | 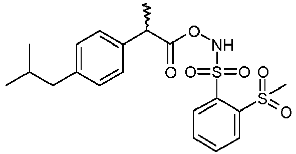 |
| 53 | (2-bromobenzene)sulfonamido benzoate | 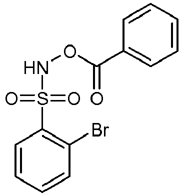 |
| 54 | (2-bromobenzene)sulfonamido 2-methylpropanoate | 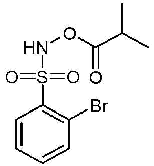 |
| 55 | (2-chlorobenzene)sulfonamido 2,2-dimethylpropanoate | 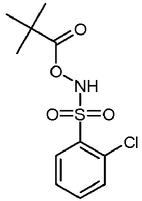 |
| 56 | [2-chloro-5-(dimethylcarbamoyl)benzene]-sulfonamido acetate | 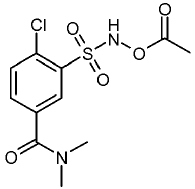 |
| 57 | [2-chloro-5-(dimethylcarbamoyl)benzene]-sulfonamido 2-(acetyloxy)benzoate | 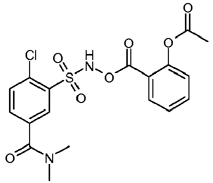 |
| 58 | (2-chlorobenzene)sulfonamido 2-methylpropanoate | 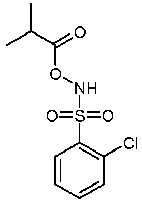 |
| 59 | (2-bromobenzene)sulfonamido 2-phenylacetate | 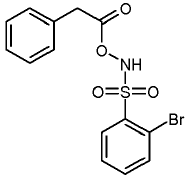 |
| 60 | (2-bromobenzene)sulfonamido 2-phenylbutanoate | 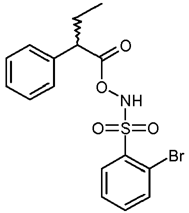 |
| 61 | (2-methanesulfonylbenzene)-sulfonamido 2-phenylbutanoate | 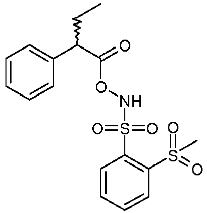 |
| 62 | (2-methanesulfonylbenzene) sulfonamido (2S)-2-(1,3-dioxo-2,3-dihydro-1H-isoindol-2-yl)-3-methylbutanoate | 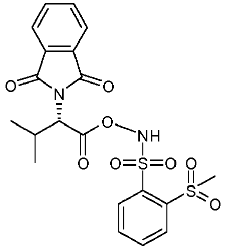 |
| 63 | (2-bromobenzene)sulfonamido (2S)-2-(1,3-dioxo-2,3-dihydro-1H-isoindol-2-yl)-3-methylbutanoate | 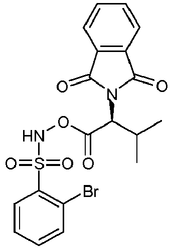 |
| 64 | (2-bromobenzene)sulfonamido 2-methyl-2-phenylpropanoate | 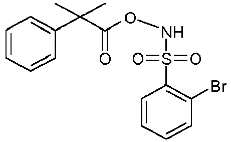 |
| 65 | (2-bromobenzene)sulfonamido 1-phenylcyclopentane-1-carboxylate | 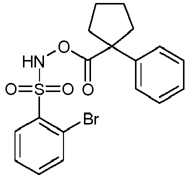 |
| 66 | (2-bromobenzene)sulfonamido 1-acetylpyrrolidine-2-carboxylate | 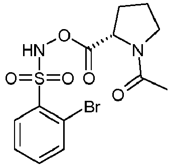 |
| 67 | (2-bromobenzene)sulfonamido (2S)-2-phenylpropanoate | 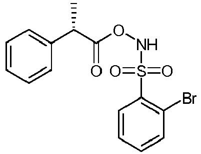 |
| 68 | (2-bromobenzene)sulfonamido (2R)-2-phenylpropanoate | 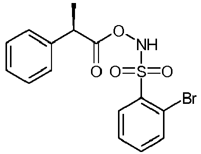 |
| 69 | (3-methanesulfonylbenzene) sulfonamido 2,2-dimethylpropanoate | 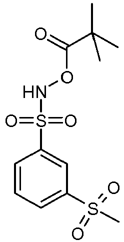 |
| 70 | (3-methanesulfonylbenzene) sulfonamido 2-methylpropanoate | 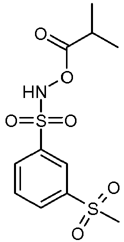 |
| 71 | (2-methanesulfonylbenzene) sulfonamido 2-(*N*-methylacetamido) acetate | 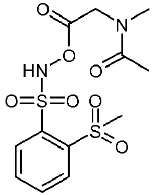 |
| 72 | (2-methanesulfonylbenzene) sulfonamido (2S)-4-methyl-2-(methylamino)pentanoate | 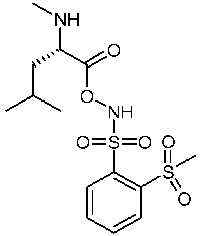 |
| 73 | (2-methanesulfonylbenzene) sulfonamido (2R)-2-(methylamino) propanoate | 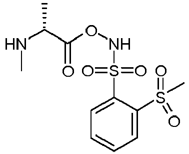 |
| 74 | (2-methanesulfonylbenzene) sulfonamido (2S)-2-(methylamino) propanoate | 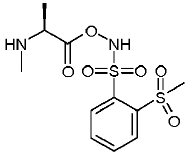 |
| 75 | (2-methanesulfonylbenzene)-sulfonamido 2-(methylamino)acetate | 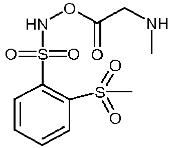 |
| 76 | (2-methanesulfonylbenzene)-sulfonamido (2S)-3-methyl-2-(methylamino)butanoate | 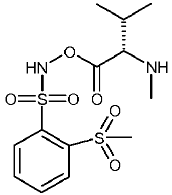 |
| 77* | methanesulfonamido 2,2-dimethylpropanoate | 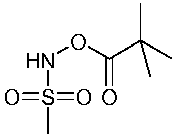 |
| 78* | [(4-chlorophenyl)methane]-sulfonamido 2,2-dimethylpropanoate | 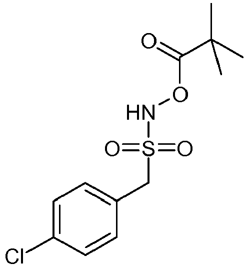 |
| 79* | *N*-(acetyloxy)-3-bromothiophene-2-sulfonamide | 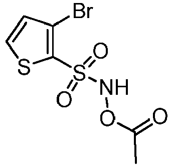 |
| 80* | 1-benzofuran-2-sulfonamido 2,2-dimethylpropanoate | 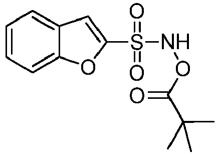 |
| 81* | 1-benzofuran-2-sulfonamido acetate | 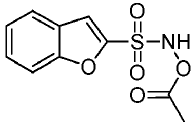 |
| 82* | 3 -bromothiophene-2-sulfonamido 2,2-dimethylpropanoate | 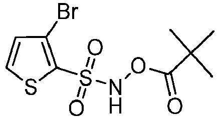 |
| 83* | 3-chlorothiophene-2-sulfonamido 2,2-dimethylpropanoate | 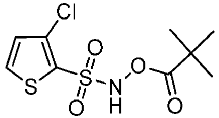 |
| 84* | 5-chlorothiophene-2-sulfonamido 2-methylpropanoate | 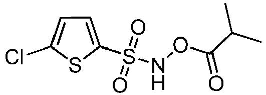 |
| 85* | 5-chlorothiophene-2-sulfonamido 2,2-dimethylpropanoate | 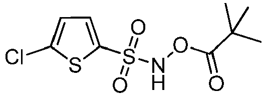 |
| 86* | pyridine-3-sulfonamido 2,2-dimethylpropanoate | 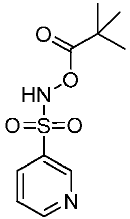 |
| 87* | pyridine-3-sulfonamido 2-methylpropanoate | 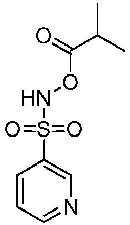 |

In some embodiments, the compound for use in the invention donates nitroxyl under physiological conditions.

For all compounds disclosed herein, where applicable due to the presence of a stereocenter, the compound is intended to embrace all possible stereoisomers of the compound depicted or described. Compositions for use in the invention include ones comprising a compound with at least one stereocenter, and include racemic mixtures or mixtures containing an enantiomeric excess of one enantiomer or single diastereomers or diastereomeric mixtures. All such isomeric forms of these compounds are expressly included herein the same as if each and every isomeric form were specifically and individually listed. The compounds herein may also contain linkages (*e.g*., carbon-carbon bonds) wherein bond rotation is restricted about that particular linkage, *e.g*. restriction resulting from the presence of a ring or double bond. Accordingly, all cis/trans and E/Z isomers are also expressly included in the present disclosure. The compounds herein may also be represented in multiple tautomeric forms, in such instances, the disclosure expressly includes all tautomeric forms of the compounds described herein, even though only a single tautomeric form may be represented.

In some aspects, the disclosure provides a substantially pure compound. "Substantially pure" intends a preparation of the compound that contains no more than 25% of impurity (*e.g.* by weight %), which impurity maybe another compound altogether or a different form of the compound (*e.g.* a different salt or isomer). Percent purity may be assessed by methods known in the art. In some aspects, a preparation of substantially pure compound is provided where the preparation contains no more than 15% of impurity. In some aspects, a preparation of substantially pure compound is provided where the preparation contains no more than 10% impurity. In some aspects, a preparation of substantially pure compound is provided where the preparation contains no more than 5% impurity. In some aspects, a preparation of substantially pure compound is provided where the preparation contains no more than 3% impurity. In some aspects, a preparation of substantially pure compound is provided where the preparation contains no more than 1% impurity.

In some aspects, the disclosure provides a compound in purified and/or isolated form, for example following column chromatography, high-pressure liquid chromatography, recrystallization, or other purification techniques. Where particular stereoisomers of compounds disclosed herein are denoted, such stereoisomers may be substantially free of other stereoisomers.

### Pharmaceutical Compositions

The present invention provides certain compounds, salts, solvates and hydrates for use in treating certain diseases or conditions.

Thus, in some aspects, the disclosure provides a pharmaceutical composition comprising an effective amount of a compound described herein or a pharmaceutically acceptable salt thereof, together with a pharmaceutically acceptable excipient.

Examples of pharmaceutically acceptable excipients include those described above, such as carriers, surface active agents, thickening or emulsifying agents, solid binders, dispersion or suspension aids, solubilizers, colorants, flavoring agents, coatings, disintegrating agents, lubricants, sweeteners, preservatives, isotonic agents, and combinations thereof. The selection and use of pharmaceutically acceptable excipients are taught in "Remington: The Science and Practice of Pharmacy", 21st Ed. (Lippincott Williams & Wilkins 2005), the disclosure of which is incorporated herein by reference.

The pharmaceutical compositions may be formulated for administration in solid or liquid form, including those adapted for the following: (1) oral administration, for example, drenches (for example, aqueous or non-aqueous solutions or suspensions), tablets (for example, those targeted for buccal, sublingual and systemic absorption), caplets, boluses, powders, granules, pastes for application to the tongue, hard gelatin capsules, soft gelatin capsules, mouth sprays, troches, lozenges, pellets, syrups, suspensions, elixirs, liquids, emulsions and microemulsions; (2) parenteral administration, for example, by subcutaneous, intramuscular, intravenous or epidural injection as, for example, a sterile solution or suspension; (3) topical application, for example, as a cream, ointment, patch, pad or spray applied to the skin; (4) intravaginally or intrarectally, for example, as a pessary, cream or foam; (5) sublingually; (6) ocularly; (7) transdermally; or (8) nasally. The pharmaceutical compositions may be for immediate, sustained or controlled release.

In some aspects, the pharmaceutical compositions are formulated for oral administration. In some aspects, the pharmaceutical compositions are formulated for intravenous administration. In some aspects, the pharmaceutical compositions are formulated for administration by inhalation.

The compounds and pharmaceutical compositions described herein may be prepared as any appropriate unit dosage form, such as capsules, sachets, tablets; powder, granules, solution, suspension in an aqueous liquid or a non-aqueous liquid, oil-in-water liquid emulsion, water-in-oil liquid emulsion, liposomes and bolus.

Tablets may be made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface-active or dispersing agent. Molded tablets may be made by molding in a suitable machine a mixture of the powdered compound moistened with an inert liquid diluent. The tablets may be optionally coated or scored and may be formulated so as to provide slow or controlled release of the active ingredient therein. Methods of formulating such slow or controlled release compositions of pharmaceutically active ingredients, such as those herein and other compounds known in the art, are known in the art and described in several issued US Patents, some of which include, but are not limited to, US Patent Nos. 4,369,174 and 4,842,866, and references cited therein. Coatings can be used for delivery of compounds to the intestine (see, *e.g.* U.S. Patent Nos. 6,638,534, 5,217,720 and 6,569,457, and references cited therein). A skilled artisan will recognize that in addition to tablets, other dosage forms can be formulated to provide slow or controlled release of the active ingredient. Such dosage forms include, but are not limited to, capsules, granulations and gel-caps.

Pharmaceutical compositions suitable for topical administration include, without limitation, lozenges comprising the ingredients in a flavored basis, such as sucrose, acacia and tragacanth; and pastilles comprising the active ingredient in a flavored basis or in an inert basis, such as gelatin and glycerin.

Pharmaceutical compositions suitable for parenteral administration include, without limitation, aqueous and non- aqueous sterile injection solutions containing, for example, anti-oxidants, buffers, bacterio stats and solutes that render the formulation isotonic with the blood of the intended recipient; and aqueous and non-aqueous sterile suspensions containing, for example, suspending agents and thickening agents. The formulations may be presented in unit-dose or multi-dose containers, for example, sealed ampules and vials, and may be stored in a freeze dried (lyophilized) condition requiring only the addition of a sterile liquid carrier, such as water, immediately prior to use. In some aspects, the aqueous composition is acidic, having a pH of about 5.5 to about 7.

Extemporaneous injection solutions and suspensions may be prepared from sterile powders, granules and tablets.

### Methods of Using the Compounds and Pharmaceutical Compositions

In some aspects, the disclosure provides a method of modulating (such as increasing or reducing) *in vivo* nitroxyl levels, comprising administering to an individual in need thereof a compound or pharmaceutical composition as described herein. In some aspects, the individual has, is suspected of having, or is at risk of having or developing a disease or condition that is responsive to nitroxyl therapy.

In some aspects, the disclosure provides a method of treating, preventing or delaying the onset and/or development of a disease or condition, comprising administering to an individual (including an individual identified as in need of such treatment, prevention or delay) an effective amount of a compound or pharmaceutical composition as described herein. Identifying an individual in need thereof can be in the judgment of a physician, clinical staff, emergency response personnel or other health care professional and can be subjective (*e.g.* opinion) or objective (*e.g.* measurable by a test or diagnostic method).

Particular diseases or conditions embraced by the methods described herein include, without limitation, cardiovascular diseases, ischemia, reperfusion injury, cancerous diseases, pulmonary hypertension and conditions responsive to nitroxyl therapy.

### Cardiovascular Diseases

The invention provides certain compounds, salts, hydrates and solvates for use in treating, inter alia, cardiovascular diseases.

Thus, in some aspects, the disclosure provides a method of treating a cardiovascular disease, comprising administering an effective amount of a compound or pharmaceutical composition as described herein to an individual in need thereof.

Examples of cardiovascular diseases include, without limitation, cardiovascular diseases that are responsive to nitroxyl therapy, coronary obstructions, coronary artery disease (CAD), angina, heart attack, myocardial infarction, high blood pressure, ischemic cardiomyopathy and infarction, pulmonary congestion, pulmonary edema, cardiac fibrosis, valvular heart disease, pericardial disease, circulatory congestive states, peripheral edema, ascites, Chagas' disease, ventricular hypertrophy, heart valve disease, heart failure, diastolic heart failure, congestive heart failure, acute congestive heart failure, acute decompensated heart failure, and cardiac hypertrophy.

In some aspects, the individual is experiencing heart failure. In some aspects, the individual is experiencing heart failure and/or undergoing treatment with a positive inotrope. In some aspects, the individual is experiencing heart failure and/or undergoing treatment with a *beta*-andrenergic receptor antagonist (also referred to herein as *beta-*antagonist or *beta*-blocker). A *beta*-antagonist includes any compound that effectively acts as an antagonist at an individual's *beta*-adrenergic receptors, and provides desired therapeutic or pharmaceutical results, such as diminished vascular tone and/or heart rate. An individual that is undergoing treatment with a *beta*-antagonist is any individual to whom a *beta*-antagonist has been administered, and in whom the *beta-* antagonist continues to act as an antagonist at the individual's *beta*-adrenergic receptors. Examples of *beta-*antagonists include, without limitation, propranolol, metoprolol, bisoprolol, bucindolol, and carvedilol.

In some aspects, the individual is experiencing heart failure and/or undergoing treatment with a *beta*-adrenergic receptor agonist (also referred to herein as *beta*-agonist). Examples of *beta*-agonists include, without limitation, dopamine, dobutamine, isoproterenol, and analogs and derivatives of such compounds.

The determination of whether an individual is undergoing treatment with a positive inotrope, *beta*-antagonist or *beta*-agonist may be made by examination of the individual's medical history, or screening of the individual for the presence of such agents by chemical tests, such as high-speed liquid chromatography, as described in Thevis et al., Biomed. Chromatogr. 2001, 15, 393-402.

In some aspects, the method further comprises administering an effective amount of at least one other positive inotrope to the individual. In some aspects, the method further comprises administering an effective amount of a *beta-* antagonist to the individual. In some aspects, the method further comprises administering an effective amount of a *beta*-agonist to the individual.

In some aspects, the cardiovascular disease is heart failure. The heart failure may be of any type or form, including any of the heart failures described herein. Nonlimiting examples of heart failure include early stage heart failure, Class I, II, III or IV heart failure, acute heart failure, congestive heart failure (CHF) and acute congestive heart failure.

In some aspects, the cardiovascular disease is CHF, and the method further comprises administering an effective amount of at least one other positive inotropic agent to the individual. In some aspects, the individual is experiencing heart failure. In some embodiments, the at least one other positive inotrope is a *beta*-adrenergic agonist. In some embodiments, the *beta*-adrenergic agonist is dobutamine.

### Ischemia or Reperfusion Injury

The invention provides certain compounds, salts, hydrates and solvates for use in treating, inter alia, ischemia or reperfusion.

Thus, in some aspects, the disclosure provides a method of treating, preventing or delaying the onset and/or development of ischemia or reperfusion injury, comprising administering an effective amount of a compound or pharmaceutical composition as described herein to a subject in need thereof.

In some aspects, the method is for preventing ischemia or reperfusion injury. In some aspects, the compound or pharmaceutical composition is administered prior to the onset of ischemia. In some aspects, the pharmaceutical composition is administered prior to procedures in which myocardial ischemia may occur, for example an angioplasty or surgery, such as a coronary artery bypass graft surgery. In some aspects, the compound or pharmaceutical composition is administered after ischemia but before reperfusion. In some aspects, the compound or pharmaceutical composition is administered after ischemia and reperfusion.

In some aspects, the subject is an individual. In some aspects, the subject is an individual at risk for an ischemic event. In some aspects, the individual is at risk for a future ischemic event, but has no present evidence of ischemia. The determination of whether an individual is at risk for an ischemic event can be performed by any method known in the art, such as examining the individual or the individual's medical history. In some aspects, the individual has had a prior ischemic event. Thus, the individual may be at risk of a first or subsequent ischemic event. Examples of individuals at risk for an ischemic event include individuals with known hypercholesterolemia, EKG changes associated with ischemia (*e.g*., peaked or inverted T- waves or ST segment elevations or depression in an appropriate clinical context), abnormal EKG not associated with active ischemia, elevated CKMB, clinical evidence of ischemia (*e.g*., crushing sub-sternal chest pain or arm pain, shortness of breath and/or diaphoresis), prior history of myocardial infarction, elevated serum cholesterol, sedentary lifestyle, angiographic evidence of partial coronary artery obstruction, echocardiographic evidence of myocardial damage, or any other evidence of a risk for a future ischemic event. Examples of ischemic events include, without limitation, myocardial infarction (MI) and neurovascular ischemia, such as a cerebrovascular accident CVA).

In some aspects, the subject is an organ that is to be transplanted. In some aspects, the compound or pharmaceutical composition is administered prior to reperfusion of the organ in a transplant recipient. In some aspects, the compound or pharmaceutical composition is administered prior to removal of the organ from the donor, for example through the perfusion cannulas used in the organ removal process. If the organ donor is a live donor, for example a kidney donor, the compound or pharmaceutical composition can be administered to the organ donor. In some aspects, the compound or pharmaceutical composition is administered by storing the organ in a solution comprising the compound or pharmaceutical composition. For example, the compound or pharmaceutical composition can be included in the organ preservation solution, such as the University of Wisconsin "UW" solution, which is a solution comprising hydroxyethyl starch substantially free of ethylene glycol, ethylene chlorohydrin and acetone (*see,* U.S. Pat. No. 4,798,824). In some aspects, the amount of the compound or pharmaceutical composition is such that ischemia or reperfusion injury to the tissues of the organ is reduced upon reperfusion in the recipient of transplanted organ. In some aspects, the method reduces tissue necrosis (the size of infarct) in at-risk tissues.

Ischemia or reperfusion injury may damage tissues other than those of the myocardium and the disclosure embraces methods of treating or preventing such damage. In some aspects, the ischemia or reperfusion injury is non-myocardial. In some aspects, the method reduces injury from ischemia or reperfusion in the tissue of the brain, liver, gut, kidney, bowel, or any part of the body other than the myocardium. In some aspects, the individual is at risk for such injury. Selecting a person at risk for non-myocardial ischemia could include a determination of the indicators used to assess risk for myocardial ischemia. However, other factors may indicate a risk for ischemia/reperfusion in other tissues. For example, surgery patients often experience surgery related ischemia. Thus, individuals scheduled for surgery could be considered at risk for an ischemic event. The following risk factors for stroke (or a subset of these risk factors) could demonstrate an individual's risk for ischemia of brain tissue: hypertension, cigarette smoking, carotid artery stenosis, physical inactivity, diabetes mellitus, hyperlipidemia, transient ischemic attack, atrial fibrillation, coronary artery disease, congestive heart failure, past myocardial infarction, left ventricular dysfunction with mural thrombus, and mitral stenosis. Ingall, Postgrad. Med. 2000, 107(6), 34-50. Further, complications of untreated infectious diarrhea in the elderly can include myocardial, renal, cerebrovascular and intestinal ischemia. Slotwiner-Nie et al., Gastroenterol. Clin. N. Am. 2001, 30(3), 625-635. Alternatively, individuals could be selected based on risk factors for ischemic bowel, kidney or liver disease. For example, treatment would be initiated in elderly individuals at risk of hypotensive episodes (such as surgical blood loss). Thus, individuals presenting with such an indication would be considered at risk for an ischemic event. In some aspects, the individual has any one or more of the conditions listed herein, such as diabetes mellitus or hypertension. Other conditions that may result in ischemia, such as cerebral arteriovenous malformation, could demonstrate an individual's risk for an ischemic event.

In some aspects, the method further comprises administering an additional therapeutic agent. The therapeutic agent may be, for example, a nitroxyl-donating compound, such as Angeli's salt or another compound described herein, a *beta*-blocker, a calcium channel blocker, an anti-platelet agent or any other therapeutic agent for reducing ischemic injury or for protecting myocardium in the individual.

### Cancerous Diseases

The present invention provides certain compounds, salts, hydrates and solvates for use in treating, inter alia, cancerous diseases.

Thus, in some embodiments, the disclosure provides a method of treating, preventing or delaying the onset and/or development of a cancerous disease, comprising administering an effective amount of a compound or pharmaceutical composition as described herein to an individual in need thereof.

In some embodiments, the individual has or is suspected of having a cancerous disease, e.g. cancer.

Cancers that may be treated by the methods described herein include, without limitation, cancers of the head and neck, which include tumors of the head, neck, nasal cavity, paranasal sinuses, nasopharynx, oral cavity, oropharynx, larynx, hypopharynx, salivary glands, and paragangliomas; cancers of the liver and biliary tree, such as hepatocellular carcinoma; intestinal cancers, such as colorectal cancer; ovarian cancer; small cell and non-small cell lung cancer; breast cancer sarcomas, such as fibrosarcoma, malignant fibrous histiocytoma, embryonal rhabdomysocarcoma, leiomysosarcoma, neurofibrosarcoma, osteosarcoma, synovial sarcoma, liposarcoma, and alveolar soft part sarcoma; neoplasms of the central nervous systems, such as brain cancer; lymphomas such as Hodgkin's lymphoma, lymphoplasmacytoid lymphoma, follicular lymphoma, mucosa-associated lymphoid tissue lymphoma, mantle cell lymphoma, B-lineage large cell lymphoma, Burkitt's lymphoma, and T-cell anaplastic large cell lymphoma.

In some aspects, the method further comprises administering an effective amount of an additional therapeutic agent to the individual. In some aspects, the additional therapeutic agent is an anti-cancer agent or a cytotoxic agent. Examples of such agents include, without limitation, alkylating agents, angiogenesis inhibitors, anti-metabolites, DNA cleavers, DNA crosslinkers, DNA intercalators, DNA minor groove binders, enediynes, heat shock protein 90 inhibitors, histone deacetylase inhibitors, microtubule stabilizers, nucleoside (purine or pyrimidine) analogs, nuclear export inhibitors, proteasome inhibitors, topoisomerase (I or II) inhibitors, tyrosine kinase inhibitors. Specific anti-cancer or cytotoxic agents include, for example, *beta*.-lapachone, ansamitocin P3, auristatin, bicalutamide, bleomycin, bleomycin, bortezomib, busulfan, calicheamycin, callistatin A, camptothecin, capecitabine, cisplatin, cryptophycins, daunorubicin, docetaxel, doxorubicin, duocarmycin, dynemycin A, etoposide, floxuridine, floxuridine, fludarabine, fluoruracil, gefitinib, gemcitabine, hydroxyurea, imatinib, interferons, interleukins, irinotecan, methotrexate, mitomycin C, oxaliplatin, paclitaxel, spongistatins, suberoylanilide hydroxamic acid (SAHA), thiotepa, topotecan, trichostatin A, vinblastine, vincristine and vindesine.

### Pulmonary Hypertension

The present invention provides certain compounds, salts, hydrates and solvates for use in treating, inter alia, pulmonary hypertension.

Thus, in some aspects, the disclosure provides a method of treating, preventing or delaying the onset and/or development of pulmonary hypertension, comprising administering an effective amount of a compound or pharmaceutical composition as described herein to an individual in need thereof. In some aspects, the pulmonary hypertension is selected from the diseases and conditions listed above in Table 1. In some aspects, the pulmonary hypertension is pulmonary arterial hypertension (PAH). In some aspects, the pulmonary hypertension is pulmonary hypertension owing to left heart disease. In some aspects, the left heart disease is left heart failure. In some aspects, the left heart failure is systolic heart failure. In some aspects, the left heart failure is diastolic heart failure. In some aspects, the left heart failure is chronic or acutely decompensated. In some aspects, the pulmonary hypertension is chronic thromboembolic pulmonary hypertension.

In some aspects, the disclosure provides a method of reducing mean pulmonary arterial pressure (MPAP), comprising administering an effective amount of a compound or a pharmaceutical composition described herein to an individual in need thereof. In some aspects, the MPAP is reduced by up to about 50%. In some aspects, the MPAP is reduced by up to about 25%. In some aspects, the MPAP is reduced by up to 20%. In some aspects, the MPAP is reduced by up to 15%. In some aspects, the MPAP is reduced by up to 10%. In some aspects, the MPAP is reduced by up to 5%. In some aspects, the MPAP is reduced to about 12 to 16 mmHg. In some aspects, the MPAP is reduced to about 15 mmHg.

### Administration Modes, Regimens and Dose Levels

Any administration regimen well known to those skilled in the art for regulating the timing and sequence of drug delivery can be used and repeated as necessary to effect treatment in the methods described herein. For example, the compound or pharmaceutical composition may be administered 1, 2, 3 or 4 times daily, by a single dose, multiple discrete doses or continuous infusion.

The compound or pharmaceutical composition may be administered prior to, at substantially the same time with, or after administration of an additional therapeutic agent. The administration regimen may include pretreatment and/or co-administration with the additional therapeutic agent. In such case, the compound or pharmaceutical composition and the additional therapeutic agent may be administered simultaneously, separately, or sequentially.

Examples of administration regimens include without limitation:
administration of each compound, pharmaceutical composition and therapeutic agent in a sequential manner; and
co-administration of each compound, pharmaceutical composition and therapeutic agent in a substantially simultaneous manner (e.g., as in a single unit dosage form) or in multiple, separate unit dosage forms for each compound, pharmaceutical composition and therapeutic agent.

Administration of the compound or pharmaceutical composition may be via any accepted mode known to one skilled in the art, for example, orally, parenterally, by inhalation spray, topically, rectally, nasally, buccally, vaginally, intraocularly, intrapulmonarily, or via an implanted reservoir. The term "parenterally" includes without limitation subcutaneously, intravenously, intramuscularly, intraperitoneally, intrathecally, intraventricularly, intrasternally, intracranially, by intraosseous injection and by infusion techniques. Administration may involve systemic exposure or may be local, such as when a compound or pharmaceutical composition is administered at the site of interest. Various tools can be used for administering at the site of interest, such as catheters, trocars, projectiles, pluronic gels, stems, sustained drug release polymers or other devices which provide for internal access. Where the compound or pharmaceutical composition is administered to an organ to be donated, such organ may be bathed in a medium containing the compound or pharmaceutical composition. Alternatively, the compound or pharmaceutical composition may be painted onto the organ, or may be applied in any suitable manner.

It will be appreciated by those skilled in the art that the "effective amount" or "dose level" will depend on various factors such as the particular administration mode, administration regimen, compound, and composition selected, and the particular disease and patient being treated. For example, the appropriate dose level may vary depending upon the activity, rate of excretion and possible toxicity of the specific compound or composition employed; the age, body weight, general health, gender and diet of the patient being treated; the frequency of administration; the other therapeutic agent(s) being co-administered; and the type and severity of the disease.

The compounds and pharmaceutical compositions described herein may be administered at suitable dose level. In some aspects, the compound or pharmaceutical composition is administered at a dose level of about 0.0001 to 4.0 grams once per day (or multiple doses per day in divided doses) for adults. Thus, in some aspects, the compound or pharmaceutical composition is administered at a dose level range in which the low end of the range is any amount between 0.1 mg/day and 400 mg/day and the high end of the range is any amount between 1 mg/day and 4000 mg/day (*e.g.,* 5 mg/day and 100 mg/day, 150 mg/day and 500 mg/day). In some aspects, the compound or pharmaceutical composition is administered at a dose level range in which the low end of the range is any amount between 0.1 mg/kg/day and 90 mg/kg/day and the high end of the range is any amount between 1 mg/kg/day and 100 mg/kg/day (*e.g.,* 0.5 mg/kg/day and 2 mg/kg/day, 5 mg/kg/day and 20 mg/kg/day).

In some aspects, the compound or pharmaceutical composition is administered at a weight base dose. In some aspects, the dose level is about 0.001 to about 10,000 mg/kg/d. In some aspects, the dose level is about 0.01 to about 1,000 mg/kg/d. In some aspects, the dose level is about 0.01 to about 100 mg/kg/d. In some aspects, the dose level is about 0.01 to about 10 mg/kg/d. In some aspects, the dose level is about 0.1 to about 1 mg/kg/d. In some embodiments, the dose level is less than about 1 g/kg/d.

The dose level can be adjusted for intravenous administration. In such case, the compound or pharmaceutical composition can be administered in an amount of between about .01 µg/kg/min to about 100 µg/kg/min, about .05 µg/kg/min to about 95 µg/kg/min, about .1 µg/kg/min to about 90 µg/kg/min, about 1.0 µg/kg/min to about 80 µg/kg/min, about 10.0 µg/kg/min to about 70 µg/kg/min, about 20 µg/kg/min to about 60 µg/kg/min, about 30 µg/kg/min to about 50 µg/kg/min, about .01 µg/kg/min to about 1.0 µg/kg/min, about .01 µg/kg/min to about 10 µg/kg/min, about 0.1 µg/kg/min to about 1.0 µg/kg/min, about 0.1 µg/kg/min to about 10 µg/kg/min, about 1.0 µg/kg/min to about 5 µg/kg/min, about 70 µg/kg/min to about 100 µg/kg/min, about 80 µg/kg/min to about 90 µg/kg/min.

The dosing interval can be adjusted according to the needs of the individual. For longer intervals of administration, extended release or depot formulations can be used.

### Kits Comprising the Compounds or Pharmaceutical Compositions

The invention provides certain compounds, salts, hydrates and solvates for use in treating certain diseases or conditions.

Thus, in some aspects, the disclosure provides a kit comprising a compound or a pharmaceutical composition described herein.

In some aspects, the kit further comprises instructions for using the compound or pharmaceutical composition. The instructions may be in any appropriate form, such as written or electronic form. In some aspects, the instructions may be written instructions. In some aspects, the instructions are contained in an electronic storage medium (*e.g.,* magnetic diskette or optical disk). In some aspects, the instructions include information as to the compound or pharmaceutical composition and the manner of administering the compound or pharmaceutical composition to an individual. In some aspects, the instructions relate to a method of use described herein (*e.g.,* treating, preventing and/or delaying onset and/or development of a disease or condition selected from cardiovascular diseases, ischemia, reperfusion injury, cancerous disease, pulmonary hypertension and conditions responsive to nitroxyl therapy).

In some aspects, the kit further comprises suitable packaging. Where the kit comprises more than one compound or pharmaceutical composition, the compounds or pharmaceutical compositions may be packaged individually in separate containers, or combined in one container where cross-reactivity and shelf life permit.

Other than in the working examples, or where otherwise indicated, all numbers expressing quantities of ingredients, reaction conditions, and so forth used in the specification and claims are to be understood as being modified by the term "about". Accordingly, unless indicated to the contrary, such numbers are approximations that may vary depending upon the-desired properties sought to be obtained by the present invention. At the very least, and not as an attempt to limit the application of the doctrine of equivalents to the scope of the claims, each numerical parameter should be construed in light of the number of significant digits and ordinary rounding techniques.

While the numerical ranges and parameters setting forth the broad scope of the invention are approximations, the numerical values set forth in the working examples are reported as precisely as possible. Any numerical value, however, inherently contains certain errors necessarily resulting from the standard deviation found in their respective testing measurements.

### EXAMPLES

The following examples are presented for illustrative purposes and should not serve to limit the scope of the invention.

### General Synthetic Methods

All NMR are recorded on one of the following instruments; Bruker AVANCE 400MHz spectrometer, Bruker 250, 360 or 500 operating at ambient probe temperature using an internal deuterium lock. Chemical shifts are reported in parts per million (ppm) at lower frequency relative to tetramethylsilane (TMS). Standard abbreviations are used throughout (s: singlet; br. s: broad singlet; d: doublet; dd: doublet of doublets; t: triplet; q: quartet; quin: quintet; m: multiplet). Coupling constants are reported in Hertz (Hz).

### EXAMPLE 1: Synthesis of Compounds 1-9

*N*-acyloxy-*tert*-butyl-carbamate is dissolved in anhydrous tetrahydrofuran and 1.05 equivalents of sodium hydride is added. The solution is stirred for five minutes until gas evolution is complete. To this solution 0.95 equivalents of an appropriate sulfonyl chloride is added and stirred until the reaction is complete (as indicated by TLC). The solvent is removed under reduced pressure and the crude *N*-sulfonyl-*N*-acyloxy-*tert*-butyl-carbamate product is purified by column chromatography. To *N*-sulfonyl-*N*-acyloxy-*tert*-butyl-carbamate, five equivalents of trifluoroacetic anhydride are added, the mixture is stirred for five minutes, and then washed several times with hexane. The resultant *N*-acyloxysulfonamide product is purified by column chromatography.

*N*-acetyloxy-benzenesulfonamide (1) is prepared according to Smith, P. A. et al., J. Am. Chem. Soc. 1960, 82, 5731-5740.

*N*-acetyloxy-2-bromobenzenesulfonamide (2). ¹H NMR (400 MHz, δ) 2.00 (3H, s), 7.52 (2H, m), 7.78 (1H, d), 8.16 (1H, d), 9.42 (1H, bs); ¹³C NMR (100 MHz, δ) 18.08, 121.02, 127.93, 133.31, 134.83, 135.40, 135.69, 168.36; IR (KBr, cm⁻¹) 1798.5, 3170.5.

*N*-acetyloxy-2,6-dichlorobenzenesulfonamide (3). ¹H NMR (400 MHz, δ) 2.08 (3H, s), 7.46 (1H, t), 7.53 (2H,d), 9.52 (1H,bs); ¹³C NMR (100MHz, δ) 18.00, 132.00, 131.75, 134.22, 136.75, 168.48; IR (KBr, cm⁻¹) 1812.9, 3221.8.

*N*-acetyloxy-2,6-dibromobenzenesulfonamide (4). ¹H NMR (400 MHz, δ) 2.10 (3H, s), 7.27 (1H, t), 7.84 (2H, d), 9.64 (1H, bs); IR (KBr, cm⁻¹) 1803.3, 3235.4.

*N*-benzoyloxy-benzenesulfonamide (5). ¹H NMR (400 MHz, δ) 7.43-7.51 (4H, m), 7.60-7.63 (2H, m), 7.89-7.97 (4H, m), 9.27 (1H, s); ¹³C NMR (100 MHz, δ) 125.67, 128.78, 128.86, 129.34, 129.72, 134.55, 134.68, 135.27, 164.98; IR (KBr, cm⁻¹) 1747.7, 3166.0.

*N*-(trifluoroacetyloxy)-benzenesulfonamide (6). ¹H NMR (400 MHz, δ) 7.63 (2H, m), 7.75 (1H, t), 7.97 (2H, d), 8.50 (1H, bs); ¹³C NMR (100 MHz, δ) 115.41 (F coupling), 128.79, 129.69, 134.50, 135.32, 155.46 (F coupling); IR (KBr, cm⁻¹) 1814.7, 3167.1 .

*N*-(trifluoroacetyloxy)-2,6-dichlorobenzenesulfonamide (7). IR (KBr, cm⁻¹) 1837.9, 3216.7

*N*-(trimethylacetyloxy)-2,6-dichlorobenzesulfonamide (8). ¹H NMR (400 MHz, δ) 1.12 (9H, s), 7.46 (1H, m), 7.52 (2H, m), 9.80 (1H, bs); ¹³C NMR (100 MHz, δ) 26.66, 38.44, 131.15, 131.71, 134.24, 136.93, 176.26; IR (KBr, cm⁻¹) 1774.2, 3211.0.

*N-*(trimethylacetyloxy)-2-bromobenzesulfonamide (9). ¹H NMR (400 MHz, δ) 1.02 (9H, s), 7.50 (2H, m), 7.79 (1H, m), 8.17 (1H, m), 9.65 (1H, bs); ¹³C NMR (100 MHz, δ) 26.64, 38.17, 121.31, 127.65, 133.65, 134.72, 135.25, 135.65, 176.09; IR (KBr, cm⁻¹) 1754.1, 3157.0.

### EXAMPLE 2: Synthesis of Compounds 2, 9-14, 18-28, 50-87 (Compounds 77-87 are Reference Examples)

### General Method 1

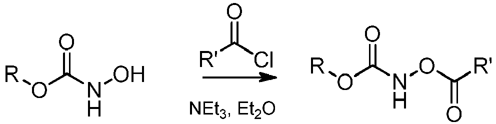

To a stirred solution of N-hydroxy carbamate (1 equiv) in diethyl ether (50 vol) cooled to 0°C is sequentially added triethylamine (1 equiv) and a solution of an acid chloride (1 equiv) in diethyl ether. The reaction mixture is stirred at 0°C until complete consumption of the starting material is observed after which time the reaction is filtered to remove triethylamine hydrochloride and the resulting filtrate is washed with sodium bicarbonate solution (10 vol). The resulting organics are dried over sodium sulfate, filtered and concentrated *in vacuo.* The crude material is either used directly without additional purification or purified by column chromatography eluting with heptane: ethyl acetate.

### General Method 2

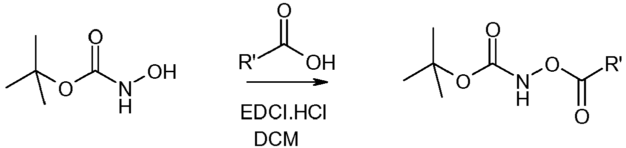

To a stirred solution of *N-tert*-butoxycarbonyl hydroxylamine (1 equiv) and a carboxylic acid (1 equiv) in DCM (10 vol) is added EDCI.HCl (1 equiv). The reaction mixture is stirred at room temperature until complete consumption of the starting material is observed by tlc. The reaction mixture is washed with water (2 x 10 vol), dried over sodium sulfate, filtered and concentrated *in vacuo.* The crude material is purified by column chromatography eluting with heptane:ethyl acetate.

A method of synthesising a compound of the disclosure from a N, O-disubstituted hydroxylamine intermediate is described in General Methods 4-6.

### General Method 3

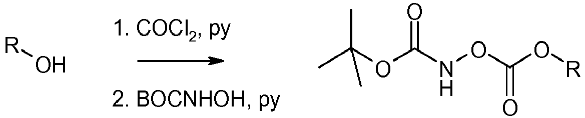

To a solution of an alcohol (1 equiv) in THF (10 vol) cooled to 5°C is sequentially added a 20% solution of phosgene in toluene (1 equiv) and pyridine (1 equiv). The reaction is stirred for 5 minutes before addition of *tert*-butyl *N*-hydroxycarbamate (1 equiv) and pyridine (1 equiv). Stirring is continued for 30 minutes at room temperature before the reaction mixture is filtered through celite™ and the resulting organics concentrated *in vacuo.* The crude reaction is diluted with diethyl ether (20 vol), washed with 0.1N HCl (5 vol) and water (5 vol), dried over sodium sulfate, filtered and concentrated *in vacuo* and purified by either silica column chromatography eluting with heptane: ethyl acetate or reverse phase preparative HPLC.

### General Method 4

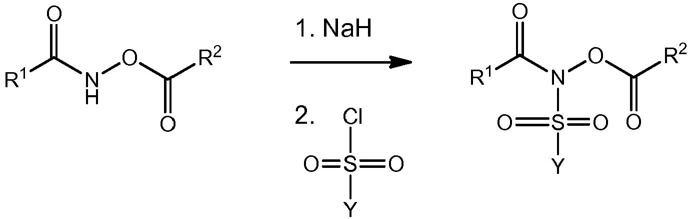

All compounds are synthesized via standard methods using the general method detailed by H. T. Nagasawa et al in J. Med. Chem. 1992, 35, 3648-3652.

A solution of N, O-disubstituted hydroxylamine (1 equiv) in THF (10 vol) is added dropwise to a stirred solution of sodium hydride (60% dispersion in oil, 1 equiv) in THF(10 vol). Stirring is continued for 30 minutes, whereupon a sulfonyl chloride (1 equiv) is added. The reaction mixture is stirred at room temperature until complete consumption of the starting material is observed by tlc whereupon the reaction mixture is quenched by the addition of water (10 vol) and extracted into ether (10 vol). The combined organics are washed with water (10 vol), dried over sodium sulfate, filtered and concentrated *in vacuo* to yield the desired material, which is purified by silica column chromatography eluting with heptane: ethyl acetate.

### General Method 5

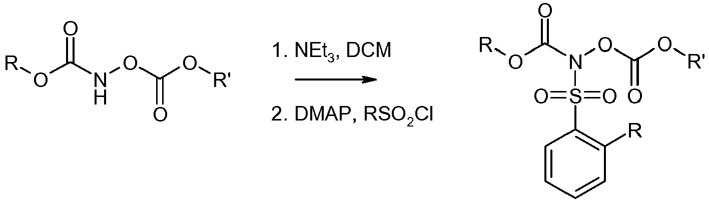

To a solution of N, O-disubstituted hydroxylamine (1 equiv) in DCM (20 vol) and triethylamine (1 equiv) is added dimethylaminopyridine (0.1 equiv) and a sulfonyl chloride (1 equiv). The reaction mixture is stirred at room temperature until complete consumption of the sulfonyl chloride is observed by tlc, whereupon the reaction mixture is quenched by the addition of water (10 vol) and extracted into DCM (10 vol). The combined organics are washed with water (10 vol), dried over sodium sulfate, filtered and concentrated *in vacuo* and either used directly without additional purification or purified directly by either silica column chromatography eluting with heptane: ethyl acetate or reverse phase preparative HPLC.

### General Method 6

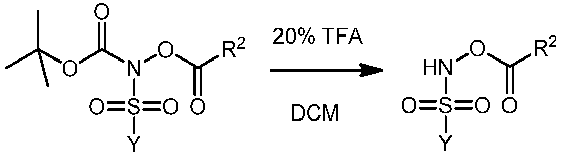

To a stirred solution of an N,O diacylated compound formed using either General Method 4 or 5 in dichloromethane (20 vol) is added trifluoroacetic acid (20-40%). The reaction mixture is stirred for 1-3 hours at room temperature and concentrated *in vacuo* to yield the title compound as a clear, colourless gum. Purification is achieved by trituation from heptane: ethyl acetate or diethyl ether.

### Preparation of N-(acetyloxy)-2-bromobenzene sulfonamide (2)

[(*tert*-Butoxy)carbonyl]amino acetate is prepared from acetyl chloride and *N-tert-*butoxycarbonyl hydroxylamine according to General Method 1 described by Carpino et al. J. Am. Chem. Soc. 1959, 955-957. (10g, 100%), ¹H NMR (500 MHz, DMSO-*d*₆) δ ppm 10.57 (1H, br. s.), 2.10 (3H, s), 1.41 (9H, s).

*tert*-Butyl (acetyloxy)[(2-bromophenyl)sulfonyl]carbamate is prepared according to General Method 4. A solution of [(*tert*-butoxy)carbonyl]amino acetate (0.68 g, 3.9 mmol) in THF (5 ml) is added dropwise to a stirred solution of sodium hydride (0.16 g of a 60% dispersion, 3.9 mmol) in THF (10 ml). Stirring is continued for 30 minutes, whereupon 2-bromobenzene sulfonyl chloride (1.0 g, 3.9 mmol) is added. The reaction mixture is stirred at room temperature for 3 hours after which time tlc (1:1 heptane:ethyl acetate) showed no starting material remained. The reaction mixture is quenched by the addition of water (30 ml) and extracted into ether (2 x 50 ml). The combined organics are dried over sodium sulfate, filtered and concentrated *in vacuo* to yield the desired material as a yellow oil, which is purified by silica column chromatography eluting with heptane: ethyl acetate (4:1; v:v). (0.96 g, 60%), ¹H NMR (360 MHz, DMSO-d6) δ ppm 8.12 - 8.26 (1H, m), 7.87 - 8.06 (1H, m), 7.61 - 7.79 (2H, m), 2.32 (3H, s), 1.26 (9H, s).

*N*-(Acetyloxy)-2-bromobenzenesulfonamide (2) is prepared from *tert*-butyl (acetyloxy) [(2-bromophenyl)sulfonyl]carbamate according to General Method 6. (0.18g, 54%), ¹H NMR (400 MHz, DMSO-d6) δ ppm 11.44 (1H, br. s.), 7.96 - 8.10 (1H, m), 7.84 - 7.97 (1H, m), 7.53 - 7.77 (2H, m), 2.07 (3H, s).

### Preparation of (2-bromobenzene)sulfonamido 2,2-dimethylpropanoate (9)

*N*-[(*tert*-Butoxy)carbonyl](2-bromobenzene)sulfonamido 2,2-dimethylpropanoate is prepared from 2-bromobenzene sulfonyl chloride, sodium hydride and [(*tert-*butoxy)carbonyl]amino 2,2-dimethylpropanoate according to General Method 4. (1.97g, 57%), ¹H NMR (250 MHz, CHLOROFORM-*d*) δ ppm 8.13 - 8.52 (1H, m), 7.71 - 7.96 (1H, m), 7.38 - 7.61 (2H, m), 2.64 - 3.03 (1H, m), 1.37 (9H, s), 1.33 (6H, d, 7.0Hz).

(2-Bromobenzene)sulfonamido 2,2-dimethylpropanoate (9) is prepared from *N*-[(*tert-*butoxy)carbonyl](2-bromobenzene)sulfonamido 2,2-dimethylpropanoate according to General Method 6. (1.46g, 96%), ¹H NMR (250 MHz, CHLOROFORM-*d*) δ ppm 9.65 (1H, s), 8.06 - 8.31 (1H, m), 7.68 - 7.92 (1H, m), 7.44 - 7.65 (2H, m), 1.03 (9H, s).

### Preparation of Preparation of N-(acetyloxy)-2-(methylsulfonyl)benzene sulfonamide (10)

*tert*-Butyl (acetyloxy){[2-(methylsulfonyl)phenyl]sulfonyl}carbamate is prepared from 2-methylsulfonylbenzenesulfonyl chloride, sodium hydride and [(*tert*-butoxy)carbonyl]amino acetate according to General Method 4. (0.5g, 16%), ¹H NMR (400 MHz, DMSO-d6) δ ppm 8.26 - 8.34 (1H, m), 8.17 - 8.25 (1H, m), 8.03 - 8.11 (2H, m), 3.46 (3H, s), 2.32 (3H, s), 1.28 (9H, s).

*N*-(Acetyloxy)-2-(methylsulfonyl)benzenesulfonamide (10) is prepared from *tert*-butyl (acetyloxy){[2-(methylsulfonyl)phenyl]sulfonyl}carbamate according to General Method 6. (0.24g, 64%), ¹H NMR (400 MHz, DMSO-d6) δ ppm 10.63 (1H, br. s.), 8.28 (1H, dd, 7.5, 1.6Hz), 8.21 (1H, dd, 7.5, 1.6Hz), 8.00 - 8.11 (2H, m), 3.47 (3H, s), 2.03 (3H, s).

### Preparation of 2-(methylsulfonyl)-N-(propanoyloxy)benzene sulfonamide (11)

[(*tert*-Butoxy)carbonyl]amino propanoate is prepared from propionyl chloride and *N-tert*-butoxycarbonyl hydroxylamine according to the method described by Carpino et al. J. Am. Chem. Soc. 1959, 955-957. (3.4g, 48%), ¹H NMR (250 MHz, DMSO-*d*₆) δ ppm 10.57 (1H, br. s.), 2.40 (2H, q, 7.5Hz), 1.40 (9H, s), 1.07 (3H, t, 7.4Hz).

*tert*-Butyl {[2-(methylsulfonyl)phenyl]sulfonyl}(propanoyloxy)carbamate is prepared from 2-methylsulfonylbenzenesulfonyl chloride, sodium hydride and [(*tert-*butoxy)carbonyl]amino propanoate according to General Method 4. (1.09g, 68%), ¹H NMR (250 MHz, DMSO-d6) δ ppm 8.16 - 8.37 (2H, m), 8.00 - 8.15 (2H, m), 3.46 (3H, s), 2.61 (2H, q, 7.5Hz), 1.29 (9H, s), 1.15 (3H, t, 7.5Hz).

2-(Methylsulfonyl)-*N*-(propanoyloxy)benzene sulfonamide (11) is prepared from *tert-*butyl {[2-(methylsulfonyl)phenyl]sulfonyl}(propanoyloxy)carbamate according to General Method 6. (0.49g, 64%), ¹H NMR (250 MHz, DMSO-d6) δ ppm 10.59 (1H, s), 8.15 - 8.41 (2H, m), 7.96 - 8.13 (2H, m), 3.47 (3H, s), 2.32 (2H, q, 7.6Hz), 0.92 (3H, t, 7.5Hz).

### Preparation of N-[(2-methylpropanoyl)oxy]-2-(methylsulfonyl)benzene sulfonamide (12)

[(*tert*-Butoxy)carbonyl]amino 2-methylpropanoate is prepared from isobutyryl chloride and *N-tert*-butoxycarbonyl hydroxylamine according to General Method 1 described by Carpino et al. J. Am. Chem. Soc. 1959, 955-957. (6.36g, 83%), ¹H NMR (250 MHz, DMSO-*d*₆) δ ppm 10.51 (1H, br. s.), 2.65 (1H, sept, 7.0Hz), 1.40 (9H, s), 1.13 (6H, d, 7.0Hz).

*tert*-Butyl [(2-methylpropanoyl)oxy]{[2-(methylsulfonyl)phenyl]sulfonyl}-carbamate is prepared from 2-methylsulfonylbenzenesulfonyl chloride, sodium hydride and [(*tert-*butoxy)carbonyl]amino 2-methylpropanoate according to General Method 4. (1.2g, 72%), ¹H NMR (250 MHz, DMSO-d6) δ ppm 8.18 - 8.34 (2H, m), 8.00 - 8.14 (2H, m), 3.46 (3H, s), 2.86 (1H, sept, 7.1Hz), 1.29 (9H, s), 1.21 (6H, d, 7.0Hz).

*N*-[(2-Methylpropanoyl)oxy]-2-(methylsulfonyl)benzenesulfonamide (12) is prepared from *tert*-butyl [(2-methylpropanoyl)oxy]{[2-(methylsulfonyl)phenyl]-sulfonyl}carbamate according to General Method 6. (0.54g, 64%), ¹H NMR (250 MHz, CHLOROFORM-d) δ ppm 10.05 (1H, s), 8.38 (1H, dd, 7.5, 1.6Hz), 8.29 (1H, dd, 7.3, 1.8Hz), 7.78 - 7.99 (2H, m), 3.44 (3H, s), 2.52 (1H, sept, 7.1Hz), 1.05 (6H, d, 7.0Hz).

### Preparation of N-[(2,2-dimethylpropanoyl)oxy]-2-(methylsulfonyl)benzene sulfonamide (13)

[(*tert*-Butoxy)carbonyl]amino 2,2-dimethylpropanoate is prepared from trimethyl acetyl chloride and *N-tert*-butoxycarbonyl hydroxylamine according to General Method 1 described by Carpino et al. J. Am. Chem. Soc. 1959, 955-957. (6.4g, 78%), ¹H NMR (250 MHz, DMSO-*d*₆)δ ppm 10.46 (1H, br. s.), 1.40 (9H, s), 1.20 (9H, s).

*tert*-Butyl [(2,2-dimethylpropanoyl)oxy]{[2-(methylsulfonyl)phenyl]-sulfonyl}carbamate is prepared from 2-methylsulfonylbenzenesulfonyl chloride, sodium hydride and [(*tert*-butoxy)carbonyl]amino 2,2-dimethylpropanoate according to General Method 4. (1.5g, 78%), ¹H NMR (250 MHz, DMSO-d6) δ ppm 8.18 - 8.37 (2H, m), 7.94 - 8.15 (2H, m), 3.46 (3H, s), 1.30 (9H, s), 1.29 (9H, s).

*N*-[(2,2-Dimethylpropanoyl)oxy]-2-(methylsulfonyl)benzenesulfonamide (13) is prepared from *tert*-butyl [(2,2-dimethylpropanoyl)oxy]{[2-(methylsulfonyl)-phenyl]sulfonyl}carbamate according to General Method 6. (0.74g, 69%), ¹H NMR (250 MHz, CHLOROFORM-d) δ ppm 10.08 (1H, s), 8.38 (1H, dd, 7.5, 1.6Hz), 8.29 (1H, dd, 7.3, 1.8Hz), 7.80 - 7.98 (2H, m), 3.44 (3H, s), 1.09 (9H, s).

### Preparation of 2-(methylsulfonyl)-N-[(phenylcarbonyl)oxy]benzene-sulfonamide (14)

[(*tert*-Butoxy)carbonyl]amino benzoate is prepared from benzoyl chloride and *N-tert-*butoxycarbonyl hydroxylamine according to General Method 1 described by Carpino et al. J. Am. Chem. Soc. 1959, 955-957. (7.2g, 80%), ¹H NMR (250 MHz, DMSO-*d*₆) δ ppm 10.89 (1H, br. s.), 7.90 - 8.12 (2H, m), 7.68 - 7.82 (1H, m), 7.51 - 7.65 (2H, m), 1.43 (9H, s).

*tert*-Butyl {[2-(methylsulfonyl)phenyl]sulfonyl}[(phenylcarbonyl)oxy]-carbamate is prepared from 2-methylsulfonylbenzenesulfonyl chloride, sodium hydride and [(*tert-*butoxy)carbonyl]amino benzoate according to General Method 4. (1.7g, 91%), ¹H NMR (250 MHz, DMSO-d6) δ ppm 8.25 - 8.45 (2H, m), 8.03 - 8.20 (4H, m), 7.77 - 7.93 (1H, m), 7.59 - 7.73 (2H, m), 3.48 (3H, s), 1.29 (9H, s).

2-(Methylsulfonyl)-*N*-[(phenylcarbonyl)oxy]benzene-sulfonamide (14) is prepared from *tert*-butyl {[2-(methylsulfonyl)phenyl]sulfonyl}[(phenylcarbonyl)oxy]-carbamate according to General Method 6. (0.87g, 70%), ¹H NMR (250 MHz, CHLOROFORM-d) δ ppm 10.32 (1H, s), 8.39 (1H, dd, 7.7, 1.4Hz), 8.29 (1H, dd, 7.8, 1.5Hz), 7.71 - 7.99 (4H, m), 7.53 - 7.69 (1H, m), 7.35 - 7.49 (2H, m), 3.48 (3H, s).

### Preparation of (2-Methanesulfonylbenzene)sulfonamido oxaN-4-yl carbonate (18)

4-{[({[(*tert*-Butoxy)carbonyl]amino}oxy)carbonyl]oxy}oxane is prepared according to General Method 3. To a solution of tetrahydropyran-4-ol (2g, 19.6 mmol) in THF (20 ml) cooled to 5°C is sequentially added a 20% solution of phosgene in toluene (10.3 ml, 19.6 mmol) and pyridine (1.6 ml, 19.6 mmol). The reaction is stirred for 5 minutes before addition of *tert-*butyl *N*-hydroxycarbamate (2.6g, 19.6 mmol) and pyridine (1.6 ml, 19.6 mmol). Stirring is continued for 30 minutes at room temperature before the reaction mixture is filtered through celite™ and the resulting organics concentrated *in vacuo.* The crude reaction is diluted with diethyl ether (50 ml), washed with 0.1N HCl (10 ml) and water (10 ml), dried over sodium sulfate, filtered and concentrated *in vacuo.* Purification is achieved by silica gel column chromatography eluting with heptane: ethyl acetate (1:1; v:v) to yield the title compound as a clear, colourless oil. (3.64g, 71%). ¹H NMR (500 MHz, CHLOROFORM-*d*) δ ppm 7.78 (1H, s), 4.92 (1H, tt, 8.4, 4.0Hz), 3.88 - 3.98 (2H, m), 3.55 (2H, ddd, 11.8, 8.7, 3.1Hz), 1.98 - 2.09 (2H, m), 1.80 (2H, m), 1.50 (9H, s).

4-{[({*N*-[(*tert*-Butoxy)carbonyl](2methanesulfonylbenzene)sulfonamido}-oxy)carbonyl]oxy}oxane is prepared according to General Method 5. To a solution of 4-{[({[(*tert*-butoxy)carbonyl]amino}oxy)carbonyl]oxy}oxane (1.0g, 3.8 mmol) in DCM (20 ml) is added triethylamine (533 µl, 3.8 mmol) with stirring. After 10 minutes, 2-methylsulfonylbenzenesulfonyl chloride (974 mg, 3.8 mmol) and DMAP (47 mg, 0.38 mmol) are added and stirring is continued for 60 minutes. The reaction is quenched by the addition of water (10 ml), extracted into DCM (20 ml), dried over sodium sulfate, filtered and concentrated *in vacuo.* The reaction mixture is purified by silica column chromatography eluting with heptane: ethyl acetate (1:1; v:v) to yield the title compound as a colourless oil (0.5 g, 27% yield). ¹H NMR (250 MHz, CHLOROFORM-*d*) δ ppm 8.36 - 8.49 (2H, m), 7.83 - 7.90 (2H, m), 4.91 - 5.04 (1H, m), 3.87 - 4.02 (2H, m), 3.51 - 3.64 (2H, m), 3.43 (3H, s), 1.98 - 2.13 (2H, m), 1.74 - 1.94 (2H, m), 1.58 (9H, s).

(2-Methanesulfonylbenzene)sulfonamido oxa*N*-4-yl carbonate (18) is prepared according to General Method 6. To a solution of 4-{[({*N*-[(*tert*-butoxy)carbonyl]-(2methanesulfonylbenzene) sulfonamido}oxy)carbonyl]oxy}oxane (500 mg, 1.3 mmol) in DCM (10ml) is added trifluoroacetic acid (2ml). Stirring is continued at room temperature until LC-MS showed complete consumption of the starting material (*c.a.* 30 minutes). The reaction is concentrated *in vacuo* and trituated with ether to give the title compound as a white solid. (0.323g, 82%), ¹H NMR (500 MHz, CHLOROFORM-d) δ ppm 9.67 (1H, s), 8.32 - 8.42 (2H, m), 7.87 - 7.98 (2H, m), 4.88 (1H, tt, 8.4, 4.2Hz), 3.87 - 3.93 (2H, m), 3.49 - 3.56 (2H, m), 3.44 (3H, s), 1.86 - 2.09 (2H, m), 1.68 - 1.75 (2H, m).

### Preparation of (2-bromobenzene)sulfonamido oxaN-4-yl carbonate (19)

4-{[({*N*-[(*tert*-Butoxy)carbonyl](2-bromobenzene)sulfonamido}oxy)-carbonyl]oxy}oxane is synthesised from 2-bromobenzene sulfonyl chloride and 4-{[({[(*tert-*butoxy)carbonyl]amino}oxy)carbonyl]oxy}oxane according to General Method 5. (1.17g, 77%), ¹H NMR (500 MHz, CHLOROFORM-*d*) δ ppm 8.22 - 8.30 (1H, m), 7.76 - 7.84 (1H, m), 7.47 - 7.57 (2H, m), 4.98 - 5.11 (1H, m), 3.91 - 4.02 (2H, m), 3.52 - 3.64 (2H, m), 1.98 - 2.14 (2H, m), 1.73 - 1.93 (2H, m), 1.39 (9H, s).

(2-Bromobenzene)sulfonamido oxa*N*-4-yl carbonate (19) is prepared from 4-{[({*N-*[(*tert*-butoxy)carbonyl](2-bromobenzene)sulfonamido}oxy)carbonyl]oxy}-oxane according to General Method 6. (0.34g, 37%), ¹H NMR (500 MHz, CHLOROFORM-d) δ ppm 8.75 (1H, s), 8.19 - 8.24 (1H, m), 7.78 - 7.84 (1H, m), 7.50 - 7.58 (2H, m), 4.82 (1H, tt, 8.3, 4.1Hz), 3.80 - 3.89 (2H, m), 3.46 - 3.53 (2H, m), 1.85 - 1.94 (2H, m), 1.65 (2H, m).

### Preparation of (1-acetylpiperidiN-4-yl)(2-methanesulfonylbenzene)sulfonamido carbonate (20)

1-(4-{[({[(*tert*-Butoxy)carbonyl]amino}oxy)carbonyl]oxy}piperidi*N*-1-yl)etha*N*-1-one is prepared from *tert*-butyl *N*-hydroxycarbamate and 1-(4-hydroxypiperidi*N*-1-yl)etha*N-*1-one using 20% solution of phosgene in toluene according to General Method 3. (0.29g, 7%), ¹H NMR (500 MHz, CHLOROFORM-*d*) δ ppm 7.68 (1H, s), 4.98 (1H, tt, 7.1, 3.6Hz), 3.76 - 3.85 (1H, m), 3.66 (1H, dt, 17.8, 3.8Hz), 3.53 - 3.60 (1H, m), 3.40 (1H, ddd, 13.8, 7.6, 3.8Hz), 2.12 (3H, s), 1.90 - 2.03 (2H, m), 1.75 - 1.89 (2H, m), 1.51 (9H, s).

(1-Acetylpiperidi*N*-4-yl)(2-methanesulfonylbenzene)sulfonamido carbonate (20) is prepared from 1-(4-{[({[(*tert*-butoxy)carbonyl]amino}oxy)carbonyl]oxy}-piperidi*N-*1-yl)etha*N*-1-one and 2-methylsulfonyl benzene sulfonyl chloride according to General Method 5. Purification of the compound from this reaction by column chromatography afforded the title compound directly without need for formal deprotection. (0.037g, 7%), ¹H NMR (500 MHz, CHLOROFORM-d) δ ppm 9.69 (1H, s), 8.38 (1H, dd, 7.6, 1.4Hz), 8.34 (1H, dd, 7.6, 1.4Hz), 7.93 (2H, m), 4.93 (1H, tt, 7.2, 3.5Hz), 3.73 - 3.83 (1H, m), 3.57 - 3.66 (1H, m), 3.47 - 3.55 (1H, m), 3.45 (3H, s), 3.31 - 3.41 (1H, m), 2.10 (3H, s), 1.84 - 2.01 (2H, m), 1.64 - 1.81 (2H, m).

### Preparation of 2-methanesulfonyl-N-[(methoxycarbonyl)oxy]benzene-1-Sulfonamide (21)

2-({[(Methoxycarbonyl)oxy]carbamoyl}oxy)-2-methylpropane is prepared from methyl chloroformate and from *tert*-butyl *N*-hydroxycarbamate. To a solution of *tert*-butyl *N-*hydroxycarbamate (1.4g, 10.6 mmol) in DCM (10 ml) is added triethylamine (1.5ml, 10.6 mmol) at 0°C. Methyl choroformate (814µl, 10.6 mmol) is added dropwise and the reaction is stirred for 18 hours at room temperature before being washed with water (2 x 10ml) and NaHCO₃ (2 x 10ml). The organics are dried over magnesium sulfate and concentrated *in vacuo* to give the title product as an oil. (1.76g, 87%), ¹H NMR (500 MHz, CHLOROFORM-d) δ ppm 7.71 (1H, br. s.), 3.92 (3H, s), 1.50 (9H, s).

1-({[(*tert*-Butoxy)carbonyl][(methoxycarbonyl)oxy]amino}sulfonyl)-2-methanesulfonyl benzene is prepared from 2-({[(methoxycarbonyl)oxy]carbamoyl}oxy)-2-methylpropane according to General Method 5. (0.96g, 60%), ¹H NMR (500 MHz, CHLOROFORM-*d*) δ ppm 8.38 - 8.47 (2H, m), 7.82 - 7.90 (2H, m), 3.99 (3H, s), 3.42 (3H, s), 1.40 - 1.47 (9H, m).

2-Methanesulfonyl-*N*-[(methoxycarbonyl)oxylbenzene-1-sulfonamide (21) is prepared from 1-({[(*tert*-butoxy)carbonyl][(methoxycarbonyl)oxy]amino}sulfonyl)-2-methane sulfonyl benzene according to General Method 6. (0.65g, 90%), ¹H NMR (500 MHz, CHLOROFORM-d) δ ppm 9.64 (1H, s), 8.32 - 8.41 (2H, m), 7.87 - 7.97 (2H, m), 3.87 (3H, s), 3.45 (3H, s).

### Preparation of 2-methanesulfonyl-N-{[(2-methoxyethoxy)carbonyl]oxy}benzene-1-sulfonamide (22)

1-{[({[(*tert*-Butoxy)carbonyl]amino}oxy)carbonyl]oxy}-2-methoxyethane is prepared from 2-methoxyethyl chloroformate and *tert*-butyl *N*-hydroxycarbamate. To a solution of *tert-*butyl *N*-hydroxycarbamate (1.5g, 11.3 mmol) in DCM (50 ml) is added triethylamine (1.6ml, 11.3 mmol) at 0°C. 2-methoxyethyl chloroformate (1.56g, 11.3 mmol) is added drop wise and the reaction stirred for 18 hours at room temperature before being washed with 0.1M HCl (10 ml), dried over sodium sulfate and concentrated *in vacuo.* The reaction mixture is purified by silica column chromatography eluting with heptane: ethyl acetate (4:1; v:v) to yield the title compound. (0.67g, 25%), ¹H NMR (500 MHz, CHLOROFORM-d) δ ppm 8.04 (1H, br. s.), 4.32 - 4.44 (2H, m), 3.57 - 3.68 (2H, m), 3.31 - 3.44 (3H, m), 1.41 - 1.56 (9H, m).

1-({[(*tert*-Butoxy)carbonyl]({[(2-methoxyethoxy)carbonyl]oxy})amino}-sulfonyl)-2-methanesulfonylbenzene is prepared from 1-{[({[(*tert*butoxy)carbonyl]-amino}oxy)carbonyl]oxy}-2-methoxyethane according to General Method 5 and is used directly in the synthesis of 2-methanesulfonyl-*N*-{[(2-methoxyethoxy)carbonyl]-oxy}benzene-1-sulfonamide without additional purification (0.59g).

2-Methanesulfonyl-*N*-{[(2-methoxyethoxy)carbonyl]oxy}benzene-1-sulfonamide (22) is prepared from 1-({[(*tert*-butoxy)carbonyl]({[(2-methoxyethoxy)carbonyl]oxy})amino} sulfonyl)-2-methanesulfonylbenzene according to General Method 6. (0.15g, 15% over two steps), ¹H NMR (250 MHz, CHLOROFORM-d) δ ppm 9.66 (1H, s), 8.28 - 8.42 (2H, m), 7.82 - 7.99 (2H, m), 4.29 - 4.42 (2H, m), 3.54 - 3.65 (2H, m), 3.44 (3H, s), 3.31 - 3.39 (3H, m).

### Preparation of 2-methanesulfonyl-N-({[2-(2-methoxyethoxy)ethoxy]carbonyl}oxy)benzene-1-sulfonamide (23)

1-(2-{[({[(*tert*-Butoxy)carbonyl]amino}oxy)carbonyl]oxy}ethoxy)-2-methoxyethane is prepared from *tert*-butyl *N*-hydroxycarbamate and 2-(2-methoxyethoxy)ethanol using 20% solution of phosgene in toluene according to General Method 3. (9.95g, 82%), ¹H NMR (250 MHz, CHLOROFORM-d) δ ppm 7.88 (1H, s), 4.36 - 4.45 (2H, m), 3.72 - 3.80 (2H, m), 3.61 - 3.68 (2H, m), 3.51 - 3.58 (2H, m), 3.37 (3H, s), 1.49 (9H, s).

1-({[(*tert*-Butoxy)carbonyl]({[2-(2-methoxyethoxy)ethoxy]carbonyl}oxy)amino}-sulfonyl)-2-methanesulfonylbenzene is prepared from 1-(2-{[({[(*tert*-butoxy)carbonyl]amino} oxy)carbonyl]oxy}ethoxy)-2-methoxyethane and 2-methylsulfonyl benzene sulfonyl chloride according to General Method 5. (2.72g, 70%), ¹H NMR (500 MHz, CHLOROFORM-*d*) δ ppm 8.40 (2H, ddd, 12.7, 7.5, 1.5Hz), 7.86 (2 H, m), 4.37 - 4.56 (2H,m), 3.71 - 3.85 (2H, m), 3.62 - 3.71 (2H, m), 3.50 - 3.60 (2H, m), 3.43 (3H, s), 3.39 (3H, s), 1.43 (9H, s).

2-Methanesulfonyl-*N*-({[2-(2-methoxyethoxy)ethoxy]carbonyl}oxy)benzene-1-sulfonamide (23) is prepared from 1-({[(*tert*-butoxy)carbonyl]({[2-(2-methoxyethoxy) ethoxy]carbonyl}oxy)amino}sulfonyl)-2-methanesulfonylbenzene to General Method 6. (0.55g, 54%), ¹H NMR (500 MHz, CHLOROFORM-d) δ ppm 9.65 (1H, s), 8.36 (2H, ddd, 9.3, 7.7, 1.6Hz), 7.82 - 8.02 (2H, m), 4.28 - 4.42 (2H, m), 3.67 - 3.74 (2H, m), 3.59 - 3.66 (2H, m), 3.51 - 3.57 (2H, m), 3.44 (3H, s), 3.38 (3H, s).

### Preparation of (4S)-4-[({[(2-methanesulfonylbenzene)sulfonamidooxy]carbonyl}oxy)methyl]-2,2-dimethyl-1,3-dioxolane (24)

(4S)-4-({[({[(*tert*-Butoxy)carbonyl]amino}oxy)carbonyl]oxy}methyl)-2,2-dimethyl-1,3-dioxolane is prepared from *tert*-butyl *N*-hydroxycarbamate and [(4R)-2,2-dimethyl-1,3-dioxola*N*-4-yl]methanol using 20% solution of phosgene in toluene according to General Method 3. (9.8g, 90%), ¹H NMR (500 MHz, CHLOROFORM-*d*) δ ppm 7.76 (1H, s), 4.33 - 4.41 (1H, m), 4.26 - 4.31 (2H, m), 4.08 - 4.16 (1H, m), 3.82 (1H, dd, 8.7, 5.6Hz), 1.50 (9H, s), 1.44 (3H, s), 1.36 (3H, s).

(4S)-4-[({[(2-Methanesulfonylbenzene)sulfonamidooxy]carbonyl}oxy)-methyl]-2,2-dimethyl-1,3-dioxolane (24) is prepared from (4S)-4-({[({[(*tert*-butoxy)carbonyl]amino} oxy)carbonyl]oxy}methyl)-2,2-dimethyl-1,3-dioxolane is and 2-methylsulfonyl benzene sulfonyl chloride according to General Method 5. Purification of the compound from this reaction by column chromatography afforded the title compound directly without need for formal deprotection. (0.2g, 7%), ¹H NMR (500 MHz, CHLOROFORM-*d*) δ ppm 8.36 (2H, ddd, 15.0, 7.7, 1.5Hz), 7.91 (2H, ddd, 12.7, 7.6, 1.5Hz), 4.18 - 4.35 (3H, m), 4.07 (1H, dd, 8.8, 6.4Hz), 3.74 (1H, dd, 8.7, 5.5Hz), 3.44 (3H, s), 1.41 (3H, s), 1.35 (3H, s).

### Preparation of N-({[(1,3-diethoxypropaN-2-yl)oxy]carbonyl}oxy)-2-methanesulfonylbenzene-1-sulfonamide (25)

2-{[({[(*tert*-Butoxy)carbonyl]amino}oxy)carbonyl]oxy}-1,3-diethoxypropane is prepared from *tert*-butyl *N*-hydroxycarbamate and 1,3-diethoxypropa*N*-2-ol using 20% solution of phosgene in toluene according to General Method 3. (10.37g, 85%), ¹H NMR (500 MHz, CHLOROFORM-*d*) δ ppm 7.73 (1H, s), 5.03 (1H, quin, 5.2Hz), 3.64 (4H, dd, 5.1, 2.7Hz), 3.48 - 3.57 (4H, m), 1.50 (9H, s), 1.19 (6H, t, 7.0Hz).

1-({[(*tert*-Butoxy)carbonyl]({[(1,3-diethoxypropa*N*-2-yl)oxy]carbonyl}-oxy)amino} sulfonyl)-2-methanesulfonylbenzene is prepared from 2-{[({[(*tert*-butoxy)carbonyl] amino}oxy)carbonyl]oxy}-1,3-diethoxypropane and 2-methylsulfonyl benzene sulfonyl chloride according to General Method 5. (2.3g, 47%), ¹H NMR (500 MHz, CHLOROFORM-d) δ ppm 8.39 (2H, ddd, 12.2, 7.5, 1.5Hz), 7.84 (2H, m), 5.06 (1H, quin, 5.1Hz), 3.68 (4H, t, 4.9Hz), 3.48 - 3.61 (4H, m), 1.43 (9H, s), 1.27 (3H, t, 7.2Hz), 1.19 (3H, t, 6.9Hz).

*N*-({[(1,3-Diethoxypropa*N*-2-yl)oxy]carbonyl}oxy)-2-methanesulfonyl-benzene-1-sulfonamide (25) is prepared from 1-({[(*tert*-butoxy)carbonyl]({[(1,3-diethoxypropa*N*-2-yl)oxy]carbonyl}oxy)amino} sulfonyl)-2-methanesulfonylbenzene according to General Method 6. (1.16g, 62%), ¹H NMR (500 MHz, CHLOROFORM-d) δ ppm 9.66 (1H, s), 8.35 (2H, ddd, 13.7, 7.6, 1.5Hz), 7.84 - 7.94 (2H, m), 4.87 - 5.05 (1H, m), 3.54- 3.63 (4H, m), 3.50 (4H, m), 3.44 (3H, s), 1.18 (6H, t, 7.0Hz).

### Preparation of 3-({[(2-methanesulfonylbenzene)sulfonamidooxy]carbonyl}oxy)-propane-1,2-diol (26)

To a solution of (4S)-4-[({[(2-methanesulfonylbenzene)sulfonamidooxy] carbonyl}oxy)methyl]-2,2-dimethyl-1,3-dioxolane (0.4g, 0.98 mmol) in acetonitrile (10 ml) is added 1M HCl (1 ml). The reaction is stirred at room temperature for 4 hours, where upon LC-MS showed complete consumption of starting material. The reaction mixture is concentrated *in vacuo* and the title compound isolated by trituation with diethyl ether. (0.03g, 3%), ¹H NMR (500 MHz, DMSO-d6) δ ppm 10.90 - 11.05 (1H, m), 8.23 - 8.34 (1H, m), 8.14 - 8.22 (1H, m), 8.00 - 8.09 (2H, m), 4.98 - 5.09 (1H, m), 4.65 - 4.78 (1H, m), 4.15 - 4.23 (1H, m), 3.97 - 4.08 (1H, m), 3.56 - 3.69 (1H, m), 3.46 (3H, s).

### Preparation of 4-({[(2-methanesulfonylbenzene)sulfonamidooxy]carbonyl}-oxy)butaN-1-ol (27)

(4-{[({[(*tert*-Butoxy)carbonyl]amino}oxy)carbonyl]oxy}butoxy)(*tert-*butyl)dimethylsilane is prepared from 4-[(*tert*-butyldimethylsilyl)oxy]buta*N*-1-ol and *tert*-butyl *N*-hydroxycarbamate using 20% solution of phosgene in toluene according to General Method 3. (1.73g, 49%), ¹H NMR (250 MHz, CHLOROFORM-*d*) δ ppm 7.63 (1H, s), 4.30 (2H, t, 6.6Hz), 3.64 (2H, t, 6.1Hz), 1.73 - 1.87 (2H, m), 1.56 - 1.66 (2H, m), 1.51 (9H, s), 0.89 (9H, s), 0.05 (6H, s).

(4-{[({*N*-[(*tert*-Butoxy)carbonyl](2-methanesulfonylbenzene)sulfonamido}-oxy)carbonyl]oxy}butoxy)(*tert*-butyl)dimethylsilane is prepared from (4-{[({[(*tert-*butoxy)carbonyl]amino}oxy)carbonyl]oxy}butoxy)(*tert*-butyl)dimethylsilane and 2-methylsulfonyl benzene sulfonyl chloride according to General Method 5. (0.53g, 31%), ¹H NMR (250 MHz, CHLOROFORM-*d*) δ ppm 8.25 - 8.45 (2H, m), 7.79 - 7.98 (2H, m), 4.11 - 4.33 (2H, m), 3.50 - 3.69 (2H, m), 3.34 - 3.45 (3H, m), 1.36 - 1.87 (13H, m), 0.86 (9H, s), 0.11 (6H, s).

### 4-({[(2-Methanesulfonylbenzene)sulfonamidooxy]carbonyl}oxy)butaN-1-ol (27)

To a solution of (4-{[({*N*-[(*tert*-butoxy)carbonyl](2-methanesulfonylbenzene) sulfonamido}oxy)carbonyl]oxy}butoxy)(*tert*-butyl)dimethylsilane (0.51g, 0.8 mmol) in THF (20 ml) is added TBAF (1.05 ml of a 1M solution in THF, 1.05mml). The resulting solution is stirred at room temperature for 3 hours, where upon water is added (5 ml) and the reaction mixture diluted with DCM (20 ml). The organics are dried over sodium sulphate, filtered and concentrated *in vacuo* to yield the crude product which is purified by silica column chromatography eluting with a heptanes: ethyl acetate gradient followed by additional purification by reverse phase preparatory HPLC. (0.02g, 7%), ¹H NMR (500 MHz, CHLOROFORM-d) δ ppm 9.31 - 9.90 (1H, m), 8.35 (2H, ddd, 14.9, 7.5, 1.6Hz), 7.91 (2H, ddd, 9.4, 7.6, 1.4Hz), 4.26 (2H, t, 6.5Hz), 3.66 (2H, t, 6.3Hz), 3.39 - 3.47 (3H, m), 1.71 - 1.83 (2H, m), 1.56 - 1.65 (2H, m).

### Preparation of 2-({[(2-methanesulfonylbenzene)sulfonamidooxy]carbonyl}oxy)-ethaN-1-ol (28)

### 2-[(tert-Butyldimethylsilyl)oxy]ethaN-1-ol

To a solution of ethane-1,2-diol (2 g, 32.22 mmol) in THF (50 ml) is added sodium hydride (60%, 1.29 g, 32.22 mmol) portionwise. The reaction mixture is stirred for 1 hour before *tert*-butyl(chloro)dimethylsilane (4.86 g, 32.22 mmol) is added as a solution in THF (15 ml) and stirring is continued for 2 hours. The reaction mixture is diluted with diethyl ether (120 ml) and washed with sodium bicarbonate solution, water and brine before the organic portion dried over sodium sulfate, filtered and concentrated *in vacuo* to yield the title compound as a clear oil. (5.4g, 96%), ¹H NMR (250 MHz, CHLOROFORM-*d*) δ ppm 2.80 (4H, d, 4.9Hz), 1.33 (9H, s), 1.23 (6H, s).

### (2-{[({[(tert-Butoxy)carbonyl]amino}oxy)carbonyl]oxy}ethoxy)(tert-butyl)-dimethylsilane

To a solution of 2-[(*tert*-butyldimethylsilyl)oxy]etha*N*-1-ol (5.44 g, 30.85 mmol) in THF (50 ml) cooled to -5°C is added diphosgene (1.85 ml, 15.43 mmol) and pyridine (2.5 ml, 30.85 mmol). The reaction is stirred for 5 minutes before addition of *tert*-butyl hydroxycarbamate (4.11 g, 30.85 mmol) and pyridine (2.5 ml, 30.85 mmol). The reaction mixture is stirred for 30 minutes, filtered through celite™ and concentrated *in vacuo.* The resulting crude product is redissolved in diethyl ether (50 ml), washed with copper sulphate solution (2 x 20 ml) and the organics collected, dried over sodium sulphate, filtered and concentrated *in vacuo.* The title compound is isolated after purification by silica column chromatography eluting with heptanes:ethyl acetate. (6.7g, 65%), ¹H NMR (500 MHz, CHLOROFORM-*d*) δ ppm 7.62 (1H, s), 4.30 - 4.35 (2H, m), 3.83 - 3.90 (2H, m), 1.51 (9H, s), 0.90 (9H, s), 0.08 (6H, s).

(2-{[({*N*-[(*tert*-Butoxy)carbonyl](2-methanesulfonylbenzene)sulfonamido}-oxy)carbonyl]oxy}ethoxy)(*tert*-butyl)dimethylsilane is prepared from (2-{[({[(*tert-*butoxy)carbonyl]amino}oxy)carbonyl]oxy}ethoxy)(*tert*-butyl)dimethylsilane and 2-methylsulfonyl benzene sulfonyl chloride according to General Method 5. (3.3g, 77%), ¹H NMR (250 MHz, CHLOROFORM-*d*) δ ppm 8.23 - 8.40 (2H, m), 7.69 - 7.83 (2H, m), 4.30 (2H, t, 5.0Hz), 3.81 (2H, t, 5.1Hz), 3.30 - 3.39 (3H, m), 1.34 (9H, s), 0.79 - 0.83 (9H, m), -0.03 - 0.03 (6H, m).

### 2-{[({N-[(tert-butoxy)carbonyl](2-methanesulfonylbenzene)sulfonamido}-oxy)carbonyl]oxy}ethaN-1-ol

To a solution of (2-{[({*N*-[(*tert*-butoxy)carbonyl](2-methanesulfonylbenzene) sulfonamido}oxy)carbonyl]oxy}ethoxy)(*tert*-butyl)dimethylsilane (2.7 g, 4.88 mmol) in DCM (20 ml) is added HCl (4.88 ml of a 4M solution in dioxane, 19.5 mmol) and the resulting solution is stirred for 2 hours after which time the solvents are removed *in vacuo* and the reaction mixture is washed with water (5 ml), dried over sodium sulphate, filtered and concentrated *in vacuo.* The compound is used directly for the synthesis of 2-({[(2 methanesulfonylbenzene)sulfonamidooxy]carbonyl}oxy)etha*N-*1-ol without further purification (1.43g, 32%).

2-({[(2-Methanesulfonylbenzene)sulfonamidooxy]carbonyl}oxy)etha*N*-1-ol (28) is prepared from 2-{[({*N*-[(*tert*-butoxy)carbonyl](2-methanesulfonylbenzene) sulfonamido}oxy)carbonyl]oxy}etha*N-*1-ol according to General Method 6. (0.26g, 24%), ¹H NMR (500 MHz, DMSO-d6) δ ppm 10.98 (1H, br. s.), 8.29 (1H, dd, 7.4, 1.7Hz), 8.18 (1H, dd, 7.2, 1.8Hz), 8.01 - 8.11 (2H, m), 4.11 - 4.19 (2H, m), 3.51 - 3.58 (2H, m), 3.46 (3H, s).

### Preparation of [2-Chloro-5-(dimethylcarbamoyl)benzene]sulfonamido-2,2-dimethylpropanoate (50)

### 4-Chloro-3-(chlorosulfonyl)benzoic acid

The following method for the chorosulfonylation of benzoic acids is described in Bioorg. Med. Chem. 2002, 639-656:
To a flask containing chlorosulfonic acid (17 ml, 250 mmol) cooled to 0°C is added 4-chlorobenzoic acid (5.2 g, 33.3 mmol) portionwise. The reaction mixture is heated to 130°C for 24 hours or until complete consumption of the starting material. The reaction mixture is cooled to ambient temperature before careful addition to ice. The resulting solid is filtered and washed with cold water (50 ml). The wet product is dissolved in diethyl ether (100 ml), dried over sodium sulfate, filtered and concentrated *in vacuo* to yield the title compound without need for additional purification. (6.1 g, 71%), ¹H NMR (500 MHz, MeOD) δ ppm 8.57 (1H, s), 7.42 - 7.76 (2H, m).

### 4-Chloro-3-(chlorosulfonyl)benzoyl chloride

4-Chloro-3-(chlorosulfonyl)benzoic acid (6.1 g, 24 mmol) is suspended in toluene (50 ml). Thionyl chloride (3.5 ml, 47 mmol) is added dropwise, and the mixture is heated to reflux for 14 hours under nitrogen until complete consumption of the carboxylic acid is observed by LCMS. The reaction mixture is concentrated to dryness to afford the expected acid chloride which is used for next step without further purification or analysis.

### 2-Chloro-5-(dimethylcarbamoyl)benzene-1-sulfonyl chloride

The following method is described in Journal of Pharmacy and Pharmacology 1963, 202-211 :
Dimethylamine hydrochloride (0.5 g, 6.2 mmol) is added to a stirred solution of 4-chloro-3-(chlorosulfonyl)benzoyl chloride (1.6 g, 5.88 mmol) in chlorobenzene (10 ml). The reaction mixture is heated to reflux for 2 hours, until complete consumption of the starting material is observed by LCMS. The reaction mixture is concentrated to dryness and the residue is taken up in diethyl ether (20 ml). The precipitate is filtered and washed with diethyl ether (2 x 10 ml), to afford the title compound (1.1 g, 64%). ¹H NMR (500 MHz, DMSO-d6) δ ppm 7.86 (1H, d, 2.0Hz), 7.43 (1H, d, 8.1Hz), 7.34 (1H, dd, 8.1, 2.2Hz), 2.97 (3H, br. s), 2.90 (3H, br. s).

*N*-[(*tert*-Butoxy)carbonyl][2-chloro-5-(dimethylcarbamoyl)benzene]sulfonamido 2,2-dimethylpropanoate is synthesised from 2-chloro-5-(dimethylcarbamoyl)benzene-1-sulfonyl chloride, sodium hydride and [(*tert*-butoxy)carbonyl]amino 2,2-dimethylpropanoate according to General Method 4. (0.5g, 40%), ¹H NMR (250 MHz, DMSO-d6) δ ppm 8.04 (1H, s), 7.46 - 7.79 (2H, m), 2.92 (3H, br. s.), 2.83 (3H, br. s.), 1.18 (9H, s) 1.12 (9H, s).

[2-Chloro-5-(dimethylcarbamoyl)benzene]sulfonamido-2,2-dimethylpropanoate (50) is prepared from *N*-[(*tert*-butoxy)carbonyl][2-chloro-5-(dimethylcarbamoyl)benzene]sulfonamido 2,2-dimethylpropanoate according to General Method 6. (0.24g, 19%), ¹H NMR (250 MHz, DMSO-*d*₆) δ ppm11.58 (1H, br. s.), 7.96 (1H, d, 1.5Hz), 7.76 - 7.82 (2H, m), 2.99 (3H, s), 2.90 (3H, s), 1.06 (9H, s).

### Preparation of (2-methanesulfonylbenzene) sulfonamido 2-(acetyloxy) benzoate (51)

[(*tert*-Butoxy)carbonyl]amino 2-(acetyloxy)benzoate is prepared from acetylsalicyloyl chloride and *N-tert*-butoxycarbonyl hydroxylamine according to General Method 1 described by Carpino et al. J. Am. Chem. Soc. 1959, 955-957. (9.0g, 81%), ¹H NMR (250 MHz, DMSO-d6) δ ppm 10.89 (1H, br. s.), 7.97 (1H, dd, 7.8, 1.7Hz), 7.75 (1H, td, 7.8, 1.8Hz), 7.46 (1H, td, 7.6, 1.1Hz), 7.31 (1H, dd, 8.1, 0.9Hz), 2.27 (3H, s), 1.42 (9H, s).

*N*-[(*tert*-Butoxy)carbonyl](2-methanesulfonylbenzene)sulfonamido 2-(acetyloxy) benzoate is synthesised from 2-methylsulfonylbenzenesulfonyl chloride, sodium hydride and [(*tert*-butoxy)carbonyl]amino 2-(acetyloxy)benzoate according to General Method 4. (5.5g, 89%), ¹H NMR (250 MHz, DMSO-d6) δ ppm 8.34 - 8.43 (1H, m), 8.07 - 8.21 (3H, m), 7.94 - 8.05 (1H, m), 7.77 (1H, td, 7.9, 1.8Hz), 7.56 - 7.66 (1H, m), 7.07 - 7.16 (1H, m), 3.45 (3H, s), 2.48 (3H, s.), 1.43 (9H, s).

(2-Methanesulfonylbenzene) sulfonamido 2-(acetyloxy) benzoate (51) is prepared from *N*-[(*tert*-butoxy)carbonyl](2-methanesulfonylbenzene)sulfonamido 2-(acetyloxy)benzoate according to General Method 6. (0.93g, 22%), ¹H NMR (500 MHz, DMSO-*d*₆) δ ppm 12.04 (1H, br. s.), 8.39 (1H, d, 7.6Hz), 8.08 - 8.21 (2H, m), 7.93 - 8.05 (2H, m), 7.67 (1H, t, 7.3Hz), 7.48 - 7.61 (1H, m), 7.00 (1H, d, 8.2Hz), 3.47 (3H, s), 1.94 (3H, s).

### Preparation of (2-methanesulfonylbenzene)sulfonamido 2-[4-(2-methylpropyl)-phenyl]propanoate (52)

[(*tert*-Butoxy)carbonyl]amino 2-[4-(2-methylpropyl)phenyl]propanoate is prepared from 2-[4-(2-methylpropyl)phenyl]propanoyl chloride (which is prepared from 2-[4-(2-methylpropyl)phenyl]propanoic acid according to the method detailed in Journal of Organic Chemistry 1991, 56, 2395-2400) and *N-tert*-butoxycarbonyl hydroxylamine using literature conditions. (5.49g, 73%), ¹H NMR (250 MHz, DMSO-d6) δ ppm 7.07 - 7.29 (4H, m), 3.89 (1H, q, 7.1Hz), 2.42 (2H, d, 7.2Hz), 1.80 (1H, sept, 6.8Hz), 1.40 (3H, d, 4.3Hz), 1.35 (9H, s), 0.85 (6H, d, 6.5Hz).

*N*-[(*tert*-Butoxy)carbonyl](2-methanesulfonylbenzene)sulfonamido 2-[4-(2-methylpropyl)phenyl]propanoate is synthesised from 2-methylsulfonylbenzene sulfonyl chloride, sodium hydride and [(*tert*-butoxy)carbonyl]amino 2-[4-(2-methylpropyl)phenyl] propanoate according to General Method 4 and used directly in the synthesis of (2-methanesulfonylbenzene)sulfonamido2-[4-(2-methylpropyl) phenyl]propanoate.

(2-Methanesulfonylbenzene) sulfonamido 2-[4-(2-methylpropyl)phenyl]-propanoate (52) is prepared from *N*-[(*tert*-butoxy)carbonyl](2-methanesulfonylbenzene)sulfonamido 2-[4-(2-methylpropyl)phenyl]propanoate according to General Method 6. (2.19g, 29% over 2 steps), ¹H NMR (250 MHz,DMSO-d6) δ ppm 10.53, (1H, br.s.), 8.21,(1H, dd, 7.9, 1.1Hz), 7.95 - 8.05 (1H, m), 7.71 - 7.87 (2H, m), 6.94 - 7.16 (4H, m), 3.78 (1H, q, 7.0Hz), 3.41 (3H, s), 2.42 (2H, d, 7.2Hz), 1.81 (1H, sept, 7.1Hz), 1.28 (3H, d, 7.2Hz), 0.84 (6H, d, 6. 5Hz).

### Preparation of (2-bromobenzene)sulfonamido benzoate (53)

*N*-[(*tert*-Butoxy)carbonyl](2-bromobenzene)sulfonamido benzoate is synthesised from 2-bromobenzenesulfonyl chloride, sodium hydride and [(*tert*-butoxy)carbonyl]amino benzoate according to General Method 4. (4.8g, 87%), ¹H NMR (500 MHz, CHLOROFORM-d) δ ppm 8.34 (1H, dd, 7.6, 2.1Hz), 8.12 - 8.22 (2H, m), 7.82 (1H, dd, 7.5, 1.7Hz), 7.63 - 7.70 (1H, m), 7.48 - 7.57 (4H, m), 1.39 (9H, s).

(2-Bromobenzene)sulfonamido benzoate (53) is prepared from *N*-[(*tert-*butoxy)carbonyl](2-bromobenzene)sulfonamido benzoate according to General Method 6. (2.4g, 52%), ¹H NMR (250 MHz, DMSO-d6) δ ppm 11.78 (1H, br. s.), 8.02 - 8.13 (1H, m), 7.89 - 8.00 (1H, m), 7.76 - 7.88 (2H, m), 7.59 - 7.75 (3H, m), 7.47 - 7.58 (2H, m).

### Preparation of (2-bromobenzene)sulfonamido 2-methylpropanoate (54)

*N*-[(*tert*-Butoxy)carbonyl](2-bromobenzene)sulfonamido 2-methylpropanoate is prepared from 2-bromobenzene sulfonyl chloride, sodium hydride and [(*tert-*butoxy)carbonyl]amino 2-methylpropanoate according to General Method 4. (2.57g, 77%), ¹H NMR (250 MHz, CHLOROFORM-*d*) δ ppm 8.23 - 8.37 (1H, m), 7.72 - 7.88 (1H, m), 7.42 - 7.59 (2H, m), 2.67 - 3.02 (1H, m), 1.37 (9H, s), 1.34 (3H, s), 1.32 (3H, s).

(2-Bromobenzene)sulfonamido 2-methylpropanoate (54) is prepared from *N*-[(*tert-*butoxy)carbonyl](2-bromobenzene)sulfonamido 2-methylpropanoate according to General Method 6. (1.41g, 72%), ¹H NMR (250 MHz, CHLOROFORM-*d*) δ ppm 9.60 (1H, s), 8.08 - 8.29 (1H, m), 7.72 - 7.94 (1H, m), 7.40 - 7.66 (2H, m), 2.46 - 2.57 (1 H, m), 0.98 (6H, d, 6.9Hz).

### Preparation of (2-chlorobenzene)sulfonamido 2,2-dimethylpropanoate (55)

*N*-[(*tert*-Butoxy)carbonyl](2-chlorobenzene)sulfonamido 2,2-dimethylpropanoate is prepared from 2-chlorobenzenesulfonyl chloride, sodium hydride and [(*tert-*butoxy)carbonyl]amino 2,2-dimethylpropanoate according to General Method 4. (4.1g, 81%), ¹H NMR (250 MHz, CHLOROFORM-d) δ ppm 7.63 - 7.70 (1H, m), 7.55 - 7.6 (2H, m), 7.40 - 7.50 (1H, m), 1.38 (9H, s), 1.37 (9H, s).

(2-Chlorobenzene)sulfonamido 2,2-dimethylpropanoate (55) is prepared from *N*-[(*tert-*butoxy)carbonyl](2-chlorobenzene)sulfonamido 2,2-dimethylpropanoate according to General Method 6.(1.46g, 48%), ¹H NMR (250 MHz, CHLOROFORM-d) δ ppm 9.57 (1H, s), 8.13 (1H, dd, 8.1, 1.1Hz), 7.55 - 7.66 (2H, m), 7.40 - 7.54 (1H, m), 1.02 (9H, s).

### Preparation of [2-chloro-5-(dimethylcarbamoyl)benzenelsulfonamido acetate (56)

*N*-[(*tert*-Butoxy)carbonyl][2-chloro-5 (dimethylcarbamoyl)benzene]-sulfonamidoacetate is prepared from 2-chloro-5-(dimethylcarbamoyl)benzene-1-sulfonyl chloride, sodium hydride and [(*tert*-butoxy)carbonyl]amino acetate according to General Method 4. (1.2g, 95%), ¹H NMR (500 MHz, DMSO-*d*₆) δ ppm 8.11 (1H, s), 7.87 (2H, s), 3.00 (3H, s), 2.91 (3H, s), 1.40 (3H, s), 1.29 (9H, s).

[2-Chloro-5-(dimethylcarbamoyl)benzenelsulfonamido acetate (56) is prepared from N-[(*tert*-butoxy)carbonyl][2-chloro-5-(dimethylcarbamoyl)benzene]-sulfonamido acetate according to General Method 6. (0.22g, 24%), ¹H NMR (500 MHz, DMSO-d6) δ ppm 11.62 (1H, br. s.), 7.97 (1H, d, 1.6Hz), 7.74 - 7.84 (2H, m), 3.00 (3H, s), 2.91 (3H, s), 2.07 (3H, s).

### Preparation of [2-chloro-5-(dimethylcarbamoyl)benzenelsulfonamido 2-(acetyloxy)benzoate (57)

*N*-[(*tert*-Butoxy)carbonyl][2-chloro-5-(dimethylcarbamoyl)benzene]sulfonamido 2-(acetyloxy)benzoate is prepared from 2-chloro-5-(dimethylcarbamoyl)benzene-1-sulfonyl chloride, sodium hydride and [(*tert*-butoxy)carbonyl]amino 2-(acetyloxy)benzoate according to General Method 4. The compound is used directly for the synthesis of [2-chloro-5-(dimethylcarbamoyl)benzene]sulfonamido 2-(acetyloxy)benzoate without full characterisation.

[2-Chloro-5-(dimethylcarbamoyl)benzene]sulfonamido 2-(acetyloxy)benzoate (57) is prepared from *N*-[(*tert*-butoxy)carbonyl][2-chloro-5-(dimethylcarbamoyl)benzene]sulfonamido 2-(acetyloxy)benzoate according to General Method 6. (0.22g, 17% over two steps), ¹H NMR (500 MHz, DMSO-d6) δ ppm 12.01 (1H, br. s.), 7.75 - 8.04 (4H, m), 7.46 - 7.72 (2H, m), 7.01 (1H, d, 8.1Hz), 2.95 (3H, br. s.), 2.80 (3H, br. s.), 1.93 (3H, s).

### Preparation of (2-chlorobenzene)sulfonamido 2-methylpropanoate (58)

*N*-[(*tert*-Butoxy)carbonyl](2-chlorobenzene)sulfonamido 2-methylpropanoate is prepared from 2-chlorobenzene sulfonyl chloride, sodium hydride and [(*tert-*butoxy)carbonyl]amino 2-methylpropanoate according to General Method 4. (3.4g, 91%), ¹H NMR (250 MHz, CHLOROFORM-d) δ ppm 8.16 - 8.29 (1H, m), 7.52 - 7.63 (2H, m), 7.39 - 7.51 (1H, m), 2.86 (1H, quin, 7.0Hz), 1.38 (9H,s), 1.33 (6H, d, 7.0Hz).

(2-Chlorobenzene)sulfonamido 2-methylpropanoate (58) is prepared from *N*-[(*tert-*butoxy)carbonyl](2-bromobenzene)sulfonamido 2-methylpropanoate according to General Method 6. (1.53g, 61%), ¹H NMR (250 MHz, CHLOROFORM-d) δ ppm 9.52 (1H, s), 8.08 - 8.20 (1H, m), 7.54 - 7.68 (2H, m), 7.40 - 7.54 (1H, m), 2.51 (1H, sept, 7.0Hz), 0.97 (6H, d, 7.0Hz).

### Preparation of (2-bromobenzene)sulfonamido 2-phenylacetate (59)

[(*tert*-Butoxy)carbonyl]amino 2-phenylacetate is prepared from phenylacetyl chloride and *N-tert*-butoxycarbonyl hydroxylamine according to General Method 1. (8.8g, 100%), ¹H NMR (500 MHz, DMSO-d6) δ ppm 10.66 (1H, br. s.), 7.24 - 7.38 (5H, m), 3.80 (2H, s), 1.38 (9H, s).

*N*-[(*tert*-Butoxy)carbonyl](2-bromobenzene)sulfonamido 2-phenylacetate (59) is prepared from 2-bromobenzene sulfonyl chloride, sodium hydride and [(*tert-*butoxy)carbonyl]amino 2-phenylacetate according to General Method 4. ¹H NMR (500 MHz, DMSO-d6) δ ppm 8.16 - 8.23 (1H, m), 7.95 - 8.00 (1H, m), 7.66 - 7.75 (2H, m), 7.26 - 7.41 (5H, m), 4.04 (2H, s), 1.24 (9H, s).

(2-Bromobenzene)sulfonamido 2-phenylacetate (59) is prepared from *N*-[(*tert-*butoxy)carbonyl](2-bromobenzene)sulfonamido 2-phenylacetate according to General Method 6. (1.6g, 54% over two steps), ¹H NMR (500 MHz, DMSO-d6) δ ppm 11.52 (1H, br. s.), 7.89 - 7.93 (2H, m), 7.57 - 7.64 (2H, m), 7.25 - 7.33 (3H, m), 7.15 - 7.19 (2H, m), 3.75 (2H, s).

### Preparation of (2-bromobenzene)sulfonamido 2-phenylbutanoate (60)

[(*tert*-Butoxy)carbonyl]amino 2-phenylbutanoate is prepared from 2-phenylbutanoyl chloride and *N-tert*-butoxycarbonyl hydroxylamine according to General Method 1. (4.27g, 42%), ¹H NMR (250 MHz, DMSO-*d*₆) δ ppm 7.20 - 7.45 (5H, m), 3.68 (2H, t, 7.6Hz), 1.73 (1H, dt, 13.8, 7.0Hz), 0.87 (3H, t, 7.3Hz).

*N*-[(*tert*-Butoxy)carbonyl](2-bromobenzene)sulfonamido 2-phenylbutanoate is prepared from 2-bromobenzene sulfonyl chloride, sodium hydride and [(*tert*-butoxy)carbonyl]amino 2-phenylbutanoate according to General Method 4. The compound is used directly for the synthesis of (2-bromobenzene)sulfonamido 2-phenylbutanoate without full characterisation.

(2-Bromobenzene)sulfonamido 2-phenylbutanoate (60) is prepared from *N*-[(*tert-*butoxy)carbonyl](2-bromobenzene)sulfonamido 2-phenylbutanoate according to General Method 6. (0.77g, 49%), ¹H NMR (500 MHz, DMSO-d6) δ ppm 11.46 (1H, s), 7.88 (1H, d, 7.9Hz), 7.73 (1H, d, 7.6Hz), 7.56 - 7.63 (1H, m), 7.47 - 7.55 (1H, m), 7.24 - 7.38 (3H, m), 7.12 - 7.22 (2H, m), 3.60 (1H, t, 7.6Hz), 1.80 - 1.98 (1H, m), 0.74 (3H, t, 7.3Hz).

### Preparation of (2-methanesulfonylbenzene)sulfonamido 2-phenylbutanoate (61)

*N*-[(*tert*-Butoxy)carbonyl](2-methanesulfonylbenzene)sulfonamido 2-phenylbutanoate is prepared from 2-methanesulfonylbenzene sulfonyl chloride, sodium hydride and [(*tert-*butoxy)carbonyl]amino 2-phenylbutanoate according to General Method 4. The compound is used directly for the synthesis of (2-methanesulfonylbenzene)sulfonamido 2-phenylbutanoate without full characterisation.

(2-Methanesulfonylbenzene)sulfonamido 2-phenylbutanoate (61) is prepared from N-[(*tert*-butoxy)carbonyl](2-methanesulfonylbenzene)sulfonamido 2-phenylbutanoate according to General Method 6. (0.57g, 36%), ¹H NMR (250 MHz, DMSO-d6) δ ppm 10.62 (1H, s), 8.23 (1H, dd, 7.8, 1.0Hz), 8.01 (1H, td, 7.5, 1.8Hz), 7.72 - 7.89 (2H, m), 7.24 - 7.38 (3H, m), 7.03 - 7.19 (2H, m), 3.60 (1H, t, 7.6Hz), 3.41 (3H, s), 1.75 - 1.99 (1H, m), 0.70 (3H, t, 7.3Hz).

### Preparation of (2-methanesulfonylbenzene)sulfonamido (2S)-2-(1,3-dioxo-2,3-dihydro-1H-isoindol-2-yl)-3-methylbutanoate (62)

(2S)-2-(1,3-Dioxo-2,3-dihydro-1H-isoindol-2-yl)-3-methylbutanoic acid is synthesised according to the method detailed in Tetrahedron 2005, 61, 38, 9031 - 9041. (16.6g, 78%), ¹H NMR (250 MHz, DMSO-*d*₆) δ ppm 7.55 - 8.09 (4H, m), 4.45 (1H, d, 7.9Hz), 3.36 (1H, br. s.), 2.58 - 2.66 (1H, m), 1.06 (3H, d, 6.5Hz), 0.82 (3H, d, 6.9Hz).

[(*tert*-Butoxy)carbonyl]amino (2S)-2-(1,3-dioxo-2,3-dihydro-1H-isoindol-2-yl)-3-methylbutanoate is prepared from (2S)-2-(1,3-dioxo-2,3-dihydro-1H-isoindol-2-yl)-3-methylbutanoyl chloride (which is in turn synthesised from (2S)-2-(1,3-dioxo-2,3-dihydro-1H-isoindol-2-yl)-3-methylbutanoic acid by reaction with thionyl chloride using standard conditions) and *N-tert*-butoxycarbonyl hydroxylamine according to General Method 1. (4.8g, 82%), ¹H NMR (250 MHz, DMSO-d6) δ ppm 10.77 (1H, br. s.), 7.83 - 8.10 (4H, m), 4.62 (1H, d, 9.1Hz), 2.66 - 2.87 (1H, m), 1.10 (3H, d, 6.7Hz), 0.86 (3H, d, 6.9Hz).

*N*-[(*tert*-Butoxy)carbonyl](2-methanesulfonylbenzene)sulfonamido (2S)-2-(1,3-dioxo-2,3-dihydro-1H-isoindol-2-yl)-3-methylbutanoate is prepared from 2-methanesulfonylbenzene sulfonyl chloride, sodium hydride and [(*tert*-butoxy)carbonyl]amino(2S)-2-(1,3-dioxo-2,3-dihydro-1H-isoindol-2-yl)-3-methylbutanoate according to General Method 4. The compound is used directly for the synthesis of (2-methanesulfonylbenzene)sulfonamido (2S)-2-(1,3-dioxo-2,3-dihydro-1H-isoindol-2-yl)-3-methylbutanoate without full characterisation. (1.44g, 90%).

(2-Methanesulfonylbenzene)sulfonamido (2S)-2-(1,3-dioxo-2,3-dihydro-1H-isoindol-2-yl)-3-methylbutanoate (62) is prepared from *N*-[(*tert*-butoxy)carbonyl](2-methanesulfonylbenzene) sulfonamido(2S)-2-(1,3-dioxo-2,3-dihydro-1H-isoindol-2-yl)-3-methyl butanoate according to General Method 6. (0.49g, 42%), ¹H NMR (250 MHz, DMSO-d6) δ ppm 10.75 (1H, br. s.), 8.08 - 8.19 (1H, m), 7.78 - 7.95 (6H, m), 7.59 - 7.71 (1H, m), 4.62 (1H, d, 8.7Hz), 3.39 (3H, s), 2.54 - 2.63 (1H, m), 0.97 (3H, d, 6.7Hz), 0.74 (3H, d, 6.9Hz).

### Preparation of (2-bromobenzene)sulfonamido (63)

### (2S)-2-(1,3-dioxo-2,3-dihydro-1H-isoindol-2-yl)-3-methylbutanoate

*N*-[(*tert*-Butoxy)carbonyl](2-bromobenzene)sulfonamido (2S)-2-(1,3-dioxo-2,3dihydro-1H-isoindol-2-yl)-3-methylbutanoate is prepared from 2-bromobenzene sulfonyl chloride, sodium hydride and [(*tert*-butoxy)carbonyl]amino(2S)-2-(1,3-dioxo-2,3-dihydro-1H-isoindol-2-yl)-3-methylbutanoate according to General Method 4. The compound is used directly for the synthesis of (2-bromobenzene)sulfonamido (2S)-2-(1,3-dioxo-2,3-dihydro-1H-isoindol-2-yl)-3-methylbutanoate without full characterisation (1.34g, 73%).

(2-Bromobenzene)sulfonamido (2S)-2-(1,3-dioxo-2,3-dihydro-1H-isoindol-2-yl)-3-methylbutanoate (63) is prepared from *N*-[(*tert*-butoxy)carbonyl](2-bromobenzene) sulfonamido(2S)-2-(1,3-dioxo-2,3-dihydro-1H-isoindol-2-yl)-3-methyl butanoate according to General Method 6. (0.47g, 40%), ¹H NMR (250 MHz, DMSO-d6) δ ppm 11.61 (1H, br. s.), 7.91 (4H, s), 7.75 (1H, dd, 7.9, 1.1Hz), 7.68 (1H, dd, 7.9, 1.7Hz), 7.47 (1H, td, 7.7, 1.7Hz), 7.31 (1H, td, 7.6, 1.1Hz), 4.59 (1H, d, 8.8Hz), 2.54 - 2.64 (1H, m), 0.98 (3H, d, 6.7Hz), 0.77 (3H, d, 6.9Hz).

### Preparation of ((2-bromobenzene)sulfonamido 2-methyl-2-phenyl propanoate (64)

### [(tert-Butoxy)carbonyl]amino 2-methyl-2-phenylpropanoate

A solution of α,α dimethyl phenylacetic acid (2g, 12.18mmol) in thionyl chloride (20ml) is heated to reflux for 1 hour after which time all of the starting acid has been consumed. The reaction mixture is concentrated *in vacuo* and the resulting acid chloride used directly for the synthesis of [(*tert*-butoxy)carbonyl]amino 2-methyl-2-phenylpropanoate,which is prepared from the described acid chloride and *N-tert*-butoxycarbonyl hydroxylamine according to General Method 1. (2.76g, 81%), ¹H NMR (500 MHz, CHLOROFORM-*d*) δ ppm 7.64 (1H, s), 7.29 (4H, dt, 15.6, 7.8Hz), 7.12 - 7.23 (1H, m), 1.60 (6H, s), 1.38 (9H, s).

*N*-[(*tert*-Butoxy)carbonyl](2-bromobenzene)sulfonamido 2-methyl-2phenylpropanoate is prepared from 2-bromobenzene sulfonyl chloride, sodium hydride and [(*tert-*butoxy)carbonyl]amino 2-methyl-2-phenylpropanoate according to General Method 4. (1.46g, 82%), ¹H NMR(500 MHz, CHLOROFORM-*d*) δ ppm 8.12 - 8.21 (1H, m), 7.65 - 7.78 (1H, m), 7.41 - 7.49 (4H, m), 7.37 (2H, t, 7.7Hz), 7.26 - 7.31 (1H, m), 1.75 (6H, s), 1.36 (9H, s).

(2-Bromobenzene)sulfonamido 2-methyl-2-phenylpropanoate (64) is prepared from N-[(*tert*-butoxy)carbonyl](2-bromobenzene)sulfonamido 2-methyl-2-phenylpropanoate according to General Method 6. (0.71g, 61%), ¹H NMR (500 MHz, DMSO-*d*₆) δ ppm 11.43 (1H, br. s.), 7.87 (1H, dd, 7.9, 1.0Hz), 7.75 (1H, dd, 7.8, 1.4Hz), 7.59 (1H, td, 7.7, 1.7Hz), 7.48 - 7.56 (1H, m), 7.30 - 7.36 (2H, m), 7.23 - 7.29 (1H, m), 7.20 (2H, d, 7.6Hz), 1.43 (6H, s).

### Preparation of (2-bromobenzene)sulfonamido 1-phenylcyclopentane-1-carboxylate

### [(tert-butoxy)carbonyl]amino 1-phenylcylopentane-1-carboxylate (65)

A solution of 1-phenyl cyclopentane carboxylic acid (2g, 10.5 mmol) in thionyl chloride (20ml) is heated to reflux for 1 hour after which time all of the starting acid has been consumed. The reaction mixture is concentrated *in vacuo* and the resulting acid chloride used directly for the synthesis [(*tert*-butoxy)carbonyl]-amino 1-phenylcyclopentane-1-carboxylate, which is prepared from the described acid chloride and *N-tert*-butoxycarbonyl hydroxylamine according to General Method 1. (2.4g, 75%), ¹H NMR (250 MHz, CHLOROFORM-*d*) δ ppm 7.62 (1H, s), 7.18 - 7.49 (5H, m), 2.63 - 2.86 (2H, m), 1.93 - 2.11 (2H, m), 1.71 - 1.90 (4H, m), 1.45 (9H, s).

*N*-[(*tert*-butoxy)carbonyl](2-bromobenzene)sulfonamido 1-phenylcyclopentane-1-carboxylate is synthesised from 2-bromobenzene sulfonyl chloride, sodium hydride and [(*tert-*butoxy)carbonyl]amino 1-phenylcyclopentane-1-carboxylate according to General Method 4. (1.41g, 82%), ¹H NMR (500 MHz, CHLOROFORM-*d*) δ ppm 8.09 - 8.18 (1H, m), 7.62 - 7.75 (1H, m), 7.38 - 7.49 (4H, m), 7.34 (2H, t, 7.6Hz), 7.23 - 7.30 (1H, m), 2.68 - 2.94 (2H, m), 1.76 - 2.17 (6H, m), 1.29 (9H, s).

(2-Bromobenzene)sulfonamido 1-phenylcyclopentane-1-carboxylate (65) is prepared from is prepared from *N*-[(*tert*-butoxy)carbonyl](2-bromobenzene)sulfonamido 1-phenylcyclopentane-1-carboxylate according to General Method 6. (0.87g, 79%), ¹H NMR (500 MHz, DMSO-*d*₆) δ ppm 11.40 (1H, s), 7.84 - 7.93 (1H, m), 7.73 (1H, dd, 7.8, 1.7Hz), 7.61 (1H, td, 7.7, 1.8Hz), 7.52 - 7.58 (1H, m), 7.29 - 7.34 (2H, m), 7.24 - 7.28 (1H, m), 7.18 - 7.23 (2H, m), 1.77 - 1.88 (2H, m), 1.59 - 1.71 (2H, m), 1.44 - 1.59 (2H, m).

### Preparation of (2-bromobenzene)sulfonamido 1-acetylpyrrolidine-2-carboxylate (66)

[(*tert*-Butoxy)carbonyl]amino-1-acetyl-L-pyrrolidine-2-carboxylate is synthesised using the method detailed in Tetrahedron 1994, 5049-5066. (1.05g, 36%), ¹H NMR (250 MHz, CHLOROFORM-*d*) δ ppm 8.15 (1H, br. s.), 4.49 - 4.73 (1H, m), 3.60 - 3.74 (1H, m), 3.45 - 3.59 (1H, m), 2.13 - 2.41 (2H, m), 2.10 (3H, s), 1.91 - 2.08 (2H, m), 1.41 - 1.51 (9H, m).

*N*-[(*tert*-butoxy)carbonyl](2-bromobenzene)sulfonamido 1-acetyl-L-pyrrolidine-2-carboxylate is synthesised from 2-bromobenzene sulfonyl chloride, sodium hydride and [(*tert-*butoxy)carbonyl]amino-1-acetyl-L-pyrrolidine-2-carboxylate according to General Method 4. (0.66g, 35%), ¹H NMR (500 MHz, CHLOROFORM-*d*) δ ppm 8.21 - 8.34 (1H, m), 7.75 - 7.83 (1H, m), 7.44 - 7.56 (2H, m), 4.51 - 4.65 (1H, m), 3.63 - 3.77 (1H, m), 3.45 - 3.62 (1H, m), 2.31 - 2.55 (2H, m), 2.15 - 2.31 (2H, m), 2.11 (3H, s), 1.49 (9H, s).

(2-Bromobenzene)sulfonamido 1-acetylpyrrolidine-2-carboxylate (66) is prepared from *N*-[(*tert*-butoxy)carbonyl](2-bromobenzene)sulfonamido-1-acetylpyrrolidine-2-carboxylate according to General Method 6. (0.08g, 15%), ¹H NMP (500 MHz, CHLOROFORM-*d*) δ ppm 9.56 (1H, br. s.), 8.11 - 8.22 (1H, m), 7.72 - 7.85 (1H, m), 7.46 - 7.61 (2H, m), 4.38 - 4.46 (1H, m), 3.55 (1H, ddd, 9.6, 7.9, 4.6Hz), 3.39 - 3.47 (1H, m), 2.03 - 2.13 (2H, m), 2.01 (3H, s), 1.81 - 1.99 (2H, m).

### Preparation of (2-bromobenzene)sulfonamido (2S)-2-phenyl propanoate (67)

[(*tert*-Butoxy)carbonyl]amino (2S)-2-phenylpropanoate is synthesised using the method detailed in Tetrahedron 1994, 5049-5066. (2.57g, 73%), ¹H NMR (500 MHz, CHLOROFORM- *d*) δ ppm 7.77 (1H, s), 7.33 - 7.37 (4H, m), 7.28 - 7.32 (1H, m), 3.90 (1H, q, 7.2Hz), 1.60 (3H, d, 7.3Hz), 1.45 (9H, s).

*N*-[(*tert*-Butoxy)carbonyl](2-bromobenzene)sulfonamido (2S)-2-phenylpropanoate is synthesised from 2-bromobenzene sulfonyl chloride, sodium hydride and [(*tert-*butoxy)carbonyl]amino (2S)-2-phenylpropanoate according to General Method 4. (0.36g, 20%), ¹H NMR (500 MHz, CHLOROFORM-*d*) δ ppm 8.10 (1H, d, 8.2Hz), 7.81 - 7.91 (1H, m), 7.79 (1H, d, 8.2Hz), 7.33 - 7.41 (5H, m), 6.95 - 7.05 (1H, m), 4.13 (1H, q, 7.1Hz), 1.67 (3H, d, 7.2Hz), 1.59 (9H, s).

(2-Bromobenzene)sulfonamido (2S)-2-phenylpropanoate (67) is prepared from N-[(*tert-*butoxy)carbonyl](2-bromobenzene)sulfonamido (2S)-2-phenylpropanoate according to General Method 6. (0.23g, 80%), ¹H NMR (500 MHz, CHLOROFORM-*d*) δ ppm 9.57 (1H, s), 7.83 (1H, dd, 7.9, 1.5Hz), 7.69 (1H, d, 7.8Hz), 7.38 (1H, td, 7.7, 1.4Hz), 7.17 - 7.26 (4H, m), 7.05 (2H, dd, 7.5, 1.7Hz), 3.66 (1H, q, 7.1Hz), 1.37 (3H, d, 7.2Hz).

### Preparation of (2-bromobenzene)sulfonamido (2R)-2-phenyl propanoate (68)

[(*tert*-Butoxy)carbonyl]amino (2*R*)-2-phenylpropanoate is synthesised using the method detailed in Tetrahedron 1994, 5049-5066. (0.92g, 69%), ¹H NMR (250 MHz, CHLOROFORM-*d*) δ ppm 7.78 (1H, s), 7.15 - 7.40 (5H, m), 3.90 (1H, q, 7.1Hz), 1.60 (3H, d, 7.2Hz), 1.45 (9H, s).

*N*-[(*tert*-Butoxy)carbonyl](2-bromobenzene)sulfonamido (2*R*)-2-phenylpropanoate is synthesised from 2-bromobenzene sulfonyl chloride, sodium hydride and [(*tert-*butoxy)carbonyl]amino (2*R*)-2-phenylpropanoate according to General Method 4. (0.86g, 51%), ¹H NMR (500 MHz, CHLOROFORM-*d*) δ ppm 8.02 - 8.36 (1H, m), 7.67 - 7.85 (1H, m), 7.28 - 7.53 (7H, m), 3.89 - 4.06 (1H, m), 1.66 (3H, d, 7.2Hz), 1.37 (9H, s).

(2-Bromobenzene)sulfonamido (2R)-2-phenylpropanoate (68) is prepared from N-[(*tert-*butoxy)carbonyl](2-bromobenzene)sulfonamido (2R)-2-phenylpropanoate according to General Method 6. (0.56g, 81%), ¹H NMR (500 MHz, CHLOROFORM-*d*) δ ppm 9.57 (1H, s), 7.83 (1H, d, 7.9Hz), 7.69 (1H, d, 7.9Hz), 7.38 (1H, t, 7.7Hz), 7.18 - 7.27 (4H, m), 7.05 (2H, dd, 7.0, 1.4Hz), 3.66 (1H, q, 7.2Hz), 1.37 (3H, d, 7.2Hz).

### Preparation of (3-methanesulfonylbenzene)sulfonamido 2,2-dimethylpropanoate (69)

N-[(*tert*-Butoxy)carbonyl](3-methanesulfonylbenzene) sulfonamido 2,2-dimethylpropanoate is synthesised from 2-methanesulfonylbenzene sulfonyl chloride, sodium hydride and [(*tert*-butoxy)carbonyl]amino 2,2-dimethylpropanoate according to General Method 4. (0.63g, 37%), ¹H NMR (500 MHz, CHLOROFORM-d)□δ ppm 8.60 (1H, s), 8.35 (1H, d, 7.9Hz), 8.26 (1H, d, 7.8Hz), 7.81 (1H, t, 7.9Hz), 1.43 (9H, s), 1.37 (9H, s).

(3-Methanesulfonylbenzene)sulfonamido 2,2-dimethylpropanoate (69) is prepared from *N*-[(*tert*-butoxy)carbonyl](3-methanesulfonylbenzene)sulfonamido-2,2-dimethyl propanoate according to General Method 6. (0.45g, 94%), ¹H NMR (500 MHz, CHLOROFORM-d) δ ppm 9.07 (1H, s), 8.53 (1H, t, 1.5Hz), 8.20 - 8.32 (2H, m), 7.84 (1H, t, 7.9Hz), 1.16 (9H, s).

### Preparation of (3-methanesulfonylbenzene)sulfonamido 2-methyl propanoate (70)

*N*-(*tert*-Butoxy)carbonyl](3-methanesulfonylbenzene) sulfonamido 2-methyl propanoate is synthesised from 2-methanesulfonylbenzene sulfonyl chloride, sodium hydride and [(*tert*-butoxy)carbonyl]amino 2-methylpropanoate according to General Method 4. (1.3g, 78%), ¹H NMR (500 MHz, CHLOROFORM-*d*) δ ppm 8.60 (1H, t, 1.7Hz), 8.35 (1H, d, 7.9Hz), 8.26 (1H, d, 7.8Hz), 7.82 (1H, t, 7.9Hz), 2.78 - 2.88 (1H, m), 1.43 (9H, s), 1.33 (6H, d, 7.0Hz).

(3-Methanesulfonylbenzene)sulfonamido 2-methylpropanoate (70) is prepared from *N-*[(*tert*-butoxy)carbonyl](3-methanesulfonylbenzene)sulfonamido-2-methyl propanoate according to General Method 6. (0.23g, 23%), ¹H NMR (500 MHz, CHLOROFORM-*d*) δ ppm 9.04 (1H, s), 8.53 (1H, s), 8.26 (2H, dd, 15.0, 7.9Hz), 7.84 (1H, t, 7.9Hz), 2.63 (1H, sept, 6.9Hz), 1.11 (6H, d, 7.0Hz).

### Preparation of (2-methanesulfonylbenzene) sulfonamido 2-(N-methylacetamido) acetate (71)

[(*tert*-Butoxy)carbonyl]amino 2-(*N*-methylacetamido)acetate is prepared from *N*-acetyl sarcosine and *N*-*tert*-butoxycarbonyl hydroxylamine according to General Method 2. (1.83g, 96%), ¹H NMR (500 MHz, CHLOROFORM-*d*) δ ppm 7.96 (1H, s), 4.30 (2H, s), 3.14 (3H, s), 2.16 (3H, s), 1.51 (9H, s).

*N*-[(*tert*-Butoxy)carbonyl](2-methanesulfonylbenzene)sulfonamido 2-(*N-*methylacetamido)acetate is synthesised from 2-methanesulfonylbenzene sulfonyl chloride and [(*tert*-butoxy)carbonyl]amino 2-(*N*-methylacetamido)acetate according to General Method 5. ¹H NMR (500 MHz, CHLOROFORM-*d*) δ ppm 8.30 - 8.42 (2H, m), 7.84 - 7.97 (2H, m), 4.12 (2H, s), 3.44 (3H, s), 3.12 (3H, s), 2.14 (3H, s), 1.45 (9H, s)

(2-Methanesulfonylbenzene) sulfonamido 2-(*N*-methylacetamido) acetate (71) is prepared from *N*-[(*tert*-butoxy)carbonyl](2-methanesulfonylbenzene)sulfonamido 2-(*N-*methylacetamido)acetate according to General Method 6. (0.07g, 9% over two steps), ¹H NMR (500 MHz, CHLOROFORM-d) δ ppm 9.99 (1H, s), 8.18 - 8.69 (2H, m), 7.60 - 8.11 (2H, m), 4.12 (2H, s), 3.44 (3H, s), 3.02 (3H, s), 2.07 (3H, s)

### Preparation of (2-methanesulfonylbenzene) sulfonamido (2S)-4-methyl-2-(methylamino)pentanoate (72)

[(*tert*-Butoxy)carbonyl]amino (2S)-2-{[(*tert*-butoxy)carbonyl](methyl)amino}-4-methylpentanoate is prepared from (2S)-2-{[(*tert*-butoxy)carbonyl](methyl)amino}-4-methylpentanoic acid and *N*-*tert*-butoxycarbonyl hydroxylamine according to General Method 2. The compound exists as rotomers and is reported as such. (1.8g, 58%), ¹H NMR(500 MHz, CHLOROFORM-*d*) δ ppm 7.49 - 8.06 (1H, m), 4.75 - 5.10 (1H, m), 2.86 (3 H, d, 7.8Hz), 1.66 - 1.88 (2H, m), 1.54 - 1.64 (1H, m), 1.50 (9H, s), 1.47 (9H, d, 3.7Hz), 0.96 (6H, dd, 10.7, 6.6Hz).

*N*-[(*tert*-Butoxy)carbonyl](2-methanesulfonylbenzene) sulfonamido (2S)-2-{[(*tert* butoxy) carbonyl](methyl)amino}-4-methylpentanoate is synthesised from 2-methanesulfonylbenzene sulfonyl chloride and [(*tert*-butoxy)carbonyl]amino (2S)-2-{[(*tert-*butoxy)carbonyl](methyl)amino}-4-methylpentanoate according to General Method 5. (0.51g, 63%), ¹H NMR (500 MHz, CHLOROFORM-*d*) δ ppm 8.34 - 8.48 (2H, m), 7.80 - 7.90 (2H, m), 4.94 - 5.31 (1H, m), 3.42 (3H, d, 9.3Hz), 2.77 - 2.92 (3H, m), 1.73 - 1.93 (2H, m), 1.58 - 1.68 (1H, m), 1.47 (9H, d, 6.4Hz), 1.41 (9H, s), 0.98 (6H, t, 7.4Hz).

(2-Methanesulfonylbenzene) sulfonamido (2S)-4-methyl-2-(methylamino)-pentanoate (72) is prepared from *N*-[(*tert*-butoxy)carbonyl](2-methanesulfonylbenzene)sulfonamido (2S)-2-{[(*tert* butoxy) carbonyl](methyl)amino}-4-methylpentanoate according to General Method 6. (0.03g, 9% as a TFA salt), ¹H NMR (500 MHz, MeOD) δ ppm 8.41 (1H, dd, 7.7, 1.1Hz), 8.32 (1H, dd, 7.6, 1.2Hz), 7.97 - 8.11 (2H, m), 4.10 (1H, dd, 8.8, 4.8Hz), 3.45 (3H, s), 2.69 (3H, s), 1.69 - 1.81 (1H, m), 1.58 - 1.69 (2H, m), 0.93 (3H, d, 6.1Hz), 0.88 (3H, d, 6.0Hz).

### Preparation of (2-methanesulfonylbenzene) sulfonamido (2R)-2-(methylamino) propanoate (73)

[(*tert*-Butoxy)carbonyl]amino (2R)-2-{[(*tert*-butoxy)carbonyl](methyl)amino} propanoate is prepared from (2R)-2-{[(*tert*-butoxy)carbonyl](methyl)amino} propanoic acid and *N*-*tert*-butoxycarbonyl hydroxylamine according to General Method 2. The compound exists as rotomers and is reported as such. (0.95g, 60%), ¹H NMR (500 MHz, CHLOROFORM-*d*) δ ppm 7.81 - 7.95 (1H, m), 4.60 - 5.04 (1H, m), 2.85 - 2.93 (3H, m), 1.40 - 1.54 (18H, m), 1.22 - 1.33 (3H, m).

N-[(tert-butoxy)carbonyl](2-methanesulfonylbenzene)sulfonamido (2R)-2-{[(*tert-*butoxy)carbonyl](methyl)amino}propanoate is synthesised from 2-methanesulfonyl benzene sulfonyl chloride and [(*tert*-butoxy)carbonyl]amino (2R)-2-{[(*tert*-butoxy) carbonyl](methyl)amino} propanoate according to General Method 5. (0.71g, 44%), ¹H NMR (500 MHz, CHLOROFORM-*d*) δ ppm 8.23 - 8.50 (2H, m), 7.78 - 7.99 (2H, m), 4.79 - 5.34 (1H, m), 3.39 - 3.51 (3H, m), 2.59 - 2.95 (3H, m), 1.47 (9H, s), 1.41 (9H, s), 1.27 (3H, t, 7.2Hz).

(2-Methanesulfonylbenzene) sulfonamido (2R)-2-(methylamino) propanoate (73) is prepared from *N*-[(*tert*-butoxy)carbonyl](2-methanesulfonylbenzene)-sulfonamido (2R)-2-{[(*tert*-butoxy)carbonyl] (methyl)amino}propanoate according to General Method 6. (0.09g, 79% as the TFA salt), ¹H NMR (500 MHz, MeOD) δ ppm 8.40 (1H, dd, 7.7, 1.3Hz), 8.33 (1H, dd, 7.6, 1.4Hz), 8.04 - 8.10 (1H, m), 7.99 - 8.04 (1H, m), 4.18 (1H, q, 7.2Hz), 3.45 (3H, s), 2.69 (3H, s), 1.49 (3H, d, 7.2Hz).

### Preparation of (2-methanesulfonylbenzene) sulfonamido (2S)-2-(methylamino) propanoate (74)

[(*tert*-Butoxy)carbonyl]amino (2S)-2-{[(*tert*-butoxy)carbonyl](methyl)amino}-propanoate is prepared from (2S)-2-{[(*tert*-butoxy)carbonyl](methyl)amino} propanoic acid and *N*-*tert*-butoxycarbonyl hydroxylamine according to General Method 2. The compound exists as rotomers and is reported as such. (0.96g, 61%), ¹H NMR (500 MHz, CHLOROFORM-*d*) δ ppm 7.73 - 7.89 (1H, m), 4.61 - 5.04 (1H, m), 2.86 - 2.96 (3H, m), 1.41 - 1.53 (21H, m).

*N*-[(*tert*-Butoxy)carbonyl](2-methanesulfonylbenzene)sulfonamido (2S)-2-{[(*tert-*butoxy)carbonyl](methyl)amino}propanoate is synthesised from 2-methanesulfonyl benzene sulfonyl chloride [(*tert*-butoxy)carbonyl]amino (2S)-2-{[(*tert-*butoxy)carbonyl](methyl)amino}propanoate according to General Method 5. (0.03g, 18%), ¹H NMR (500 MHz, CHLOROFORM-*d*) δ ppm 8.21 - 8.53 (2H, m), 7.73 - 8.02 (2H, m), 4.81 - 5.40 (1H, m), 3.36 - 3.51 (3H, m), 2.60 - 2.98 (3H, m), 1.47 (9H, s), 1.33 - 1.44 (12H, m).

(2-Methanesulfonylbenzene) sulfonamido (2S)-2-(methylamino) propanoate (74) is prepared from *N*-[(*tert*-butoxy)carbonyl](2-methanesulfonylbenzene)-sulfonamido (2S)-2-{[(*tert*-butoxy)carbonyl] (methyl)amino}propanoate according to General Method 6. (0.02g, 90% as a TFA salt), ¹H NMR (500 MHz, DMSO-d6) δ ppm 9.52 (1H, br. s.), 8.29 (1H, d, 7.5Hz), 8.23 (1H, d, 7.5Hz), 7.98 - 8.13 (2H, m), 4.24 (1H, q, 7.0Hz), 3.48 (3H, s), 2.53 (3H, s), 1.33 (3H, d, 7.2Hz).

### Preparation of (2-methanesulfonylbenzene)sulfonamido 2-(methylamino)acetate (75)

[(*tert*-Butoxy)carbonyl]amino 2-[(*tert*-butoxy)carbonyl](methyl)amino}-acetate is prepared from {[(*tert*-butoxy)carbonyl](methyl)amino}acetate and *N*-*tert*-butoxycarbonyl hydroxylamine according to General Method 2. The compound exists as rotomers and is reported as such. (1.5g, 93%), ¹H NMR (500 MHz, CHLOROFORM-*d*) δ ppm 7.92 (1H, d, m), 4.03 - 4.23 (2H, m), 2.95 - 3.01 (3H, m), 1.41 - 1.54 (18H, m)

*N-*[(*tert*-Butoxy)carbonyl](2-methanesulfonylbenzene)sulfonamido 2-{[(*tert-*butoxy)carbonyl](methyl)amino}acetate is synthesised from 2-methanesulfonyl benzene sulfonyl chloride and [(*tert*-butoxy)carbonyl]amino 2-{[(*tert-*butoxy)carbonyl](methyl)amino}acetate according to General Method 5. (1.3g, 51%), ¹H NMR (500 MHz, CHLOROFORM-*d*) δ ppm 8.48 (1H, dd, 7.5, 1.5Hz), 8.35 - 8.44 (1H, m), 7.81 - 7.93 (2H, m), 3.90 - 4.63 (2H, m), 3.42 (3H, d, 2.0Hz), 2.97 (3H, d, 14.2 Hz), 1.41 - 1.50 (18H, m).

(2-Methanesulfonylbenzene)sulfonamido 2-(methylamino)acetate (75) is prepared from *N*-[(*tert*-butoxy)carbonyl](2-methanesulfonylbenzene)sulfonamido 2-{[(*tert-*butoxy)carbonyl](methyl)amino}acetate according to General Method 6. (0.07g, 95% as the TFA salt), ¹H NMR (500 MHz, MeOD) δ ppm 8.40 (1H, dd, 7.9, 1.1 Hz), 8.33 (1H, dd, 7.7, 1.3Hz), 8.06 (1H, td, 7.6, 1.3Hz), 8.00 (1H, td, 7.6, 1.3Hz), 4.14 (2H, s), 3.46 (3H, s), 2.74 (3H, s).

### (2-Methanesulfonylbenzene)sulfonamido (2S)-3-methyl-2-(methylamino)butanoate (76)

[(*tert*-Butoxy)carbonyl]amino (2S)-2-{[(*tert*-butoxy)carbonyl](methyl)amino}-3-methylbutanoate is prepared from (2S)-2-{[(*tert*-butoxy)carbonyl](methyl)amino}-3-methylbutanoate and *N*-*tert*-butoxycarbonyl hydroxylamine according to General Method 2. The compound exists as rotomers and is reported as such. (1.18g, 42%), ¹H NMR (500 MHz, CHLOROFORM-*d*)□δ ppm 7.67 - 7.85 (1H, m), 4.29 - 4.64 (1H, m), 3.50 (3H, d, 5.2Hz), 2.19 - 2.32 (1H, m), 1.49 (9H, s), 1.47 (9H, s), 1.05 (3H, dd, 12.7, 6.4Hz), 0.93 (3H, d, 6.6Hz).

*N*-[(*tert*-butoxy)carbonyl](2-methanesulfonylbenzene)sulfonamido (2S)-2-{[(*tert-*butoxy)carbonyl](methyl)amino}-3-methylbutanoate is synthesised from 2-methanesulfonyl benzene sulfonyl chloride [(*tert*-butoxy)carbonyl]amino (2S)-2-{[(*tert-*butoxy)carbonyl](methyl)amino}-3-methylbutanoate according to General Method 5. (0.68g, 68%), ¹H NMR (250 MHz, CHLOROFORM-*d*) δ ppm 8.31 - 8.50 (2H, m), 7.77 - 7.97 (2H, m), 4.25 - 5.02 (1H, m), 3.31 - 3.47 (3H, m), 2.79 - 2.93 (3H, m), 2.21 - 2.41 (1H, m), 1.36 - 1.52 (18H, m), 1.03 - 1.12 (3H, m), 0.91 - 1.00 (3H, m).

(2-Methanesulfonylbenzene)sulfonamido (2S)-3-methyl-2-(methylamino)-butanoate (76) is prepared from *N*-[(*tert*-butoxy)carbonyl](2methanesulfonyl-benzene)-sulfonamido (2S)-2-{[(*tert*butoxy)carbonyl](methyl)amino}-3-methylbutanoate according to General Method 6. (0.25g, 87%), ¹H NMR (500 MHz, CHLOROFORM-d) δ ppm 10.06 (1 H, s), 8.38 - 8.42 (1H, m), 8.31 (1H, d, 7.6Hz), 7.90 - 8.01 (2H, m), 3.71 (1H, d, 4.6Hz), 3.45 (3H, s), 2.69 (3H, s), 2.29 - 2.40 (1H, m), 1.03 (3H, d, 6.9Hz), 1.00 (3H, d, 6.8Hz).

### Preparation of methanesulfonamido 2,2-dimethylpropanoate (77) (Reference Example)

*N*-[(*tert*-Butoxy)carbonyl]methanesulfonamido 2,2-dimethylpropanoateTo a solution of [(*tert*-butoxy)carbonyl]amino 2,2-dimethylpropanoate (1.0g, 4.6 mmol) in DCM (20 ml) is added triethylamine (0.47g, 4.6 mmol) and methane sulfonyl chloride (0.53g, 4.6 mmol). The reaction mixture is stirred for 90 minutes before the reaction mixture is diluted with DCM (50 ml) and quenched with water (20 ml). The organics are washed with water (20 ml), dried over sodium sulphate, filtered and concentrated *in vacuo* to yield the crude compound as a yellow oil, which is purified by silica gel column chromatography eluting with DCM. (1.3g, 100%), ¹H NMR (500 MHz, CHLOROFORM-d) δ ppm 3.43 (3H, s), 1.55 (9H, s), 1.34 (9H, s).

Methanesulfonamido 2,2-dimethylpropanoate (77) is prepared from *N*-[(*tert-*butoxy)carbonyl]methanesulfonamido 2,2-dimethylpropanoate according to General Method 6. (0.47g, 72%), ¹H NMR (500 MHz, CHLOROFORM-*d*) δ ppm 8.70 (1 H, s), 3.09 (3 H, s), 1.34 (9 H, s).

### Preparation of [(4-chlorophenyl)methane]sulfonamido 2,2-dimethylpropanoate (78) (Reference Example)

*N*-[(*tert*-Butoxy)carbonyl][(4-chlorophenyl)methane]sulfonamido 2,2 dimethyl propanoate is prepared from (4-chlorophenyl)methanesulfonyl chloride and [(*tert-*butoxy)carbonyl]amino 2,2-dimethylpropanoate according to General Method 8. (0.4g, 32%), ¹H NMR (500 MHz, CHLOROFORM-*d*) δ ppm 7.33 - 7.43 (4H, m), 4.56 - 5.04 (2H, m), 1.55 (9H, s), 1.29 (9H, s).

[(4-Chlorophenyl)methane]sulfonamido 2,2-dimethylpropanoate (78) is prepared from *N*-[(*tert*-butoxy)carbonyl][(4-chlorophenyl)methane]sulfonamido 2,2 dimethyl propanoate according to General Method 6. (0.18g, 89%), ¹H NMR (500 MHz, CHLOROFORM-*d*) δ ppm 8.55 (1 H, s), 7.37 - 7.43 (4 H, m), 4.38 (2 H, s), 1.25 (9 H, s).

### Preparation of N-(acetyloxy)-3-bromothiophene-2-sulfonamide (79) (Reference Example)

*tert*-Butyl (acetyloxy)[(3-bromothiophe*N*-2-yl)sulfonyl]carbamate is prepared from 3-bromothiophene-2-sulfonyl chloride, sodium hydride and [(*tert*-butoxy)carbonyl] amino acetate according to General Method 4. (0.8g, 35%), ¹H NMR (250 MHz, CHLOROFORM-*d*) δ ppm 7.68 (1H, d, 5.3Hz), 7.15 (1H, d, 5.2Hz), 2.30 (3H, s), 1.48 (9H, s).

*N*-(Acetyloxy)-3-bromothiophene-2-sulfonamide (79) is prepared from *tert*-butyl (acetyloxy)[(3-bromothiophe*N*-2-yl)sulfonyl]carbamate according to General Method 6. (0.22g, 36%), ¹H NMR (250 MHz, DMSO-d6) δ ppm 10.68 (1H, s), 7.66 (1H, d, 5.3Hz), 7.23 (1H, d, 5.3Hz), 2.14 (3H, s).

### Preparation of 1-benzofuraN-2-sulfonamido 2,2-dimethylpropanoate (80) (Reference Example)

*N*-[(*tert*-Butoxy)carbonyl]1-benzofura*N*-2-sulfonamido 2,2-dimethylpropanoate is prepared from [(*tert*-butoxy)carbonyl]amino 2,2-dimethylpropanoate, sodium hydride and 1-benzofura*N*-2-sulfonyl chloride according to General Method 4. (3.1g, 75%), ¹H NMR (250 MHz, DMSO-d6) δ ppm 8.06 (1H, d, 0.8Hz), 7.92 (1H, d, 7.3Hz), 7.81 (1H, dd, 8.5, 0.8Hz), 7.64 (1H, ddd, 8.5, 7.2, 1.4Hz), 7.42 - 7.53 (1H, m), 1.37 (9H, s), 1.28 (9H, s).

1-Benzofura*N*-2-sulfonamido 2,2-dimethylpropanoate (80) is prepared from *N*-[(*tert-*butoxy)carbonyl]1-benzofura*N*-2-sulfonamido 2,2-dimethylpropanoate according to General Method 6. (2.17g, 63% over two steps), ¹H NMR (500 MHz, DMSO-d6) δ ppm 11.90 (1H, br. s.), 7.87 (1H, d, 7.8Hz), 7.86 (1H, d, 0.8Hz), 7.78 (1H, dd, 8.5, 0.7Hz), 7.60 (1H, td, 7.9, 1.2Hz), 7.41 - 7.48 (1H, m), 1.12 (9 H, s).

### Preparation of 1-benzofuraN-2-sulfonamido acetate (81) (Reference Example)

*N*-[(*tert*-Butoxy)carbonyl]1-benzofura*N-*2-sulfonamido acetate is prepared from [(*tert-*butoxy)carbonyl]amino acetate, sodium hydride and 1-benzofura*N*-2-sulfonyl chloride according to General Method 4. (3.1g, 75%), ¹H NMR (250 MHz, DMF) δ ppm 8.07 (1H, d, 0.9Hz), 7.91 (1H, d, 7.3Hz), 7.82 (1H, dd, 8.5, 0.8Hz), 7.64 (1H, td, 7.8, 1.4Hz), 7.41 - 7.52 (1H, m), 2.32 (3H, s), 1.37 (9H, s).

1-Benzofura*N*-2-sulfonamido acetate (81) is prepared from *N*-[(*tert*-butoxy)carbonyl]1-benzofura*N*-2-sulfonamido acetate according to General Method 6. (0.79g, 34%), ¹H NMR (500 MHz, DMSO-d6) δ ppm 11.95 (1H, br. s.), 7.83 - 7.91 (2H, m), 7.73 - 7.81 (1H, m), 7.60 (1H, td, 7.9, 1.2Hz), 7.39 - 7.49 (1H, m), 2.10 (3H, s).

### Preparation of 3-bromothiophene-2-sulfonamido 2,2 dimethyl propanoate (82) (Reference Example)

*N*-[(*tert*-Butoxy)carbonyl]3-bromothiophene-2-sulfonamido 2,2-dimethylpropanoate is synthesised from 3-bromothiophene-2-sulfonyl chloride (synthesised according to the method detailed in Bioorganic and Medicinal Chemistry Letters 1996, 6, 2651 - 2656), sodium hydride and [(*tert*-butoxy)carbonyl]amino 2,2-dimethylpropanoate according to General Method 4. (0.2g, 12%), ¹H NMR (500 MHz, DMSO-d6) δ ppm 8.25 (1H, d, 5.2Hz), 7.44 (1H, d, 5.2 Hz), 1.39 (9H, s), 1.29 (9H, s).

3-Bromothiophene-2-sulfonamido 2,2-dimethylpropanoate (82) is prepared from *N-*[(*tert*-butoxy)carbonyl]3-bromothiophene-2-sulfonamido 2,2-dimethylpropanoate according to General Method 6. (0.48g, 51%), ¹H NMR (500 MHz, DMSO-d6) δ ppm 11.57 (1H, br. s.), 8.14 (1H, d, 5.2Hz), 7.37 (1H, d, 5.4Hz), 1.15 (9H, s).

### Preparation of 3-chlorothiophene-2-sulfonamido 2,2 dimethyl propanoate (83) (Reference Example)

3-Chlorothiophene-2-sulfonyl chloride is synthesised according to the method detailed in Bioorganic and Medicinal Chemistry Letters 1996, 6, 2651 - 2656:
To a stirred solution of 3-chlorothiophene (10 g, 84 mmol) in dichloromethane (25 ml) cooled to 0°C is added chlorosulfonic acid (16 ml, 252 mmol) dropwise. After 2 hours at 0°C the reaction mixture is carefully poured onto ice and extracted into dichloromethane (2 x 250ml). The organics are combined and dried over sodium sulfate, filtered and concentrated *in vacuo* to afford the title compound as a mixture with the other isomer. Both isomers are separated and the title compound isolated by silica column chromatography eluting with hexane:ethyl acetate. (3.7g, 20%), ¹H NMR (500 MHz, CHLOROFORM-d) δ ppm 7.75 (1H, d, 5.3Hz), 7.15 (1H, d, 5.3Hz).

*N*-[(*tert*-Butoxy)carbonyl]3-chlorothiophene-2-sulfonamido 2,2-dimethylpropanoate is synthesised from 3-chlorothiophene-2-sulfonyl chloride, sodium hydride and [(*tert-*butoxy)carbonyl]amino 2,2-dimethylpropanoate according to General Method 4. (1.72g, 94%), ¹H NMR (500 MHz, DMSO-d6) δ ppm 8.29 (1H, d, 5.3Hz), 7.40 (1H, d, 5.2Hz), 1.39 (9H, s), 1.28 (9H, s).

3-Chlorothiophene-2-sulfonamido 2,2-dimethylpropanoate (83) is prepared from *N-*[(*tert*-butoxy)carbonyl]3-chlorothiophene-2-sulfonamido 2,2-dimethylpropanoate according to General Method 6. (0.74g, 65%), ¹H NMR (250 MHz, DMSO-d6) δ ppm 11.54 (1H, br. s.), 8.17 (1H, d, 5.3Hz), 7.34 (1H, d, 5.3Hz), 1.15 (9H, s).

### Preparation of 5-chlorothiophene-2-sulfonamido 2-methylpropanoate (84) (Reference Example)

*N*-[(*tert*-Butoxy)carbonyl]5-chlorothiophene-2-sulfonamido 2-methylpropanoate is synthesised from 5-chlorothiophene sulfonyl chloride, sodium hydride and [(*tert-*butoxy)carbonyl]amino 2-methylpropanoate according to General Method 4. (1.7g, 100%), ¹H NMR (500 MHz, CHLOROFORM-*d*) δ ppm 7.65 (1H, d, 4.1Hz), 6.99 (1H, d, 4.1Hz), 2.81 (1H, sept, 7.0Hz), 1.49 (9H, s), 1.31 (6H, d, 7.0Hz).

5-Chlorothiophene-2-sulfonamido 2-methylpropanoate (84) is prepared from *N-*[(*tert-*butoxy)carbonyl]-5-chlorothiophene-2-sulfonamido 2-methylpropanoate according to General Method 6. (0.58g, 60%), ¹H NMR (500 MHz, CHLOROFORM-d) δ ppm 9.03 (1H, s), 7.56 (1H, d, 4.1Hz), 7.01 (1H, d, 4.0Hz), 2.68 (1H, sept, 7.0Hz), 1.18 (6H, d, 7.0Hz).

### Preparation of 5-chlorothiophene-2-sulfonamido 2,2 dimethyl propanoate (85) (Reference Example)

*N*-[(*tert*-Butoxy)carbonyl]5-chlorothiophene-2-sulfonamido 2,2-dimethylpropanoate is synthesised from 5-chlorothiophene sulfonyl chloride, sodium hydride and [(*tert-*butoxy)carbonyl]amino 2,2-dimethylpropanoate according to General Method 4. (1.89g, 100%), ¹H NMR (500 MHz, CHLOROFORM-*d*) δ ppm 7.65 (1H, d, 4.1Hz), 6.99 (1H, d, 4.1Hz), 1.48 (9H, s), 1.35 (9H, s).

5-Chlorothiophene-2-sulfonamido 2,2-dimethylpropanoate (85) is prepared from *N-*[(*tert*-butoxy)carbonyl]5-chlorothiophene-2-sulfonamido 2,2-dimethylpropanoate according to General Method 6. (0.66g, 46%), ¹H NMR (250 MHz, CHLOROFORM-d) δ ppm 9.07 (1H, s), 7.55 (1H, d, 4.1Hz), 7.01 (1H, d, 4.1Hz), 1.22 (9H, s).

### Preparation of pyridine-3-sulfonamido 2,2-dimethylpropanoate (86) (Reference Example)

*N*-[(*tert*-Butoxy)carbonyl]pyridine-3-sulfonamido 2,2-dimethylpropanoate is synthesised from 3-pyridine sulfonyl chloride, sodium hydride and [(*tert-*butoxy)carbonyl]amino 2,2-dimethylpropanoate according to General Method 4. (0.99g, 58%), ¹H NMR (500 MHz, CHLOROFORM-*d*) δ ppm 9.16 - 9.26 (1H, m), 8.89 (1H, d, 4.4Hz), 8.36 (1H, d, 8.2Hz), 7.54 (1H, dd, 8.2, 4.9Hz), 1.42 (9H, s), 1.37 (9H, s).

Pyridine-3-sulfonamido 2,2-dimethylpropanoate (86) is prepared from *N*-[(*tert-*butoxy)carbonyl]pyridine-3-sulfonamido 2,2-dimethylpropanoate according to General Method 6. (0.91g, 89% as the TFA salt), ¹H NMR (500 MHz, DMSO-d6) δ ppm 11.41 (1H, s), 9.00 (1H, d, 2.4Hz), 8.94 (1H, dd, 4.8, 1.5Hz), 8.27 (1H, dt, 8.1, 2.0Hz), 7.76 (1H, dd, 8.1, 4.8Hz), 1.10 (9H, s).

### Preparation of pyridine-3-sulfonamido 2-methylpropanoate (87) (Reference Example)

*N*-[(*tert*-Butoxy)carbonyl]pyridine-3-sulfonamido 2-methylpropanoate is synthesised from 3-pyridine sulfonyl chloride, sodium hydride and [(*tert*-butoxy)carbonyl]amino 2-methylpropanoate according to General Method 4.(0.6g, 37%), ¹H NMR (500 MHz, CHLOROFORM-*d*)□δ ppm 9.21 (1H, d, 1.6Hz), 8.89 (1H, dd, 4.7, 1.3Hz), 8.34 (1H, dd, 7.4, 1.4Hz), 7.53 (1H, dd, 8.2, 4.9Hz), 2.84 (1H, sept, 7.0Hz), 1.42 (9H, s), 1.32 (6H, d, 6.9Hz).

Pyridine-3-sulfonamido 2-methylpropanoate (87) is prepared from *N*-[(*tert-*butoxy)carbonyl]pyridine-3-sulfonamido 2-methylpropanoate according to General Method 6. (0.62g, 99% as the TFA salt), ¹H NMR (500 MHz, DMSO-d6) δ ppm 11.42 (1H, s), 9.01 (1H, d, 2.2Hz), 8.94 (1H, dd, 4.9, 1.4Hz), 8.28 (1H, dt, 8.1, 2.0Hz), 7.74 (1H, dd, 8.0, 4.9Hz), 2.58 - 2.64 (1H, m), 1.05 (6H, d, 7.1Hz).

### EXAMPLE 3: Synthesis of Compounds 15 and 16 (Reference Examples)

*O*-(tetrahydro-2H-pyran-2-yl) hydroxylamine is prepared according to literature procedure (Martin, N.I. et al., Org. Lett. 2006, 8, 4035-4038), and subsequently coupled to an appropriate sulfonyl chloride. The resultant *O*-(tetrahydro-2H-pyran-2-yl)-sulfonamide is dissolved in dichloromethane and one equivalent of triethylamine is added. The reaction mixture is then stirred while 1.1 equivalent of an appropriate acid chloride is added. Upon completion of the reaction (as indicated by TLC), the solvent is removed under reduced pressure and the crude *N*-(tetrahydro-2H-pyran-2-yloxy)-*N*-acylsulfonamide product is purified by column chromatography. The purified *N*-(tetrahydro-2H-pyran-2-yloxy)-*N*-acyl-sulfonamide is then dissolved in methanol, 10 molar percent *p*-toluenesulfonic acid is added, and the solution is stirred until the reaction is complete (as indicated by TLC). The solvent is removed under reduced pressure and the resultant *N*-hydroxy-*N*-acylsulfonamide product is purified by column chromatography.
*N*-hydroxy-*N*-benzoyl-benzenesulfonamide (15) (Reference Example). ¹H NMR (400 MHz, δ) 7.48-7.70 (10 H, m); 13C NMR (100 MHz, δ) 128.24, 129.17, 129.24, 130.65, 131.45, 134.10, 134.32, 134.87, 172.34; IR (KBr, cm⁻¹) 1730,3250; FAB-MS 278.04810 (M+H) (278.04870 cal.).
*N*-hydroxy-*N*-trimethylacetyl-2,6-dichlorobenzenesulfonamide (16) (Reference Example). ¹H NMR (400 MHz, δ) 7.40 (1 H, m), 7.49 (2 H, m); IR (KBr, cm⁻¹) 1688.7, 3359.7

### EXAMPLE 4: Synthesis of Compound 17 (Reference Example)

### General Method 7

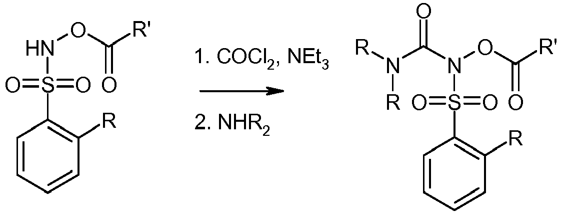

To a solution of the compound formed in General Method 6 in DCM cooled to 0°C is added triethylamine (2 equiv). The solution is stirred at this temperature for 5 minutes before phosgene (a 1.9M solution in toluene, 1.5 equiv) is added. The solution is stirred for a further 45 minutes before quenching with water. The organics are dried over sodium sulfate, filtered and concentrated *in vacuo.* The crude carbamoyl chloride is redissolved in DCM and triethylamine (1.1 equiv) and a secondary amine (1 equiv) is added. The reaction is stirred at room temperature for 1 h before quenching with water. The organics are dried over sodium sulfate, filtered and concentrated *in vacuo.* Purification is achieved by trituation with heptane: ethyl acetate.

N-[(2-bromophenyl)sulfonyl]-N-hydroxymorpholine-4-carboxamide is prepared from N-(acetyloxy)-2-bromobenzenesulfonamide and morpholine according to General Method 7. δH (250 MHz, CHLOROFORM-d) δ 8.02 - 8.20 (1H, m), 7.73 - 7.86 (1H, m), 7.37 - 7.55 (2H, m), 3.69 - 3.79 (4H, m), 3.26 - 3.36 (4H, m).

### EXAMPLE 5: Synthesis of Compounds 29-49

### General Method 8

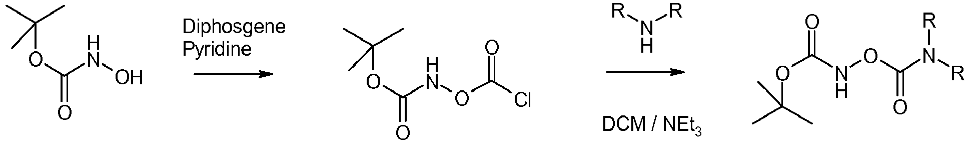

To a solution of *tert*-butyl *N*-hydroxycarbamate (1 equiv) in THF (20 vol) is added diphosgene (0.48 equiv) followed by pyridine (1 equiv) dropwise. The reaction mixture is stirred for 1 hour at room temperature, filtered and concentrated *in vacuo.* The residue is dissolved in DCM (10 vol) and added drop wise to a solution of amine (1 equiv), and triethylamine (1 equiv per basic centre in compound) in DCM (10 vol) at 0°C. The reaction mixture is stirred at room temperature until reaction is complete, before being washed with water and re-extracted with further aliquots of DCM. The crude product is dried over sodium sulfate, filtered and concentrated *in vacuo.* The title compound is purified directly by either silica column chromatography eluting with heptane: ethyl acetate or DCM: methanol followed by trituration where necessary.

### General Method 9

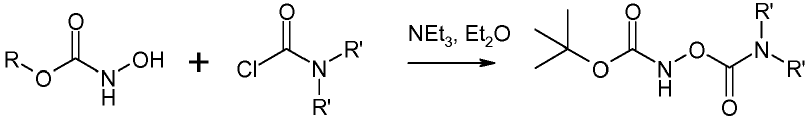

To a solution of *tert*-butyl *N*-hydroxycarbamate (1 equiv) and triethylamine (1.05 equiv) in diethyl ether (20 vol) or DCM (20 vol) cooled to 0°C is added a carbamoyl chloride (1 equiv). The reaction mixture is stirred at room temperature for 18 hours, filtered and concentrated *in vacuo.* The crude reaction mixture is diluted with DCM (20 vol) and washed with NaHCO₃ solution (2 x 10 vol) before being dried over sodium sulfate, filtered and concentrated *in vacuo.* The title compound is purified directly by either silica column chromatography eluting with heptane: ethyl acetate or reverse phase preparative HPLC.

### General Method 10

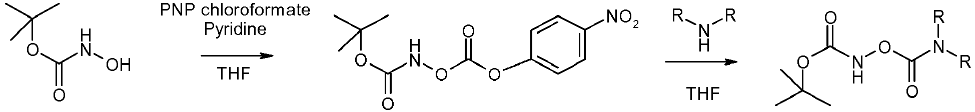

To a solution of *tert*-butyl *N*-hydroxycarbamate (1 equiv) in THF (4 vol) and pyridine (1 equiv) at 0°C is added *para* nitro chloroformate (1 equiv) in THF (2.5 vol) drop wise. The reaction mixture is stirred for 1 hour before being filtered and the amine (1 equiv) is added. The reaction mixture is stirred at room temperature for 18 hours and concentrated *in vacuo.* Compounds without basic centres are dissolved in DCM (10 vol) and washed with NaHCO₃ solution (2 x 5 vol) before being dried over sodium sulfate, filtered and concentrated *in vacuo.* The title compound is purified directly by either silica column chromatography eluting with heptane: ethyl acetate or DCM: methanol followed by trituration where necessary or reverse phase preparative HPLC.

### General Method 11

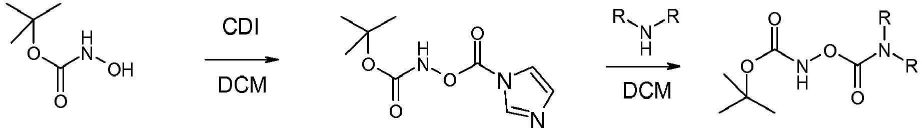

To a solution of *tert*-butyl *N*-hydroxycarbamate (0.9 equiv) in DCM (10 vol) is added carbonyldiimidazole (1 equiv). The reaction is stirred at room temperature for 1 hour when the amine (1 equiv) is added. The reaction mixture is stirred at room temperature for 18 hours and washed with water water (2 x) before being dried over sodium sulfate, filtered and concentrated *in vacuo.* The title compound is purified directly by either silica column chromatography eluting with heptane: ethyl acetate or DCM: methanol.

### General Method 12

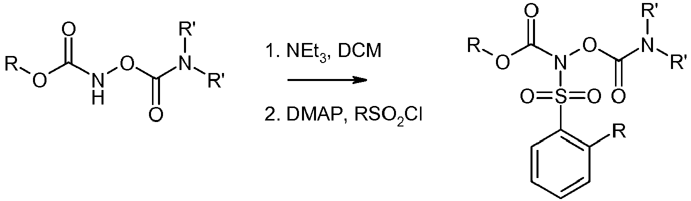

To a solution of *N,O*-disubstituted hydroxylamine (1 equiv) in DCM and triethylamine (1 equiv) is added dimethylaminopyridine (0.1 equiv) and a sulfonyl chloride (1 equiv). The reaction mixture is stirred at room temperature until complete consumption of the sulfonyl chloride is observed by tlc. The reaction mixture is concentrated *in vacuo* and purified directly by either silica column chromatography eluting with heptane: ethyl acetate or reverse phase preparative HPLC.

### General Method 13

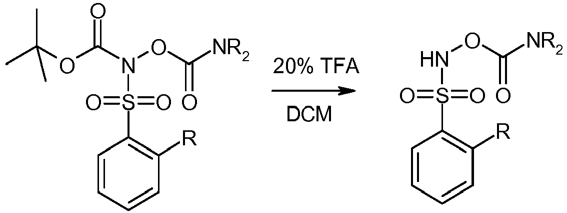

To a stirred solution of the compound formed in General Method 12 in dichloromethane is added trifluoroacetic acid. The reaction mixture is stirred at room temperature until complete consumption of the starting material is observed by LC-MS and concentrated *in vacuo* to yield the title compound as a clear, colourless gum. Purification is achieved by either silica column chromatography, reverse phase preparative HPLC or trituation from heptane:ethyl acetate or ether.

### Preparation of (2-methanesulfonylbenzene)sulfonamido N,N-dimethylcarbamate (29)

[(*tert*-Butoxy)carbonyl]amino *N,N-*dimethylcarbamate is prepared according to General Method 9. To a solution of *tert*-butyl *N*-hydroxycarbamate (2.0g, 15mmol) and triethylamine (2.2 ml, 15.7 mmol) in diethyl ether (25 ml) cooled to 0°C is added dimethyl carbamoyl chloride (1.6g, 15 mmol). The reaction mixture is stirred at room temperature for 18 hours and filtered to remove any triethylamine hydrochloride formed during the reaction and concentrated *in vacuo.* The crude reaction mixture is diluted with DCM (50 ml) and washed with NaHCO₃ solution (2 x 10 ml) before being dried over sodium sulfate, filtered and concentrated *in vacuo.* The title compound is used directly without any additional purification. (2.4g, 78% yield), ¹H NMR (500 MHz, CHLOROFORM-*d*) δ ppm 7.82 (1H, br. s.), 3.02 (3H, s), 2.98 (3H, s), 1.49 (9H, s).

*N*-[(*tert*-Butoxy)carbonyl](2-methanesulfonylbenzene)sulfonamido-*N,N*-dimethyl carbamate is prepared according to General Method 12. To a solution of [(*tert-*butoxy)carbonyl]amino *N,N-*dimethylcarbamate (0.5g, 2.4 mmol) in DCM (10 ml) and triethylamine (341 µl, 2.4 mmol) is added dimethylaminopyridine (0.03g, 0.24 mmol) and 2-methylsulfonylbenzenesulfonyl chloride (0.625g, 2.4 mmol). The reaction mixture is stirred at room temperature until complete consumption of the sulfonyl chloride is observed by tlc. The reaction mixture is concentrated *in vacuo* and purified directly by silica column chromatography eluting with heptane: ethyl acetate (1:1, v:v) to yield the title compound as a white solid. (0.45g, 42%), ¹H NMR (500 MHz, CHLOROFORM-*d*) δ ppm 8.54 - 9.01 (1H, m), 8.32 - 8.44 (1H, m), 7.76 - 7.90 (2H, m), 3.42 (3H, s), 3.09 (3H, s), 3.03 (3H, s), 1.41 (9H, s).

Alternatively, *N*-[(*tert*-butoxy)carbonyl](2-methanesulfonylbenzene)-sulfonamido-*N*,*N-*dimethyl carbamate is prepared *via* the following method. A solution of [(*tert-*butoxy)carbonyl]amino *N,N-*dimethylcarbamate (1g, 4.9 mmol) in THF (5 ml) is added dropwise to a stirred solution of sodium hydride (60% dispersion in oil, 0.235g, 5.2 mmol) in THF (25 ml). Stirring is continued for 30 minutes, whereupon 2-methylsulfonylbenzenesulfonyl chloride (1.35g, 5.4 mmol) is added. The reaction mixture is stirred at room temperature for 3 hours after which time tlc (1:1 heptane:ethyl acetate) showed no starting material remained. The reaction mixture is quenched by the addition of water and extracted into ether. The combined organics are dried over sodium sulfate, filtered and concentrated *in vacuo* to yield the desired material, which is purified by silica column chromatography eluting with heptane: ethyl acetate (1:1; v:v). (1.1g, 53%).

(2-Methanesulfonylbenzene)sulfonamido *N,N*-dimethylcarbamate (29) is prepared according to General Method 13. To a stirred solution of *N*-[(*tert*-butoxy)carbonyl](2-methanesulfonylbenzene) sulfonamido *N,N*-dimethyl carbamate (0.45g, 1.1 mmol) in dichloromethane (10ml) is added trifluoroacetic acid (2 ml). The reaction mixture is stirred at room temperature until complete consumption of the starting material is observed by LC-MS (c.a. 1h) and concentrated *in vacuo* to yield the title compound as a clear, colourless gum. Purification is achieved trituation from ether, yielding the title compound as a white solid. (0.25g, 77%), ¹H NMR (500 MHz, CHLOROFORM-d) δ ppm 9.92 (1H, s), 8.37 (1H, dd, 7.7, 1.1Hz), 8.28 (1H, dd, 7.7, 1.1Hz), 7.90 (1H, td, 7.7, 1.3Hz), 7.79 - 7.87 (1H, m), 3.42 (3H, s), 2.81 (3H, s), 2.80 (3H, s).

### Preparation of (2-bromobenzene)sulfonamido N,N-dimethylcarbamate (30)

*N*-[(*tert*-Butoxy)carbonyl](2-bromobenzene)sulfonamido *N,N*-dimethylcarbamate is prepared from [(*tert*-butoxy)carbonyl]amino *N,N-*dimethylcarbamate and 2-bromobenzenesulfonyl chloride according to General Method 12. (0.655g, 37%). ¹H NMR (250 MHz, CHLOROFORM-*d*) δ ppm 8.25 - 8.37 (1H, m), 7.71 - 7.82 (1H, m), 7.44 - 7.55 (2H, m), 3.02 (3H, s), 2.99 (3H, s), 1.50 (9H, s).

(2-Methanesulfonylbenzene)sulfonamido *N,N*-dimethylcarbamate (30) is prepared from *N*-[(*tert*-butoxy)carbonyl](2-bromobenzene)sulfonamido *N,N-*dimethylcarbamate according to General Method 13. (0.102g, 19%), δH (500 MHz, CHLOROFORM-d) 8.08 - 8.28 (1H, m), 7.75 - 7.87 (1H, m), 7.44 - 7.62 (2H, m), 2.84 (3H, s), 2.81 (3H, s).

### Preparation of (2-methanesulfonylbenzene)sulfonamido morpholine-4-carboxylate (31)

[(*tert*-Butoxy)carbonyl]amino morpholine-4-carboxylate is prepared from morpholine-4-carbonyl chloride and according to General Method 9. (1.71g, 105%), ¹H NMR (500 MHz, CHLOROFORM-d) δ ppm 7.78 (1H, s), 3.66 - 3.78 (4H, m), 3.46 - 3.65 (4H, m), 1.50 (9H, s).

*N*-[(*tert*-Butoxy)carbonyl](2-methanesulfonylbenzene)sulfonamido morpholine-4-carboxylate is prepared from [(*tert*-butoxy)carbonyl]amino morpholine-4-carboxylate and 2-methylsulfonylbenzenesulfonyl chloride according to General Method 12. (1.12g, 61%), ¹H NMR (500 MHz, CHLOROFORM-d) δ ppm 8.54 - 8.66 (1H, m), 8.32 - 8.43 (1H, m), 7.74 - 7.91 (2H, m), 3.49 - 3.86 (8H, m), 3.41 (3H, s), 1.43 (9H, s).

(2-Methanesulfonylbenzene)sulfonamido morpholine-4-carboxylate (31) is prepared from *N*-[(*tert*-butoxy)carbonyl](2-methanesulfonylbenzene)sulfonamido morpholine-4-carboxylate according to General Method 13. (0.76g, 86%), ¹H NMR (500 MHz, CHLOROFORM-d) δ ppm 9.99 (1H, s), 8.38 (1H, dd, 7.8, 1.3Hz), 8.27 (1H, dd, 7.7, 1.4Hz), 7.92 (1H, td, 7.6, 1.4Hz), 7.83 - 7.88 (1H, m), 3.54 - 3.59 (4H, m), 3.41 - 3.45 (3H, m), 3.29 - 3.37 (4H, m).

### Preparation of (2-methanesulfonylbenzene)sulfonamido 4-acetyl piperazine-1-carboxylate (32)

[(*tert*-Butoxy)carbonyl]amino 4-acetylpiperazine-1-carboxylate is prepared according to General Method 8. To a solution of *tert*-butyl *N*-hydroxycarbamate (1.0g, 7.44 mmol) in THF (20 ml) is added diphosgene (0.44ml, 3.57 mmol) followed by pyridine (0.6ml, 7.44 mmol) drop wise. The reaction mixture is stirred for 1 hour at room temperature, filtered and concentrated. The residue is dissolved in DCM (10 ml) and added drop wise to a solution of 4-acetylpiperazine (0.95g, 7.44 mmol), in triethylamine (1.0ml, 7.44 mmol) and DCM (10 ml) at 0°C. The reaction mixture is stirred at room temperature for 18 hours. The reaction mixture is washed with water, dried over sodium sulfate, filtered and concentrated *in vacuo.* The title compound is purified directly by silica column chromatography eluting with ethyl acetate yielding the title compound as a white solid. (0.75g, 35%), ¹H NMR (500 MHz, CHLOROFORM-d) δ ppm 7.70 (1H, br. s.), 3.24 - 3.68 (8H, m), 2.06 (3H, s), 1.43 (9H, s).

*N*-[(*tert*-Butoxy)carbonyl](2-methanesulfonylbenzene)sulfonamido 4-acetylpiperazine-1-carboxylate is prepared from [(*tert*-butoxy)carbonyl]amino 4-acetylpiperazine-1-carboxylate and 2-methylsulfonylbenzenesulfonyl chloride according to General Method 12. (0.89g, 70%), ¹H NMR (500 MHz, CHLOROFORM-d) δ ppm 8.45 - 8.62 (1H, m), 8.23 - 8.37 (1H, m), 7.72 - 7.83 (2H, m), 3.35 - 3.75 (8H, m), 3.32 (3H, s), 2.04 - 2.10 (3H, m), 1.35 (9H, s)

(2-Methanesulfonylbenzene)sulfonamido 4-acetylpiperazine-1-carboxylate (32) is prepared from *N*-[(*tert*-butoxy)carbonyl](2-methanesulfonylbenzene)-sulfonamido 4-acetylpiperazine-1-carboxylate according to General Method 13. (0.67g, 93%), ¹H NMR (500 MHz, CHLOROFORM-d) δ ppm 9.82 - 10.14 (1H, m), 8.40 (1H, dd, 7.7, 1.3Hz), 8.28 (1H, d, 7.7Hz), 7.94 (1H, td, 7.6, 1.3Hz), 7.87 (1H, t, 7.6Hz), 3.52 - 3.60 (2H, m), 3.45 (3H, s), 3.38 - 3.44 (4H, m), 3.28 - 3.35 (2H, m), 2.13 (3H, s).

### Preparation of (2-methanesulfonylbenzene)sulfonamido N-cyclohexyl-N-methylcarbamate (33)

*tert*-Butyl *N*-{[cyclohexyl(methyl)carbamoyl]oxy}carbamate is prepared according to General Method 10. To a solution of *tert*-butyl *N*-hydroxycarbamate (2.0g, 15.0 mmol) in THF (8 ml) and pyridine (1.2ml, 15.0 mmol)) at 0°C is added *para* nitro chloroformate (3.0g, 15.0 mmol) in THF (7.5 ml) drop wise. The reaction mixture is stirred for 1 hour before being filtered and *N*-methylcyclohexanamine (1.96ml, 15.0 mmol) added. The reaction mixture is stirred at room temperature for 18 hours and concentrated *in vacuo.* Dissolved in DCM and washed with NaHCO₃ solution (2 x) before being dried over sodium sulfate, filtered and concentrated *in vacuo,* the title compound is purified directly by silica column chromatography eluting with DCM: methanol and taken to the next step with no further purification (0.81g).

*N*-[(*tert*-Butoxy)carbonyl](2-methanesulfonylbenzene)sulfonamido *N*-cyclohexyl-*N-*methylcarbamate is prepared from 2-methylsulfonylbenzenesulfonyl chloride and *tert*-butyl *N-*{[cyclohexyl(methyl)carbamoyl]oxy}carbamate according to General Method 12. (0.44g, 6% over two steps). ¹H NMR (500 MHz, CHLOROFORM-d) δ ppm 8.52 - 8.65 (1H, m), 8.23 - 8.36 (1H, m), 7.69 - 7.82 (2H, m), 3.79 - 4.00 (1H, m), 3.34 (3H, s), 2.86 (3H, s), 0.90 - 1.88 (19H, m).

(2-Methanesulfonylbenzene)sulfonamido *N*-cyclohexyl-*N*-methylcarbamate (33) is prepared from *N*-[(*tert*-butoxy)carbonyl](2-methanesulfonylbenzene)-sulfonamido *N-*cyclohexyl-*N*-methylcarbamate according to General Method 13. (0.33g, 94%), ¹H NMR (500 MHz, CHLOROFORM-d) δ ppm 9.84 - 10.16 (1H, m), 8.38 (1H, dd, 7.8, 0.9Hz), 8.26 (1H, d, 7.6Hz), 7.89 (1H, t, 7.6Hz), 7.75 - 7.84 (1H, m), 3.53 - 3.79 (1H, m), 3.43 (3H, s), 2.67 (3H, br. s.), 1.58 - 1.95 (3H, m), 0.80 - 1.54 (7H, m).

### Preparation of (2-methanesulfonylbenzene)sulfonamido piperazine-1-carboxylate (34)

[(*tert*-Butoxy)carbonyl]amino piperazine-1-carboxylate is prepared from is prepared from *tert*-butyl *N*-hydroxycarbamate and *tert*-butyl piperazine-1-carboxylate according to General Method 10. (0.75g, 35%), ¹H NMR (250 MHz, CHLOROFORM-d) δ ppm 7.76 (1H, s), 3.43 - 3.62 (8H, m), 1.50 (9H, s), 1.48 (9H, s).

1-[(*tert*-Butoxy)carbonyl]amino 4-*tert*-butyl piperazine-1,4-dicarboxylate is prepared from 2-methylsulfonylbenzenesulfonyl chloride and [(*tert*-butoxy)carbonyl]amino piperazine-1-carboxylate according to General Method 12. (0.86g, 57%), ¹H NMR (500 MHz, CHLOROFORM-d) δ ppm 8.52 - 8.67 (1H, m), 8.31 - 8.49 (1H, m), 7.76 - 7.96 (2H, m), 3.45 - 3.78 (8H, m), 3.40 (3H, s), 1.48 (9H, s), 1.42 (9H, s).

(2-Methanesulfonylbenzene)sulfonamido piperazine-1-carboxylate (34) is prepared from *N*-[(*tert*-butoxy)carbonyl](2-methanesulfonylbenzene)sulfonamidopiperazine-1-carboxylate according to General Method 13. The title compound is collected as a TFA salt (0.69g, 94%). ¹H NMR (500 MHz, DMSO-d6) δ ppm 10.53 (1H, br. s.), 8.67 - 8.89 (2H, m), 8.29 (1H, dd, 7.9, 1.3Hz), 8.23 (1H, dd, 7.8, 1.2Hz), 8.09 (1H, td, 7.7, 1.3Hz), 7.98 - 8.06 (1H, m), 3.48 (3H, s), 3.36 - 3.47 (4H, m), 2.99 - 3.10 (4H, m).

### Preparation of (2-methanesulfonylbenzene)sulfonamido N-(2-methoxyethyl)-N-methylcarbamate (35)

[(*tert*-Butoxy)carbonyl]amino *N*-(2-methoxyethyl)carbamate is prepared from *tert*-butyl *N*-hydroxycarbamate and (2-methoxyethyl)(methyl)amine according to General Method 8. (0.58g, 32%), ¹H NMR (500 MHz, CHLOROFORM-d) δ ppm 7.79 (1H, s), 3.46 - 3.64 (4H, m), 3.36 (3H, d, 4.4Hz), 3.06 (3H, d, 10.6Hz), 1.50 (9H, s).

*N*-[(*tert*-Butoxy)carbonyl](2-methanesulfonylbenzene)sulfonamido *N*-(2-methoxyethyl) carbamate is prepared from 2-methylsulfonylbenzenesulfonyl chloride and [(*tert-*butoxy)carbonyl]amino *N*-(2-methoxyethyl)carbamate according to General Method 12. (0.58g, 53%), ¹H NMR (500 MHz, CHLOROFORM-d) δ ppm 8.63 - 8.73 (1H, m), 8.29 - 8.42 (1H, m), 7.86 (2H, dd, 5.8, 3.2Hz), 3.50 - 3.75 (4H, m), 3.43 (3H, d, 4.4Hz), 3.38 (3H, d, 4.6Hz), 3.14 (3H, d, 14.0Hz), 1.42 (9H, s).

(2-Methanesulfonylbenzene)sulfonamido *N*-(2-methoxyethyl)-*N*-methylcarbamate (35) is prepared from *N*-[(*tert*-butoxy)carbonyl](2-methanesulfonylbenzene)sulfonamido *N*-(2-methoxyethyl) carbamate according to General Method 13. (0.40g, 89%), ¹H NMR (500 MHz, CHLOROFORM-d) δ ppm 9.95 - 10.05 (1H, m), 8.37 (1H, dd, 7.7, 1.3Hz), 8.23 - 8.30 (1H, m), 7.90 (1H, td, 7.7, 1.3Hz), 7.78 - 7.86 (1H, m), 3.39 - 3.45 (4H, m), 3.21 - 3.35 (6H, m), 2.77 - 2.91 (3H, m).

### Preparation of (2-methanesulfonylbenzene)sulfonamido 4-(pyridiN-4-yl)piperazine-1-carboxylate (36)

[(*tert*-Butoxy)carbonyl]amino 4-(pyridi*N*-4-yl)piperazine-1-carboxylate is prepared from *tert*-butyl *N*-hydroxycarbamate and 1-(pyridi*N*-4-yl)piperazine according to General Method 10. (1.06g, 43%), ¹H NMR (500 MHz, CHLOROFORM-d) δ ppm 8.31 (2H, d, 6.6Hz), 7.85 (1H, br. s.), 6.71 (2H, d, 6.6Hz), 3.66 - 3.84 (4H, m), 3.34 - 3.49 (4H, m), 1.51 (9H, s).

*N*-[(*tert*-Butoxy)carbonyl](2-methanesulfonylbenzene)sulfonamido 4-(pyridi*N*-4-yl) piperazine-1-carboxylate is prepared from 2-methylsulfonylbenzene-sulfonyl chloride and [(*tert*-butoxy)carbonyl]amino 4-(pyridi*N*-4-yl)piperazine-1-carboxylate according to General Method 12 and carried to the next step.

(2-Methanesulfonylbenzene)sulfonamido 4-(pyridi*N*-4-yl)piperazine-1-carboxylate (36) is prepared from *N*-[(*tert*-butoxy)carbonyl](2-methanesulfonyl-benzene)sulfonamido 4-(pyridi*N*-4-yl) piperazine-1-carboxylate according to General Method 13 and is collected as a TFA salt. (0.27g, 16% over two steps), ¹H NMR (500 MHz, DMSO-d6) δ ppm 13.62 (1H, br. s.), 10.52 (1H, br. s.), 8.26 - 8.34 (3H, m), 8.20 - 8.26 (1H, m), 8.07 (1H, td, 7.6, 1.4Hz), 7.98 - 8.05 (1H, m), 7.14 (2H, d, 7.6Hz), 3.67 (4H, br. s.), 3.47 (3H, s), 3.42 (4H, br. s).

### Preparation of (2-methanesulfonylbenzene)sulfonamido 4-(morpholiN-4-yl)piperidine-1-carboxylate (37)

[(*tert*-Butoxy)carbonyl]amino 4-(morpholi*N*-4-yl)piperidine-1-carboxylate is prepared from is prepared from *tert*-butyl *N*-hydroxycarbamate and 4-(piperidi*N*-4-yl)morpholine according to General Method 8. (0.29g, 12%), ¹H NMR (500 MHz, CHLOROFORM-d) δ ppm 7.77 (1H, s), 4.13 - 4.34 (2H, m), 3.66 - 3.90 (4H, m), 2.81 - 3.04 (2H, m), 2.49 - 2.70 (3H, m), 2.28 - 2.49 (1H, m), 1.80 - 1.99 (2H, m), 1.56 - 1.61 (3H, m), 1.48 - 1.54 (9H, m).

*N*-[(*tert*-Butoxy)carbonyl](2-methanesulfonylbenzene)sulfonamido 4-(morpholi*N*-4-yl)piperidine-1-carboxylate is prepared from 2-methylsulfonylbenzenesulfonyl chloride and [(*tert*-butoxy)carbonyl]amino 4-(morpholi*N*-4-yl)piperidine-1-carboxylate according to General Method 12 (0.48g) and taken to next step without further purification.

(2-Methanesulfonylbenzene)sulfonamido 4-(morpholi*N*-4-yl)piperidine-1-carboxylate (37) is prepared from *N*-[(*tert*-butoxy)carbonyl](2-methanesulfonylbenzene)sulfonamido 4-(morpholi*N*-4-yl)piperidine-1-carboxylate according to General Method 13 and is isolated as a TFA salt. (0.25g, 50% over two steps), ¹H NMR (500 MHz, DMSO-d6) δ ppm 10.45 (1H, br. s.), 9.70 (1H, br. s.), 8.29 (1H, dd, 7.7, 1.3Hz), 8.22 (1H, dd, 7.7, 1.3Hz), 8.09 (1H, td, 7.7, 1.3Hz), 8.00 - 8.06 (1H, m), 3.53 - 4.23 (6H, m), 3.48 (3H, s), 3.09 (2H, br. s.), 2.69 - 2.92 (2H, m), 2.01 (2H, d, 12.0Hz), 1.38 (2H, br. s.).

### Preparation of (2-methanesulfonylbenzene)sulfonamido N,N-diethylcarbamate (38)

[(*tert*-Butoxy)carbonyl]amino *N,N*-diethylcarbamate is prepared from *tert*-butyl *N-*hydroxycarbamate and diethyl carbamoyl chloride according to General Method 9. (0.67g, 39%), ¹H NMR (500 MHz, CHLOROFORM-*d*) δ ppm 7.84 (1H, br. s.), 3.33 (4H, q, 7.1Hz), 1.48 (9H, s), 1.11 - 1.28 (6H, m).

*N*-[(*tert*-Butoxy)carbonyl](2-methanesulfonylbenzene)sulfonamido *N,N* diethylcarbamate is prepared from [(*tert*-butoxy)carbonyl]amino *N,N*-diethylcarbamate and 2-methylsulfonylbenzenesulfonyl chloride according to General Method 12. (0.47g, 36%), ¹H NMR (500 MHz, CHLOROFORM-*d*) δ ppm 8.55 - 8.70 (1H, m), 8.29 - 8.36 (1H, m), 7.77 - 7.87 (2H, m), 3.18 - 3.52 (7H, m), 1.35 (9H, s), 1.22 (3H, t, 7.1Hz), 1.16 (3H, t, 7.0Hz).

(2-Methanesulfonylbenzene)sulfonamido *N,N*-diethylcarbamate (38) is prepared from *N*-[(*tert*-butoxy)carbonyl](2-methanesulfonylbenzene)sulfonamido N,N diethylcarbamate according to General Method 13. (0.2g, 55%), ¹H NMR (500 MHz, CHLOROFORM-d) δ ppm 10.08 (1H, s), 8.37 (1H, dd, 7.9, 1.3Hz), 8.25 (1H, dd, 7.7, 1.3Hz), 7.89 (1H, td, 7.6, 1.3Hz), 7.77 - 7.83 (1H, m), 3.43 (3H, s), 3.01 - 3.24 (4H, m), 0.79 - 1.15 (6H, m).

### Preparation of (2-methanesulfonylbenzene)sulfonamido 40(piperidiN-1-yl)piperidine-1-carboxylate (39)

[(*tert*-Butoxy)carbonyl]amino 4-(piperidi*N*-1-yl)piperidin-1-carboxylate is prepared from *tert*-butyl *N*-hydroxycarbamate and 4-(piperidi*N-*1-yl)piperidine according to General Method 10 and is carried to the next step without further purification (0.5g).

*N*-[(*tert*-Butoxy)carbonyl](2-methanesulfonylbenzene)sulfonamido 4-(piperidin*N*-1-yl)piperidine-1-carboxylate is prepared from 2-methylsulfonylbenzene-sulfonyl chloride and [(*tert*-butoxy)carbonyl]amino 4-(piperidi*N*-1-yl)piperidine-1-carboxylate_according to General Method 12 and is carried to the next step without further purification (0.63g).

(2-Methanesulfonylbenzene)sulfonamido4-(piperidin*N*-1-yl)piperidine-1-carboxylate (39) is prepared from *N*-[(*tert*-butoxy)carbonyl](2-methanesulfonylbenzene)sulfonamido 4-(piperidi*N*-1-yl)piperidine-1-carboxylate according to General Method 13 and is freebased using aqueous NaHCO₃. (0.21g, 3% over three steps), ¹H NMR (500 MHz, DEUTERIUM OXIDE) δ ppm 8.18 (1H, dd, 7.8, 1.2Hz), 8.12 (1H, dd, 7.8, 1.2Hz), 7.79 (1H, td, 7.7, 1.3Hz), 7.68 - 7.75 (1H, m), 3.88 - 4.05 (2H, m), 3.39 - 3.45 (3H, m), 2.80 - 3.38 (5H, m), 2.63 - 2.80 (2H, m), 1.21 - 2.01 (10H, m).

### Preparation of (2-methanesulfonylbenzene)sulfonamido N-methoxy-N-methylcarbamate (40)

[(*tert*-Butoxy)carbonyl]amino *N*-methoxy-*N-*methylcarbamate is prepared from *tert-*butyl *N*-hydroxycarbamate and *N*-Methoxy-*N*-methylcarbamoyl chloride according to General Method 9. (2.48g, 100%), ¹H NMR (500 MHz, CHLOROFORM-*d*) δ ppm 7.83 (1H, s), 3.76 (3H, s), 3.23 (3H, s), 1.48 (9H, s).

*N*-[(*tert*-Butoxy)carbonyl](2-methanesulfonylbenzene)sulfonamido *N*-methoxy-*N-*methylcarbamate is prepared from [(*tert*-butoxy)carbonyl]amino *N*-methoxy-*N-*methylcarbamate and 2-methylsulfonylbenzenesulfonyl chloride according to General Method 12. ¹H NMR (500 MHz, CHLOROFORM-*d*) δ ppm 8.55 - 8.63 (1H, m), 8.34 - 8.42 (1H, m), 7.81 - 7.88 (2H, m), 3.80 (3H, s), 3.42 (3H, s), 3.30 (3H, s), 1.42 (9H, s).

(2-Methanesulfonylbenzene)sulfonamido *N*-methoxy-*N*-methylcarbamate (40) is prepared from *N*-[(*tert*-butoxy)carbonyl](2-methanesulfonylbenzene)sulfonamido *N*-methoxy-*N*-methylcarbamate according to General Method 13. (0.85g, 50% from [(*tert-*butoxy)carbonyl]amino *N*-methoxy-*N*-methylcarbamate), ¹H NMR (500 MHz, CHLOROFORM-d) δ ppm 9.83 (1H, br. s.), 8.38 (1H, dd, 7.7, 1.1Hz), 8.31 (1H, dd, 7.7, 1.1Hz), 7.89 - 7.95 (1H, m), 7.82 - 7.89 (1H, m), 3.56 (3H, s), 3.44 (3H, s), 3.07 (3H, s).

### Preparation of (2-methanesulfonylbenzene)sulfonamido pyrrolidine-1-carboxylate (41)

[(*tert*-Butoxy)carbonyl]amino pyrrolidine-1-carboxylate is prepared from *tert*-butyl *N-*hydroxycarbamate and pyrrolidine-1-carbonyl chloride according to General Method 9. (0.59g, 23%), ¹H NMR (500 MHz, CHLOROFORM-*d*) δ ppm 7.79 (1H, s), 3.25 - 3.58 (4H, m), 1.80 - 2.10 (4H, m), 1.50 (9H, s).

*N*-[(*tert*-Butoxy)carbonyl](2-methanesulfonylbenzene)sulfonamido-pyrrolidine-1-carboxylate is prepared from [(*tert*-butoxy)carbonyl]amino pyrrolidine-1-carboxylate and 2-methylsulfonylbenzenesulfonyl chloride according to General Method 12 and is used directly for the synthesis of the title compound.

(2-Methanesulfonylbenzene)sulfonamido pyrrolidine-1-carboxylate is prepared from N-[(*tert*-butoxy)carbonyl](2-methanesulfonylbenzene)sulfonamido-pyrrolidine-1-carboxylate according to General Method 13. (0.43g, 49% over two steps), ¹H NMR (500 MHz, CHLOROFORM-d) δ ppm 9.84 (1H, s), 8.37 (1H, dd, 7.9, 1.3Hz), 8.28 (1H, dd, 7.7, 1.3Hz), 7.90 (1H, td, 7.7, 1.3Hz), 7.80 - 7.86 (1H, m), 3.43 (3H, s), 3.18 - 3.29 (4H, m), 1.75 - 1.88 (4H, m).

### Preparation of 2-[(carboxymethyl)({[(2-methanesulfonylbenzene) sulfonamidooxy]carbonyl})amino]acetic acid (42)

tert-Butyl 2-{[2-(*tert*-butoxy)-2-oxoethyl][({[(*tert*-butoxy)carbonyl]-amino}oxy)carbonyl]amino}acetate is prepared from *tert*-butyl *N*-hydroxycarbamate and *tert-*butyl 2-{[2-(*tert*-butoxy)-2-oxoethyl]amino}acetate according to General Method 11. To a solution of *tert*-butyl *N*-hydroxycarbamate (1.22g, 9.17 mmol) in DCM (30ml) is added carbonyldiimidazole (1.65g, 10.19 mmol). The reaction is stirred at room temperature for 1 hour when *tert*-butyl 2- {[2-(*tert*-butoxy)-2-oxoethyl]amino}acetate (2.50g, 10.19 mmol) is added. The reaction mixture is stirred at room temperature for 18 hours and washed with water water (2x20ml) before being dried over sodium sulfate, filtered and concentrated *in vacuo.* The title compound is purified directly by silica column chromatography eluting with heptane: ethyl acetate yielding the title compound as a white solid. (2.46g, 60%), ¹H NMR (500 MHz, CHLOROFORM-d) δ ppm 7.72 (1 H, s), 4.08 (2 H, s), 4.04 (2 H, s), 1.41 - 1.51 (27 H, m).

*tert*-Butyl 2-{[2-(*tert*-butoxy)-2-oxoethyl][({*N*-[(*tert*-butoxy)carbonyl](2-methanesulfonyl benzene)sulfonamido}oxy)carbonyl]amino}acetate is prepared from *tert*-butyl 2-{[2-(*tert*-butoxy)-2-oxoethyl][({[(*tert*-butoxy)carbonyl]amino}oxy)-carbonyl]amino}acetate and 2-methylsulfonylbenzenesulfonyl chloride according to General Method 12. (0.74g, 13%), ¹H NMR (500 MHz, CHLOROFORM-d) δ ppm 8.48 - 8.55 (1 H, m), 8.27 - 8.31 (1 H, m), 7.71 - 7.80 (2 H, m), 3.90 - 4.13 (4 H, m), 3.33 (3 H, s), 1.42 (9 H, s), 1.40 (9 H, s), 1.34 (9 H, s).

2-[(Carboxymethyl)({[(2-methanesulfonyl benzene)sulfonamidooxy]-carbonyl}) amino]acetic acid (42) is prepared from *tert*-butyl 2-{[2-(*tert*-butoxy)-2-oxoethyl][({*N-*[(*tert-*butoxy)carbonyl](2-methanesulfonylbenzene)sulfonamido}oxy)-carbonyl]amino}acetate according to General Method 13. (0.27g, 54%), ¹H NMR (500 MHz, DMSO-d6) δ ppm 12.76 (2H, br. s.), 10.55 (1H, s), 8.25 (1H, dd, 7.7, 1.3Hz), 8.10 - 8.15 (1H, m), 8.04 (1H, d, 1.3Hz), 7.96 (1H, d, 1.3Hz), 3.86 (4H, d, 2.7Hz), 3.45 (3H, s).

### Preparation of (2-methanesulfonylbenzene)sulfonamido 4-carbamoylpiperidine-1-carboxylate (43)

[(*tert*-Butoxy)carbonyl]amino 4-carbamoylpiperidine-1-carboxylate is prepared from *tert*-butyl *N*-hydroxycarbamate and piperidine-4-carboxamide according to General Method 8 and taken to the next step without further purification (0.67g).

*N*-[(*tert*-Butoxy)carbonyl](2-methanesulfonylbenzene)sulfonamido 4-carbamoyl piperidine-1-carboxylate is prepared from [(*tert*-butoxy)carbonyl]amino 4-carbamoylpiperidine-1-carboxylate and 2-methylsulfonylbenzenesulfonyl chloride according to General Method 12 and taken to the next step without further purification (0.66g).

(2-Methanesulfonylbenzene)sulfonamido 4-carbamoylpiperidine-1-carboxylate (43) is prepared from *N*-[(*tert*-butoxy)carbonyl](2-methanesulfonylbenzene)sulfonamido 4-carbamoylpiperidine-1-carboxylate according to General Method 13. (0.35g, 12% over three steps), ¹H NMR (500 MHz, DMSO-d6) δ ppm 10.38 (1H, s), 8.26 (1H, dd, 7.8, 1.2Hz), 8.19 (1H, dd, 7.7, 1.1Hz), 8.06 (1H, t, 7.6Hz), 8.01 (1H, t, 7.6Hz), 7.28 (1H, br. s.), 6.83 (1H, br. s.), 3.59 - 3.74 (2H, m), 3.46 (3H, s), 2.77 - 2.91 (1H, m), 2.66 - 2.77 (1H, m), 2.23 (1H, tt, 11.3, 3.7Hz), 1.54 - 1.71 (2H, m), 1.26 - 1.44 (1H, m), 1.09 - 1.26 (1H, m).

### Preparation of (2-methanesulfonylbenzene)sulfonamido N-methyl-N-(pyridiN-3-ylmethyl)carbamate (44)

*tert-Butyl N*-{[methyl(pyridi*N*3-ylmethyl)carbamoyl]oxy}carbamate is prepared from *tert*-butyl *N*-hydroxycarbamate and methyl(pyridi*N*-3-ylmethyl)amine according to General Method 8 and taken to the next step without further purification (0.52g).

*tert-Butyl N-*[(2-methanesulfonylbenzene)sulfonyl]-*N*-{[methyl(pyridi*N*-3-ylmethyl) carbamoyl]oxy}carbamate is prepared from *tert*-butyl *N*-{[methyl(pyridi*N*-3-ylmethyl)carbamoyl]oxy}carbamate and 2-methylsulfonylbenzenesulfonyl chloride according to General Method 12. (0.26g, 7% over two steps), ¹H NMR (500 MHz, CHLOROFORM-d) δ ppm 8.54 - 8.82 (3 H, m), 8.28 - 8.46 (1 H, m), 7.94 - 8.19 (1 H, m), 7.80 - 7.94 (2 H, m), 7.54 (1 H, br. s.), 4.53 - 4.84 (2 H, m), 3.42 (3 H, s), 2.93 - 3.18 (3 H, m), 1.44 (9 H, s).

(2-Methanesulfonylbenzene)sulfonamido *N*-methyl-*N*-(pyridi*N*-3-ylmethyl)carbamate (44) is prepared from *tert*-butyl *N*-[(2-methanesulfonylbenzene)-sulfonyl]-*N*-{[methyl (pyridi*N-*3-ylmethyl)carbamoyl]oxy} carbamate according to General Method 13. Compound is freebased using aqueous NaHCO₃. (0.13g, 64%), ¹H NMR (500 MHz, CHLOROFORM-d) δ ppm 10.04 (1H, br. s.), 8.48 - 8.70 (1H, m), 8.10 - 8.47 (2H, m), 7.11 - 8.08 (5H, m), 4.21 - 4.41 (2H, m), 3.34 - 3.49 (3H, m), 2.70 - 2.92 (3H, m).

### Preparation of 2-({[(2-methanesulfonylbenzene)sulfonamidooxy] carbonyl}(methyl)amino) acetic acid (45)

*tert*-Butyl 2-{[({[(*tert*-butoxy)carbonyl]amino}oxy)carbonyl](methyl)-amino}acetate is prepared from *tert*-butyl *N*-hydroxycarbamate and *tert*-butyl 2-(methylamino)acetate according to General Method 11. (1.31g, 39%), ¹H NMR (250 MHz, CHLOROFORM-d) δ ppm 1.48 (dd, 4.11, 1.37 Hz, 18 H) 3.05 (d, 9.90 Hz, 3 H) 3.96 (d, 10.20 Hz, 2 H) 7.62 - 7.87 (m, 1H).

*tert*-Butyl 2-{[({*N-*[(*tert*-butoxy)carbonyl](2-methanesulfonylbenzene)-sulfonamido}oxy)carbonyl](methyl)amino}acetate is prepared from *tert*-butyl 2-{[({[(*tert-*butoxy) carbonyl]amino}oxy)carbonyl](methyl)amino}acetate and 2-methylsulfonylbenzene sulfonyl chloride according to General Method 12 and taken to next step without furher purification (1.2g).

2-({[(2-Methanesulfonylbenzene)sulfonamidooxy]carbonyl}(methyl)amino) acetic acid (45) is prepared from *tert*-butyl 2-{[({*N*-[(*tert*-butoxy)carbonyl](2-methanesulfonyl benzene)sulfonamido}oxy)carbonyl](methyl)amino}acetate_according to General Method 13. (0.59g, 37% over two steps), ¹H NMR (500 MHz, MeOD) δ ppm 8.34 (2H, d, 7.6Hz), 7.98 (2H, d, 7.6Hz), 3.86 (2H, s), 3.42 (3H, s), 2.85 (3H, s).

### Preparation of (2-methanesulfonylbenzene)sulfonamido N-methyl-N-(1-methylpiperidiN-4-yl(carbamate (46)

*tert*-Butyl *N*-{[methyl(pyrid*N*-3-ylmethyl)carbamoyl]oxy}carbamate is prepared from *tert*-butyl *N*-hydroxycarbamate and N,1-dimethylpiperidi*N*-4-amine hydrochloride according to General Method 11. Triethylamine (1 equiv) is added prior to addition of amine. (2.74g, 61%), ¹H NMR (250 MHz, CHLOROFORM-d) δ ppm 7.86 (1H, br. s.), 3.97 (1H, br. s.), 2.83 - 2.99 (5H, m), 2.28 (3H, s), 2.05 (2H, td, 11.6, 3.0Hz), 1.61 - 1.93 (4H, m), 1.42 - 1.54 (9H, m).

*tert*-Butyl *N*-[(2-methanesulfonylbenzene)sulfonyl]-*N*-{[methyl(pyridi*N*-3-ylmethyl) carbamoyl]oxy}carbamate is prepared from *tert*-butyl *N*-{[methyl(pyridi*N*-3-ylmethyl)carbamoyl]oxy}carbamate and 2-methylsulfonylbenzenesulfonyl chloride according to General Method 12 and taken to the next step without further purification (1.2g).

(2-Methanesulfonylbenzene)sulfonamido *N*-methyl-*N*-(1-methylpiperidi*N*-4-yl)carbamate (46) is prepared from *tert*-butyl *N*-[(2-methanesulfonylbenzene)-sulfonyl]-*N-*{[methyl(pyridi*N*-3-ylmethyl)carbamoyl]oxy}carbamate according to General Method 13. Compound is freebased using aqueous NaHCO₃. (0.31g, 15% over two steps), ¹H NMR (500 MHz, CHLOROFORM-d) δ ppm 8.37 (1H, d, 7.3Hz), 8.25 (1H, d, 7.6Hz), 7.84 - 7.93 (1H, m), 7.81 (1H, dd, 7.6, 1.0Hz), 3.61 - 3.82 (1H, m), 3.43 (3H, s), 2.87 (2H, br. s.), 2.68 (3H, s), 2.27 (3H, s), 1.97 (2H, d, 11.3Hz), 1.35 - 1.77 (4H, m).

### Preparation of 2-[(carboxymethyl)({[(2-methanesulfonylbenzene) sulfonamidooxy]-carbonyl})amino]acetic acid (47)

*tert*-Butyl 2-{[2-(*tert*-butoxy)-2-oxoethyl][({(*tert*-butoxy)carbonyl]amino}-oxy)carbonyl]amino}acetate is prepared from *tert*-butyl *N*-hydroxycarbamate and di-*tert*-butyl 2,2'-iminodiacetate according to General Method 11. (2.46g, 59.7%), ¹H NMR (500 MHz, CHLOROFORM-*d*) δ ppm 7.72 (1H, s), 4.08 (2H, s), 4.04 (2H, s), 1.41 - 1.51 (27H, m).

*tert*-Butyl 2-{[2-(*tert*-butoxy)-2-oxoethyl][({*N*-[(*tert*-butoxy)carbonyl](2-methane sulfonylbenzene) sulfonamido}oxy)carbonyl]amino}acetate is prepared from *tert*-butyl 2-{[2-(*tert*-butoxy)-2-oxoethyl][({[(*tert*-butoxy)carbonyl]amino}oxy)carbonyl]amino}acetate and 2-methylsulfonylbenzenesulfonyl chloride according to General Method 12. (0.74g, 13.6%), ¹H NMR (500 MHz, CHLOROFORM-*d*) δ ppm 8.48 - 8.55 (1H, m), 8.27 - 8.31 (1H, m), 7.71 - 7.80 (2H, m), 3.90 - 4.13 (4H, m), 3.33 (3H, s), 1.42 (9H, s), 1.40 (9H, s), 1.34 (9H, s).

2-[(Carboxymethyl)({[(2-methanesulfonylbenzene)sulfonamidooxy]-carbonyl})amino] acetic acid (47) is prepared from *tert*-butyl 2-{[2-(*tert*-butoxy)-2-oxoethyl][({*N*-[(*tert-*butoxy)carbonyl](2-methane sulfonylbenzene)sulfonamido}-oxy)carbonyl]amino}acetate according to General Method 13. (0.27g, 54%), ¹H NMR (500 MHz, DMSO-d6) δ ppm 12.76 (2H, br. s.), 10.55 (1H, s), 8.25 (1H, dd, 7.7, 1.3Hz), 8.10 - 8.15 (1H, m), 8.04 (1H, d, 1.3Hz), 7.96 (1H, d, 1.3Hz), 3.86 (4H, d, 2.7Hz), 3.45 (3H, s).

### Preparation of (2-methanesulfonylbenzene)sulfonamido 2-oxoimidazolidine-1-carboxylate (48)

[(*tert*-Butoxy)carbonyl]amino 2-oxoimidazolidine-1-carboxylate is prepared from 2-oxoimidazolidine-1-carbonyl chloride and *tert*-butyl hydroxycarbamate according to General Method 9. (0.76g, 23.0%), ¹H NMR (500 MHz, CHLOROFORM-*d*) δ ppm 7.85 - 8.01 (1H, m), 5.77 - 5.95 (1H, m), 4.04 (2H, d, 8.4Hz), 3.54 (2H, t, 8.2Hz), 1.45 - 1.54 (9H, s).

*N*-[(*tert*-Butoxy)carbonyl](2-methanesulfonylbenzene) sulfonamido 2-oxoimidazolidine-1-carboxylate is prepared from [(*tert*-butoxy)carbonyl]amino 2-oxoimidazolidine-1-carboxylate and 2-methylsulfonyl benzenesulfonyl chloride according to General Method 12 and is used crude for synthesis of (2-methanesulfonylbenzene)sulfonamido 2-oxoimidazolidine-1-carboxylate (0.5g, 34.8%).

(2-Methanesulfonylbenzene)sulfonamido 2-oxoimidazolidine-1-carboxylate (48) is prepared from *N*-[(*tert*-butoxy)carbonyl](2-methanesulfonylbenzene) sulfonamido 2-oxoimidazolidine-1-carboxylate according to General Method 13. (0.13g, 33%), ¹H NMR (500 MHz, DMSO-d6) d ppm 10.60 - 10.77 (1H, m), 8.19 - 8.32 (2H, m), 7.93 - 8.07 (2H, m), 7.49 - 7.64 (1H, m), 3.61 - 3.70 (2H, m), 3.47 (3H, s), 3.22 - 3.30 (2H, m).

### Preparation of (2-methanesulfonylbenzene)sulfonamido 3-oxopiperazine-1-carboxylate (49)

[(*tert*-Butoxy)carbonyl]amino 3-oxopiperazine-1-carboxylate is prepared from *tert*-butyl hydroxycarbamate and piperazi*N*-2-one according to General Method 11. (0.3g, 12%), ¹H NMR (500 MHz, DMSO-*d*6) δ ppm 10.59 (1H, br. s.), 8.16 (1H, br. s.), 3.82 - 3.98 (2H, m), 3.47 - 3.64 (2H, m), 3.16 - 3.26 (2H, m), 1.40 (9H, s).

*N*-[(*tert*-Butoxy)carbonyl](2-methanesulfonylbenzene)sulfonamido 3-oxopiperazine-1-carboxylate is prepared from [(*tert*-butoxy)carbonyl]amino 3-oxopiperazine-1-carboxylate and 2-methylsulfonyl benzenesulfonyl chloride according to General Method 12. (0.29g, 52.1%), ¹H NMR (500 MHz, CHLOROFORM-*d*) δ ppm 8.47 - 8.69 (1H, m), 8.39 (1H, dd, 7.3, 1.8Hz), 7.76 - 7.97 (2H, m), 6.14 (1H, br. s.), 4.18 - 4.62 (2H, m), 3.43 - 4.05 (4H, m), 3.39 (3H, s), 1.43 (9H, s).

(2-Methanesulfonylbenzene)sulfonamido 3-oxopiperazine-1-carboxylate (49) is prepared from *N*-[(*tert*-butoxy)carbonyl](2-methanesulfonylbenzene) sulfonamido 3-oxopiperazine-1-carboxylate according to General Method 13. (0.22g, 95.8%), ¹H NMR (500 MHz, DMSO-d6) δ ppm 10.51 (1H, s), 8.29 (1H, dd, 7.8, 1.2Hz), 8.22 (1H, d, 7.7Hz), 8.14 (1H, br. s.), 8.08 (1H, td, 7.6, 1.1Hz), 7.99 - 8.05 (1H, m), 3.68 - 3.79 (2H, m), 3.47 (3H, s), 3.33 - 3.42 (2H, m), 3.00 - 3.20 (2H, m).

### EXAMPLE 6: HNO Production Via N₂O Quantification

Nitrous oxide is produced via the dimerization and dehydration of HNO, and is the most common marker for HNO production (Fukuto, J.M. et al., Chem. Res. Toxicol. 2005, 18, 790-801). HNO, however, can also be partially quenched by oxygen to yield a product that does not produce N₂O (*see* Mincione, F. et al., J. Enzyme Inhibition 1998, 13, 267-284; and Scozzafava, A. et al., J. Med. Chem. 2000, 43, 3677-3687.) Using Angeli's salt (AS) as a benchmark, the relative amounts of N₂O released from compounds are examined via gas chromatography (GC) headspace analysis.

The ability of compounds to donate HNO is assessed. Results are provided Table 3. N₂O results are reported relative to Angeli's salt. All decompositions are carried out at 37 °C under argon.

**Table 3**

| **Compound** | **%N₂O pH 7.4⁴** | **%N₂O esterase⁵** |
|---|---|---|
| 1 | | |
| 2 | <3 (5 hr) | 53 (1 hr) |
| 3 | <3 (5 hr) | 56 (1 hr) |
| 4 | | |
| 5 | | |
| 6 | | |
| 7 | | |
| 8 | | |
| 9 | | |
| 15* | 19 | |
| 16* | 41 | |

| | | |
|---|---|---|
| ⁴ Compound incubated in PBS buffer, pH 7.4. ⁵ Compound incubated in PBS buffer, pH 7.4 with 2-4 mg added esterase. The time at which each N₂O measurement was made is given in parentheses. * Reference Example | | |

### EXAMPLE 6A: HNO Production Via N₂O Quantification

Compounds are tested in the assay described in Example 6, with the following modification. Test compounds are assessed with and also without the addition of Pig Liver Esterase (PLE) at 37 °C for 90 minutes in PBS buffer at pH 7.4. Certain compounds described herein are tested and show detectable levels of HNO. Certain compounds described herein exhibit enhanced HNO production in the presence of PLE. Compound stability is also determined by assessing the half-life of the compounds in PBS at 37 °C at pH 7.4 with and without the addition of PLE according to methods known in the art, *e.g.,* in PCT publication No. PCT/US2007/0067 10.

### EXAMPLE 7: In Vitro Model to Determine Ability of Compounds or Pharmaceutical Compositions to Treat, Prevent and/or Delay Onset and/or Development of a Disease or Condition

### Cardiovascular Diseases or Conditions

*In vitro* models of cardiovascular disease can also be used to determine the ability of any of the compounds and pharmaceutical compositions described herein to treat, prevent and/or delay the onset and/or the development of a cardiovascular disease or condition in an individual. An exemplary *in vitro* model of heart disease is described below.

*In-vitro* models could be utilized to assess vasorelaxation properties of the compounds and pharmaceutical compositions. Isometric tension in isolated rat thoracic aortic ring segment can be measured as described previously by Crawford, J.H. et al., Blood 2006, 107, 566-575. Upon sacrifice, aortic ring segments are excised and cleansed of fat and adhering tissue. Vessels are then cut into individual ring segments (2-3 mm in width) and suspended from a force-displacement transducer in a tissue bath. Ring segments are bathed at 37 °C in a bicarbonate-buffered, Krebs-Henseleit (K-H) solution of the following composition (mM): NaCl 118; KCl 4.6; NaHCO3 27.2; KH2PO4 1.2; MgSO4 1.2; CaCl2 1.75; Na2EDTA 0.03; and glucose 11.1 and perfused continuously with 21% O2/5% CO2/74% N2. A passive load of 2 g is applied to all ring segments and maintained at this level throughout the experiments. At the beginning of each experiment, indomethacin-treated ring segments are depolarized with KCl (70 mM) to determine the maximal contractile capacity of the vessel. Rings are then washed extensively and allowed to equilibrate. For subsequent experiments, vessels are submaximally contracted (50% of KCl response) with phenylephrine (PE, 3 × 10⁻⁸ - 10⁻⁷ M), and L-NMMA, 0.1 mM, is also added to inhibit eNOS and endogenous NO production. After tension development reaches a plateau, compounds or pharmaceutical compositions are added cumulatively to the vessel bath and effects on tension monitored.

*In vitro* models can be utilized to determine the effects of the compounds and pharmaceutical compositions in changes in developed force and intracellular calcium in heart muscles. Developed force and intracellular calcium can be measured in rat trabeculae from normal or diseased (*i.e.* rats with congestive heart failure or hypertrophy) as described previously (Gao W.D. et al., Circ. Res. 1995, 76:1036-1048). Rats (Sprague-Dawley, 250-300 g) are used in these experiments. The rats are anesthetized with pentobarbital (100 mg/kg) via intra-abdominal injection, the heart exposed by mid-sternotomy, rapidly excised and placed in a dissection dish. The aorta is cannulated and the heart perfused retrograde (∼15 mM/min) with dissecting Krebs-Henseleit (H-K) solution equilibrated with 95% O2 and 5% CO2. The dissecting K-H solution is composed of (mM): NaCl 120, NaHCO3 20, KCl 5, MgCl2 1.2, glucose 10, CaCl2 0.5, and 2,3- butanedione monoximine (BDM) 20, pH 7.35-7.45 at room temperature (21-22°C). Trabeculae from the right ventricle of the heart are dissected and mounted between a force transducer and a motor arm and superfused with normal K-H solution (KCl, 5 mM) at a rate of ∼10 ml/min and stimulated at 0.5 Hz. Dimensions of the muscles are measured with a calibration reticule in the ocular of the dissection microscope (×40, resolution ∼10 µm).

Force is measured using a force transducer system and is expressed in millinewtons per square millimeter of cross-sectional area. Sarcomere length is measured by laser diffraction. Resting sarcomere length is set at 2.20-2.30 µm throughout the experiments.

Intracellular calcium is measured using the free acid form of fura-2 as described in previous studies (Gao *et al*., 1994; Backx *et al*., 1995; Gao *et al*., 1998). Fura-2 potassium salt is microinjected iontophoretically into one cell and allowed to spread throughout the whole muscle (via gap junctions). The tip of the electrode (∼0.2 µm in diameter) is filled with fura-2 salt (1 mM) and the remainder of the electrode is filled with 150 mM KCl. After a successful impalement into a superficial cell in non-stimulated muscle, a hyperpolarizing current of 5-10 nA is passed continuously for ∼15 min. Fura-2 epifluorescence is measured by exciting at 380 and 340 nm. Fluorescent light is collected at 510 nm by a photomultiplier tube. The output of photomultiplier is collected and digitized. Ryanodine (1.0 µM) is used to enable steady-state activation. After 15 min of exposure to ryanodine, different levels of tetanizations are induced briefly (∼4-8 seconds) by stimulating the muscles at 10 Hz at varied extracellular calcium (0.5- 20 mM). All experiments are performed at room temperature (20-22° C).

### Diseases or conditions implicating ischemia/reperfusion

*In vitro* models can also be used to determine the ability of any of the compounds and pharmaceutical compositions described herein to treat, prevent and/or delay the onset and/or the development of a disease or condition implicating ischemia/reperfusion injury in an individual.

### Cancer

Antitumor activities of the compounds described herein can be assessed using *in vitro* proliferation assays of tumor cells using well-known methods, such as described in Norris A. J. et al. Intl. J. Cancer 2008, 122:1905-1910.

Cells of an appropriate cell line, e.g. human breast cancer cell line MCF-7, are seeded in 96-well tissue culture microtiter plates at ∼4000 cells per well for an overnight incubation. Serial 10-fold dilutions of test compounds are added, and the cells are incubated for 72 h. The cell viability is determined using the CellTiter-Glo™ Luminescent Cell Viability Assay (Promega; Madison, WI). IC₅₀ is measured as the concentration of drug required for inhibiting cell growth by 50%.

### EXAMPLE 8: In Vivo and/or Ex Vivo Models to Determine Ability of Compounds and Pharmaceutical Compositions to Treat, Prevent and/or Delay Onset and/or Development of a Disease or Condition

### Cardiovascular Diseases or Condition

*In vivo* models of cardiovascular disease can also be used to determine the ability of any of the compounds and pharmaceutical compositions described herein to treat, prevent and/or delay the onset and/or the development of a cardiovascular disease or condition in an individual. An exemplary animal model of heart disease is described below.

*In vivo* cardiovascular effects obtained with a compound or pharmaceutical composition may be assessed in a control (normal) dog. The study is conducted in adult (25 kg) mongrel (male) dogs chronically instrumented for conscious hemodynamic analysis and blood sampling, as previously described (Katori, T. et al., Circ. Res. 2005, 96, 234-243.). Micromanometer transducers in the left ventricle provide pressure, while right atrial and descending aortic catheters provide fluid-pressures and sampling conduits. Endocardial sonomicrometers (anteriorposterior, septal-lateral) measure short-axis dimensions, a pneumatic occluder around the inferior vena cave facilitated pre-load manipulations for pressure-relation analysis. Epicardial pacing leads are placed on the right atrium, and another pair is placed on the right ventricle free wall linked to a permanent pacemaker to induce rapid pacing-cardiac failure. After 10 days of recovery, animals are evaluated at baseline sinus rhythm and with atrial pacing (120-160 bpm). Measurements include conscious hemodynamic recordings for cardiac mechanics.

Compounds described herein are administrated to a healthy control dog at the dose of 1-5 µg/kg/min and the resulting cardiovascular data is obtained.

Demonstration that a compound described herein improves cardiac hemodynamics in hearts with congestive failure: After completing protocols under baseline conditions, congestive heart failure is induced by tachypacing (210 bpm x 3 weeks, 240 bpm x 1 week), as previously described (Katori, T. et al., Circ. Res. 2005, 96: 234-243.). Briefly, end-diastolic pressure and dP/dtₘₐₓ are measured weekly to monitor failure progression. When animals demonstrate a rise in EDP more than 2x, and dP/dtₘₐₓ of >50% baseline, they are deemed ready for congestive heart failure studies.

The values for test compounds and pharmaceutical compositions are obtained after 15 min continuous i.v. infusion (2.5 or 1.25 µg/kg/min) in control and heart failure preparations, respectively, both in the absence and in the presence of volume restoration. For comparison, the same hemodynamic measurements are obtained with AS in heart failure preparations.

### Diseases or Conditions Implicating Ischemia/Reperfusion

*Ex-vivo* models of ischemia/reperfusion can also be used to determine the ability of any of the compounds described herein to treat, prevent and/or delay the onset and/or the development of a disease or condition implicating ischemia/reperfusion injury in an individual. An exemplary *ex vivo* model of ischemia/reperfusion injury is described below.

Male Wistar rats are housed in identical cages and allowed access to tap water and a standard rodent diet *ad libitum.* Each animal is anesthetized with 1 g/kg urethane *i.p.* 10 min after heparin (2,500 U, *i.m*.) treatment. The chest is opened, and the heart is rapidly excised, placed in ice-cold buffer solution and weighed. Isolated rat hearts are attached to a perfusion apparatus and retrogradely perfused with oxygenated buffer solution at 37° C. The hearts are instrumented as previously described in Rastaldo et al., Am. J. Physiol. 2001, 280, H2823-H2832, and Paolocci et al., Am. J. Physiol. 2000, 279, H1982-H1988. The flow is maintained constant (approximately 9 mL/min/g wet weight) to reach a typical coronary perfusion pressure of 85-90 mmHg. A constant proportion of 10% of the flow rate is applied by means of one of two perfusion pumps (Terumo, Tokyo, Japan) using a 50 mL syringe connected to the aortic cannula. Drug applications are performed by switching from the syringe containing buffer alone to the syringe of the other pump containing the drug (compound or pharmaceutical composition described herein) dissolved in a vehicle at a concentration 1 0× to the desired final concentration in the heart. A small hole in the left ventricular wall allows drainage of the thebesian flow, and a polyvinyl- chloride balloon is placed into the left ventricle and connected to an electromanometer for recording of left ventricular pressure (LVP). The hearts are electrically paced at 280-3 00 bpm and kept in a temperature-controlled chamber (37° C.). Coronary perfusion pressure (CPP) and coronary flow are monitored with a second electromanometer and an electromagnetic flow- probe, respectively, both placed along the perfusion line. Left ventricular pressure, coronary flow and coronary perfusion pressure are recorded using a TEAC R-7 1 recorder, digitized at 1000 Hz and analyzed off-line with DataQ-Instruments/CODAS software, which allow quantification of the maximum rate of increase of LVP during systole (dP/dtₘₐₓ).

Hearts are perfused with Krebs-Henseleit solution gassed with 95% O2 and 5% CO2 of the following composition: 17.7 mM sodium bicarbonate, 127 mM NaCl, 5.1 mM KCl, 1.5 mM CaCl2, 1.26 mM MgCl2, 11 mM D-glucose, supplemented with 5 µg/mL lidocaine.

The test compound or pharmaceutical compositions are diluted in buffer immediately prior to use. Hearts are allowed to stabilize for 30 min, and baseline parameters are recorded. Typically, coronary flow is adjusted within the first 10 min and kept constant from thereon. After 30 min stabilization, hearts are randomly assigned to one of the treatment groups, and subjected to 30 min global, no-flow ischemia, followed by 30 min of reperfusion (I/R). Pacing of the hearts is stopped at the beginning of the ischemic period and restarted after the third minute of reperfusion.

Hearts in a control group are perfused with a buffer for an additional 29 min after stabilization. Treated hearts are exposed to a test compound or pharmaceutical composition (*e.g.,* 1 µM final concentration for about 20 min followed by a 10 min buffer wash-out period).

In all hearts, pacing is suspended at the onset of ischemia and restarted 3 minutes following reperfusion. As isolated heart preparations may deteriorate over time (typically after 2-2.5 hours perfusion), the re-flow duration is limited to 30 minutes in order to minimize the effects produced by crystalloid perfusion on heart performance, and consistently with other reports.

Assessment of ventricular function: To obtain the maximal developed LVP, the volume of the intra-ventricular balloon is adjusted to an end-diastolic LVP of 10 mmHg during the stabilization period, as reported in Paolocci, *supra,* and Hare et al., J. Clin. Invest. 1998, 101, 1424-31. Changes in developed LVP, dP/dtₘₐₓ and the end-diastolic value induced by the I/R protocol are continuously monitored. The difference between the end- diastolic LVP (EDLVP) before the end of the ischemic period and during pre-ischemic conditions is used as an index of the extent of contracture development. Maximal recovery of developed LVP and dP/dtₘₐₓ during reperfusion is compared with respective pre-ischemic values.

Assessment of myocardial injury: Enzyme release is a measure of severe myocardial injury that has yet to progress to irreversible cell injury. Samples of coronary effluent (2 mL) are withdrawn with a catheter inserted into the right ventricle via the pulmonary artery. Samples are taken immediately before ischemia and at 3, 6, 10, 20 and 30 min of reperfusion. LDH release is measured as previously described by Bergmeyer et al., Verlag Chemie 1974. Data are expressed as cumulative values for the entire reflow period.

To corroborate the data relative to myocardial injury, determined by LDH release, infarct areas are also assessed in a blinded fashion. At the end of the course (30 min reperfusion), each heart is rapidly removed from the perfusion apparatus, and the LV dissected into 2-3 mm circumferential slices. Following 15 min of incubation at 37° C. in 0.1% solution of nitro blue tetrazolium in phosphate buffer as described in Ma et al., Proc. Natl. Acad. Sci. 1999, 96, 14617-14622, unstained necrotic tissue is separated from the stained viable tissue. The areas of viable and necrotic tissue are carefully separated by an independent observer who is not aware of the origin of the hearts. The weight of the necrotic and non-necrotic tissues is then determined and the necrotic mass expressed as a percentage of total left ventricular mass.

Data may be subjected to statistical methods such as ANOVA followed by the Bonferroni correction for post hoc t tests.

### Cancer

Anticancer activities of compounds described herein can be assessed using *in vivo* mouse xenograft models using methods described in Norris A. J. et al., Intl. J. Cancer 2008, 122, 1905- 1910 and Stoyanovsky, D.A. et al., J. Med. Chem. 2004, 47, 210-217).

Mice are inoculated with appropriate tumor cells by subcutaneous injection into the lower flank. Therapy can be started after 1-3 weeks when the tumors have reached an average volume of ∼50-60 mm³. Tumor diameters are measured with digital calipers, and the tumor volume is calculated. The anti-tumor efficacy of test compounds is assessed by comparison of tumor size in test group to that in the control group.

### EXAMPLE 9: In Vivo Animal Studies (Acute Treatment, Intravenous Infusion)

This example demonstrates the efficacy of compounds and pharmaceutical compositions described herein to lower pulmonary artery pressure in rats with monocrotaline-induced PH.

Rats (250-250 g) are anesthetized via an intra-muscular (i.m.) injection of ketamine/xylazine (80/10 mg/kg). A half dose (40 mg/kg ketamine/5 mg/kg xylazine) is given as supplemental anesthesia as needed. Animals are placed on a heating pad set to maintain body temperature at approximately 37°C. Body temperature is monitored throughout the experiment. Once consciousness is lost, a pressure transducer is inserted into a femoral artery to measure arterial blood pressure. A fluid filled catheter is inserted through the right jugular vein into the pulmonary artery to measure pulmonary artery pressure via a pressure transducer. A cannula is placed into the left jugular vein for dosing.

Monocrotaline is administered via a single subcutaneous injection (60 mg/kg) approximately 3 weeks prior to the terminal procedure. A baseline pulmonary artery pressure of >30 mmHg is required to initiate study of the compounds described herein. A compound or pharmaceutical composition as described herein is administered intravenously in a dose-escalation manner in 20 minute intervals from doses of 10 to 300 µg/kg/min. Hemodynamic indices, including MAP (mean arterial pressure), SAP (systolic arterial pressure), DAP (diastolic arterial pressure), HP (heart rate), MPAP (mean pulmonary arterial pressure), SPAP (systolic arterial pressure), DPAP (diastolic pulmonary arterial pressure), are measured.

For the terminal procedure, after surgical instrumentation and an approximate 10 minute pre-dose equilibration period, test compound or pharmaceutical composition solutions are infused via jugular vein catheter. At the end of the experiment, rats are euthanized under anesthesia via pentobarbital overdose.

### EXAMPLE 10: In Vivo Animal Studies (Acute Treatment, Intravenous Infusion or Inhaled Administration)

This example demonstrates the efficacy of the compounds and pharmaceutical compositions described herein to lower pulmonary artery pressure in dogs with hypoxia-induced PH.

Healthy dogs (10-15 kg) are anesthetized with pentobarbital (20-40 mg/kg. intravenously) and anesthesia is maintained by continuous infusion of pentobarbital at rate of 5-10 mg/kg/h. Dogs are intubated via a tracheotomy, and artificially respired (while monitoring inspired oxygen and expired CO₂). The left femoral vein and artery are cannulated for dose administration and arterial blood pressure recording. The right jugular vein is cannulated with a pulmonary artery pressure catheter (Swan Ganz catheter), to measure both pulmonary arterial pressure (PAP) and pulmonary wedge pressure (PWP). This catheter is also used for measurement of cardiac output via thermodilution techniques following rapid injection of cold 5 mL saline. Electrocardiograms are monitored throughout the experiment.

During the baseline and control measurements inspired oxygen is maintained at 40%. Hypoxia is induced by adding nitrogen to the respiratory gas at a rate sufficient to reduce respired oxygen to 10% (FiO2=10%). Each hypoxic condition is maintained for 15-30 minutes and then normoxic (FiO2=40%) control condition is returned. Each dose of test compound or pharmaceutical composition is intravenously administered during the 30 minute hypoxic condition; no drug is infused during the subsequent normoxia until the next dose is given. Test compounds or pharmaceutical compositions are given intravenously in the range of 1 to 100 µg/kg/min and various hemodynamic indices are recorded. Alternatively, in this experiment test compounds or pharmaceutical compositions are administered using an inhalation nebulizer at dose levels of 0.1-1 g/kg in 5-10 time period during each hypoxia period.

### EXAMPLE 11: In Vivo Animal Studies (Chronic Treatment, Continuous Intravenous Infusion)

This example demonstrates the efficacy of the compounds and pharmaceutical compositions described herein to retard the progression of disease in rats with monocrotaline-induced PH.

Rats (200-250g) are surgically implanted with a pressure transducer equipped telemetry transmitter. The transmitter assembly is secured internally; the fluid-filled catheter is placed into the jugular vein with the tip of the pressure transducer positioned in the right ventricle for collection of right ventricular pressure (RVP) data. Additionally, all animals, with the exception of the sham group, are implanted with femoral vein cannulas for the purposes of dosing.

Monocrotaline (MCT) is administered to vehicle-control animals by subcutaneous injection. One week following the MCT injection, the vehicle-control animals are administered saline or a low or high dose of a test compound or pharmaceutical composition by continuous intravenous infusion for two weeks. The test and vehicle control article are administered by external pump. Weekly clinical observations are performed on animals.

For cardiovascular evaluations, RVP data is collected with animals allowed free movement in the home cage. The animals are monitored for at least 24 hours prior to MCT administration. RVP is also monitored at 24 hours following the end of the two week infusion, and occurs for at least 24 hours. All animals are necropsied at the end of the study. Weights of lungs and pulmonary artery, heart and each individual chamber are evaluated. The weights of the heart, LV, RV, and ratio to body weight are reported. The small pulmonary arteries from each animal are evaluated for medial thickness, neointima, and smooth muscle hypertrophy.

### EXAMPLE 12: In Vivo Animal Studies (Chronic Treatment, Oral Administration)

This example demonstrates the efficacy of the compounds and pharmaceutical compositions described herein to retard the progression of disease in rats with monocrotaline-induced PH.

The general methodology for this experiment is similar to that of Example 12 above. One difference is that the route of administration is oral, with a dosing regimen of once to four times daily at dose levels of 0.1-1 g/kg.

### EXAMPLE 13: In Vivo Animal Studies (Chronic Treatment, Continuous Intravenous Infusion)

This example demonstrates the efficacy of the compounds and pharmaceutical compositions described herein to reverse the progression of disease in rats with monocrotaline-induced PH.

In this study, rats (200-250 g) rats are surgically implanted with a pressure transducer equipped telemetry transmitter. The transmitter assembly is secured internally; the fluid-filled catheter is placed into the jugular vein with the tip of the pressure transducer positioned in the right ventricle for collection of right ventricular pressure (RVP) data. Additionally, all animals, with exception of sham group, are implanted with femoral vein cannulas for the purposes of dosing.

The vehicle and control article, monocrotaline (MCT), are administered by subcutaneous injection. Three weeks following the MCT injection, animals are administered saline or a low or high dose of a test compound or pharmaceutical composition by continuous intravenous infusion for three weeks. The test compound or pharmaceutical composition and vehicle control article are administered by external pump. Weekly clinical observations are performed on the animals.

For cardiovascular evaluations, RVP data is collected with animals allowed free movement in the home cage. The animals are monitored for at least 24 hours prior to MCT administration. RVP is also monitored for at least 24 hours following the end of the two week infusion. All animals are necropsied at the end of the study. Weights of lungs and pulmonary artery, heart and each individual chamber are evaluated. The weights of the heart, LV, RV, and ratio to body weight are reported. The small pulmonary arteries from each animal are evaluated for medial thickness, neointima, and smooth muscle hypertrophy.

### EXAMPLE 14: In Vivo Animal Studies (Chronic Treatment, Oral Administration)

This example demonstrates the efficacy of the compounds and pharmaceutical compositions described herein to reverse the progression of disease in rats with monocrotaline-induced PH.

The general methodology is similar to that of Example 14, with the exception that the route of administration is oral, with a dosing regimen of one to four times daily at dose levels of 0.1-1 g/kg.

### EXAMPLE 15: In Vivo Animal Studies (Chronic Treatment, Inhaled Administration)

This example demonstrates the efficacy of the compounds and pharmaceutical compositions described herein to retard progression of disease in rats with monocrotaline-induced PH.

The general methodology is similar to that of Example 12 above, with the exception that the route of administration is via inhalation, with a dosing regimen of one to four times daily at dose levels of 0.1-1 g/kg.

### EXAMPLE 16: In Vivo Animal Studies (Chronic Treatment, Inhaled Administration)

This example demonstrates the efficacy of the compounds and pharmaceutical compositions described herein to reverse the progression of disease in rats with monocrotaline-induced PH.

The general methodology is similar to that of Example 12, with the exception that the route of administration is via inhalation, with a dosing regimen of one to four times daily at dose levels of 0.1-1 g/kg.

### EXAMPLE 17: In Vivo Animal Studies (Acute Treatment, Intravenous Infusion and Inhaled Administration)

This example demonstrates the efficacy of the compounds and pharmaceutical compositions described herein to lower pulmonary artery pressure in dogs with thromboxane-induced PH.

Experimental PH is induced by continuous infusion of a thromboxane A2 receptor agonist analog (for example U46619, Tocris Bioscience). The thromboxane A2 receptor agonist analog infusion rate (0.1-1 mg/kg/min) is adjusted to maintain a systolic pulmonary artery pressure (PAP) at 40 mmHg in anesthetized and mechanically ventilated dogs. The left femoral vein and artery are cannulated for dose administration and arterial blood pressure recording. The right jugular vein is cannulated with a pulmonary artery pressure catheter (Swan Ganz catheter), to measure both pulmonary arterial pressure (PAP) and pulmonary wedge pressure (PWP). This catheter is also used for measurement of cardiac output via thermodilution techniques following rapid injection of cold 5 mL saline. Electrocardiograms are monitored throughout the experiment.

Once a stable steady-state in hemodynamic is achieved, various doses of the test compounds or pharmaceutical compositions are given intravenously at dose rates in the range of 1 to 100 µg/kg/min and various hemodynamic indices are recorded. Alternatively, in this experiment the test compounds or pharmaceutical compositions are administered using an inhalation nebulizer at dose levels of 0.1-1 g/kg in 5-10 time period.

### EXAMPLE 18: Human Clinical Trials to Determine Ability of Compounds or Pharmaceutical Compositions to Treat, Prevent and/or Delay Onset and/or Development of a Disease or Condition

Any of the compounds and pharmaceutical compositions described herein can also be tested in humans to determine the ability of the compounds or pharmaceutical compositions to treat, prevent and/or delay the onset and/or the development of a disease or condition. Standard methods can be used for these clinical trials. In one exemplary method, individuals with a disease or condition described herein, such as congestive heart failure, are enrolled in a tolerability, pharmacokinetics and pharmacodynamics phase I study of a therapy using the compounds described herein in standard protocols. Then a phase II, double-blind randomized controlled trial is performed to determine the efficacy of the compounds using standard protocols.

## Claims

1. A compound of formula (I) 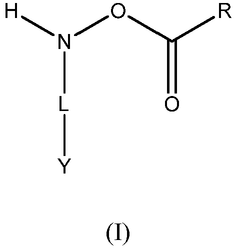 or a pharmaceutically acceptable salt, hydrate, or solvate thereof wherein:
R is hydrogen, alkyl, heterocycloalkyl, aryl, benzyl, alkoxy, heterocycloalkoxy, aryloxy, benzyloxy, -NR³R⁴ or -N(OR³)R⁴, wherein said alkyl, heterocycloalkyl, aryl, benzyl, alkoxy, heterocycloalkoxy, aryloxy and benzyloxy are unsubstituted or substituted with one or more substituents;
R³ and R⁴ are independently alkyl, heterocycloalkyl or aryl, wherein said alkyl, heterocycloalkyl and aryl are unsubstituted or substituted with one or more substituents;
L is SO₂,
Y is aryl and said aryl is unsubstituted or substituted with one or more substituents independently selected from W, and
W is halo, -OH, -CN, -NO₂, -COR¹, -COOR¹, -CONR¹R², -CH(C(O)R¹)₂, -SO₂R¹, or -COX, wherein X is halo and R¹ and R² are independently alkyl or aryl, or R¹ and R² are taken together to form a cycloalkyl or heterocycloalkyl, wherein said cycloalkyl and heterocycloalkyl are unsubstituted or substituted with one or more substituents;
and where the substituents which may be present when the above groups include said one or more substituents are the same or different and are selected from halo, hydroxyl, amino, alkylamino, arylamino, dialkylamino, diarylamino, cyano, nitro, mercapto, oxo, carbonyl, thio, imino, formyl, carbamido, carbamyl, carboxyl, thioureido, thiocyanato, sulfoamido, sulfonylalkyl, sulfonylaryl, alkyl, alkenyl, alkoxy, mercaptoalkoxy, aryl, heteroaryl, cyclyl, heterocyclyl, wherein alkyl, alkenyl, alkyloxy, aryl, heteroaryl, cyclyl, and heterocyclyl are optionally substituted with alkyl, aryl, heteroaryl, halogen, hydroxyl, amino, mercapto, cyano, nitro, oxo, thioxo, or imino;
said compound, salt, hydrate, or solvate being for use in a method of treating a disease or condition selected from cardiovascular diseases, ischemia, reperfusion injury, cancerous disease and pulmonary hypertension.

2. The compound, salt, hydrate or solvate of claim 1 for a use as defined in claim 1, wherein W is chloro, bromo or -SO₂R¹.

3. The compound, salt, hydrate or solvate of claim 1 for a use as defined in claim 1, wherein R is alkyl or phenyl, wherein said alkyl and phenyl are unsubstituted or substituted with one or more halos.

4. The compound, salt, hydrate or solvate of claim 1 for a use as defined in claim 1, which compound is selected from:
N-acetyloxy-benzenesulfonamide;
N-benzoyloxy-benzenesulfonamide;
N-(trifluoroacetyloxy)-benzenesulfonamide;
*N*-(acetyloxy)-2-bromobenzenesulfonamide;
*N*-acetyloxy-2,6-dichlorobenzenesulfonamide; and
pharmaceutically acceptable salts, hydrates and solvates thereof.

5. A compound, salt, hydrate or solvate of any one of claims 1 to 4 for a use as defined in claim 1, wherein the disease or condition is a cardiovascular disease.

6. A compound, salt, hydrate or solvate of claim 5 for a use as defined in claim 5, wherein the cardiovascular disease is heart failure.

7. A compound, salt, hydrate or solvate of claim 6 for a use as defined in claim 6, wherein the heart failure is congestive heart failure, acute congestive heart failure, or acute decompensated heart failure.

8. A compound, salt, hydrate or solvate of any one of claims 1 to 4 for a use as defined in claim 1, wherein the disease or condition is ischemia or reperfusion injury.

9. A compound, salt, hydrate or solvate of any one of claims 1 to 4 for a use as defined in claim 1, wherein the disease or condition is a cancerous disease.

10. A compound, salt, hydrate or solvate of any one of claims 1 to 4 for a use as defined in claim 1, wherein the disease or condition is pulmonary hypertension.

## Patentansprüche

1. Verbindung der Formel (I) 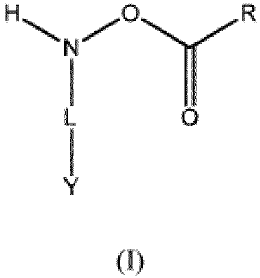 oder ein pharmazeutisch verträgliches Salz, Hydrat oder Solvat davon, worin:
R Wasserstoff, Alkyl, Heterocycloalkyl, Aryl, Benzyl, Alkoxy, Heterocycloalkoxy, Aryloxy, Benzyloxy, -NR³R⁴ oder -N(OR³)R⁴ ist, wobei die Alkyl, Heterocycloalkyl, Aryl Benzyl, Alkoxy, Heterocycloalkoxy, Aryloxy und Benzyloxy unsubstituiert oder substituiert sind mit einem oder mehreren Substituenten;
R³ und R⁴ unabhängig voneinander Alkyl, Heterocycloalkyl oder Aryl sind, wobei das Alkyl, Heterocycloalkyl und Aryl unsubstituiert oder substituiert sind mit einem oder mehreren Substituenten;
L SO₂ ist,
Y Aryl ist und das Aryl unsubstituiert oder substituiert ist mit einem oder mehreren Substituenten, die unabhängig ausgewählt sind aus W, und
W Halogen, -OH, -CN, -NO₂, -COR¹, -COOR¹, -CONR¹R², -CH(C(O)R¹)₂, SO₂R¹ oder -COX ist, worin X Halogen ist und R¹ und R² unabhängig voneinander Alkyl oder Aryl sind, oder R¹ und R² zusammengenommen sind, um ein Cycloalkyl oder Heterocycloalkyl zu bilden, wobei das Cycloalkyl und Heterocycloalkyl unsubstituiert oder substituiert sind mit einem oder mehreren Substituenten;
und wobei die Substituenten, die vorhanden sein können, wenn die vorgenannten Gruppen den einen oder die mehreren Substituenten einschliessen, gleich oder verschieden sind und ausgewählt sind aus Halogen, Hydroxyl, Amino, Alkylamino, Arylamino, Dialkylamino, Diarylamino, Cyano, Nitro, Mercapto, Oxo, Carbonyl, Thio, Imino, Formyl, Carbamido, Carbamyl, Carboxyl, Thioureido, Thiocyanato, Sulfoamido, Sulfonylalkyl, Sulfonylaryl, Alkyl, Alkenyl, Alkoxy, Mercaptoalkoxy, Aryl, Heteroaryl, Cyclyl, Heterocyclyl, wahlweise substituiert mit Alkyl, Aryl, Heteroaryl, Halogen, Hydroxyl, Amino, Mercapto, Cyano, Nitro, Oxo, Thioxo oder Imino;
wobei die Verbindung, Salz, Hydrat oder Solvat zur Verwendung in einem Verfahren zum Behandeln einer Erkrankung oder eines Zustands dienen, ausgewählt aus kardiovaskulären Erkrankungen, Ischämie, Reperfusionsverletzung, Krebserkrankung, pulmonaler Hypertonie.

2. Verbindung, Salz, Hydrat oder Solvat nach Anspruch 1 zur Verwendung nach Anspruch 1, wobei W Chlor, Brom oder -SO₂R¹ ist.

3. Verbindung, Salz, Hydrat oder Solvat nach Anspruch 1 zur Verwendung nach Anspruch 1, wobei R Alkyl oder Phenyl ist, wobei das Alkyl oder Phenyl unsubstituiert oder substituiert ist mit einem oder mehreren Halogenen.

4. Verbindung, Salz, Hydrat oder Solvat nach Anspruch 1 zur Verwendung nach Anspruch 1, wobei die Verbindung ausgewählt ist aus:
N-Acetyloxy-benzolsulfonamid;
N-Benzoyloxy-benzolsulfonamid;
N-(Trifluoracetyloxy)-benzolsulfonamid;
N-(Acetyloxy)-2-brombenzolsulfonamid;
N-Acetyloxy-2,6-dichlorbenzolsulfonamid; und
pharmazeutisch verträglichen Salzen, Hydraten und Solvaten davon.

5. Verbindung, Salz, Hydrat oder Solvat nach einem der Ansprüche 1 bis 4 zur Verwendung nach Anspruch 1, wobei die Erkrankung oder der Zustand eine kardiovaskuläre Erkrankung ist.

6. Verbindung, Salz, Hydrat oder Solvat nach Anspruch 5 zur Verwendung nach Anspruch 5, wobei die kardiovaskuläre Erkrankung Herzinsuffizienz ist.

7. Verbindung, Salz, Hydrat oder Solvat nach Anspruch 6 zur Verwendung nach Anspruch 6, wobei die Herzinsuffizienz kongestive Herzinsuffizienz, akute kongestive Herzinsuffizienz oder akute dekompensierte Herzinsuffizienz ist.

8. Verbindung, Salz, Hydrat oder Solvat nach einem der Ansprüche 1 bis 4 zur Verwendung nach Anspruch 1, wobei die Erkrankung oder der Zustand Ischämie oder Reperfusionsverletzung ist.

9. Verbindung, Salz, Hydrat oder Solvat nach einem der Ansprüche 1 bis 4 zur Verwendung nach Anspruch 1, wobei die Erkrankung oder der Zustand eine Krebserkrankung ist.

10. Verbindung, Salz, Hydrat oder Solvat nach einem der Ansprüche 1 bis 4 zur Verwendung nach Anspruch 1, wobei die Erkrankung oder der Zustand pulmonale Hypertension ist.

## Revendications

1. Composé de la formule (I): 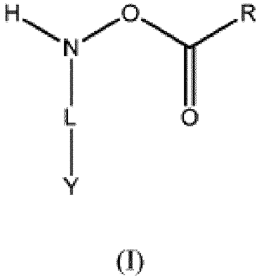 ou un sel pharmaceutiquement acceptable, un hydrate ou un solvate de celui-ci, dans lequel:
R est un hydrogène, un alkyle, un hétérocycloalkyle, un aryle, un benzyle, un alcoxy, un hétérocycloalcoxy, un aryloxy, un benzyloxy, -NR³R⁴ ou -N(OR³)R⁴, dans lequel lesdits alkyle, hétérocycloalkyle, aryle, benzyle, alcoxy, hétérocycloalcoxy, aryloxy et benzyloxy sont non substitués ou substitués avec un ou plusieurs substituants;
R³ et R⁴ sont indépendamment un alkyle, un hétérocycloalkyle ou un aryle, dans lequel lesdits alkyle, hétérocycloalkyle et aryle sont non substitués ou substitués avec un ou plusieurs substituants;
L est SO₂;
Y est un aryle et ledit aryle est non substitué ou substitué avec un ou plusieurs substituants indépendamment choisis parmi W, et
W est un halo, -OH, -CN, -NO₂, -COR¹, -COOR¹, -CONR¹R², -CH(C(O)R¹)₂, - SO₂R¹ ou -COX, dans lequel X est un halo et R¹ et R² sont indépendamment un alkyle ou un aryle, ou R¹ et R² sont pris ensemble pour former un cycloalkyle ou un hétérocycloalkyle, dans lequel lesdits cycloalkyle et hétérocycloalkyle sont non substitués ou substitués avec un ou plusieurs substituants;
et dans lequel les substituants qui peuvent être présents lorsque les groupes ci-dessus incluent lesdits un ou plusieurs substituants sont identiques ou différents et sont choisis parmi un halo, un hydroxyle, un amino, un alkylamino, un arylamino, un dialkylamino, un diarylamino, un cyano, un nitro, un mercapto, un oxo, un carbonyle, un thio, un imino, un formyle, un carbamido, un carbamyle, un carboxyle, un thiouréido, un thiocyanato, un sulfoamido, un sulfonylalkyle, un sulfonylaryle, un alkyle, un alcényle, un alcoxy, un mercaptoalcoxy, un aryle, un hétéroaryle, un cyclyle, un hétérocyclyle, dans lequel l'alkyle, l'alcényle, l'alkyloxy, l'aryle, l'hétéroaryle, le cyclyle et l'hétérocyclyle sont optionnellement substitués avec un alkyle, un aryle, un hétéroaryle, un halogène, un hydroxyle, un amino, un mercapto, un cyano, un nitro, un oxo, un thioxo ou un imino;
ledit composé, sel, hydrate ou solvate étant pour une utilisation dans une méthode de traitement d'une maladie ou d'un état choisi parmi des maladies cardiovasculaires, une ischémie, une lésion de reperfusion, une maladie cancéreuse et une hypertension pulmonaire.

2. Composé, sel, hydrate ou solvate selon la revendication 1 pour une utilisation telle que définie dans la revendication 1, dans lequel W est un chloro, un bromo ou -SO₂R¹.

3. Composé, sel, hydrate ou solvate selon la revendication 1 pour une utilisation telle que définie dans la revendication 1, dans lequel R est un alkyle ou un phényle, dans lequel lesdits alkyle et phényle sont non substitués ou substitués avec un ou plusieurs halos.

4. Composé, sel, hydrate ou solvate selon la revendication 1 pour une utilisation telle que définie dans la revendication 1, lequel composé est choisi parmi:
N-acétyloxy-benzènesulfonamide;
N-benzoyloxy-benzènesulfonamide;
N-(trifluoroacétyloxy)-benzènesulfonamide;
N-(acétyloxy)-2-bromobenzènesulfonamide;
N-acétyloxy-2,6-dichlorobenzènesulfonamide; et
des sels pharmaceutiquement acceptables, des hydrates et des solvates de ceux-ci.

5. Composé, sel, hydrate ou solvate selon l'une quelconque des revendications 1 à 4 pour une utilisation telle que définie dans la revendication 1, où la maladie ou l'état est une maladie cardiovasculaire.

6. Composé, sel, hydrate ou solvate selon la revendication 5 pour une utilisation telle que définie dans la revendication 5, où la maladie cardiovasculaire est une insuffisance cardiaque.

7. Composé, sel, hydrate ou solvate selon la revendication 6 pour une utilisation telle que définie dans la revendication 6, où l'insuffisance cardiaque est une insuffisance cardiaque congestive, une insuffisance cardiaque congestive aiguë ou une insuffisance cardiaque décompensée aiguë.

8. Composé, sel, hydrate ou solvate selon l'une quelconque des revendications 1 à 4 pour une utilisation telle que définie dans la revendication 1, où la maladie ou l'état est une ischémie ou une lésion de reperfusion.

9. Composé, sel, hydrate ou solvate selon l'une quelconque des revendications 1 à 4 pour une utilisation telle que définie dans la revendication 1, où la maladie ou l'état est une maladie cancéreuse.

10. Composé, sel, hydrate ou solvate selon l'une quelconque des revendications 1 à 4 pour une utilisation telle que définie dans la revendication 1, où la maladie ou l'état est une hypertension pulmonaire.
